(19) **Europäisches Patentamt**

**European Patent Office**

**Office européen des brevets**

(11) **EP 0 843 539 B1**

(12) **EUROPEAN PATENT SPECIFICATION**

(45) Date of publication and mention
of the grant of the patent:
**06.12.2006 Bulletin 2006/49**

(21) Application number: **95910178.3**

(22) Date of filing: **03.02.1995**

(51) Int Cl.:
***A61F 13/15*** (2006.01)

(86) International application number:
**PCT/US1995/001472**

(87) International publication number:
**WO 1995/020931 (10.08.1995 Gazette 1995/34)**

(54) **ABSORBENT ARTICLES**

ABSORBIERENDER ARTIKEL

ARTICLES ABSORBANTS

(84) Designated Contracting States:
**AT BE CH DE DK ES FR GB GR IE IT LI LU NL PT SE**

(30) Priority: **04.02.1994 US 192240**

(43) Date of publication of application:
**27.05.1998 Bulletin 1998/22**

(73) Proprietor: **THE PROCTER & GAMBLE COMPANY**
**Cincinnati, Ohio 45202 (US)**

(72) Inventors:
  • **OSBORN, Thomas, Ward III**
    **Cincinnati, OH 45224 (US)**
  • **HINES, Letha, Margie**
    **Cincinnati, OH 45215 (US)**
  • **PATTERSON, Rebecca, White**
    **Cincinnati, OH 45243 (US)**

  • **LAVASH, Bruce, William**
    **West Chester, OH 45069 (US)**
  • **CARRIER, Michael, Edward**
    **Cincinnati, OH 45251 (US)**
  • **BERGMAN, Carl, Louis**
    **Loveland, OH 45140 (US)**
  • **GARTH, Shirley, Florence**
    **Cincinnaty, OH 45225 (US)**

(74) Representative: **Veronese, Pancrazio et al**
**Procter & Gamble Italia S.p.A.**
**Italian Research Center**
**Via Aterno 92/94**
**66020 Sambuceto di San Giovanni Teatino**
**(Chieti) (IT)**

(56) References cited:
**EP-A- 0 321 980**        **WO-A-93/01785**
**WO-A-94/02094**

**Description**

FIELD OF THE INVENTION

The present invention relates to absorbent articles such as sanitary napkins, pantiliners, and incontinence pads. More particularly, the present invention relates to sanitary napkins that provide improved protection through a combination of improved contact with the wearer's body and improved coverage of the wearer's undergarments.

BACKGROUND OF THE INVENTION

Absorbent articles such as sanitary napkins, pantiliners, and incontinence pads are devices that are typically worn in the crotch region of an undergarment. These devices are designed to absorb and retain liquid and other discharges from the human body and to prevent body and clothing soiling Sanitary napkins are a type of absorbent article worn by women in a pair of panties that is normally positioned between the wearer's legs, adjacent to the perineal area of the body.

It has been found that it is desirable to maintain absorbent articles in close contact with the wearers body. The maintenance of close contact of such products to the perineal area is referred to herein as body contact. Close body contact allows the absorbent article to absorb the body exudates at their source or intercept and absorb body liquids flowing along the crevices of the wearer's body Achieving good body contact limits the chance for the body exudates to flow off of or out of the absorbent article. Current products, however, are not believed to conform to the body of the wearer as closely as desired. Thus, leakage is possible when wearing these products.

Typically, the currently marketed disposable absorbent articles of the types mentioned above also are made of materials that will not stretch That is, the materials and the article itself will not stretch under the forces that the absorbent article is normally subjected to when worn. Recently, however, efforts have been directed toward providing extensible absorbent articles for improved comfort and conformity with the wearer's body and undergarments. WO 94/02094 discloses an absorbent article which is extensible/stretchable and includes a stretchable backsheet. PCT Application Publication No. 93/01785 and its corresponding U.S. application Serial No. 071915,133, both filed July 23, 1992 (of which the present application is a continuation-in-part), discloses extensible absorbent articles. The search for improvements to the features of such absorbent articles has, however, continued.

Thus, a need exists for an absorbent article, such as a sanitary napkin, that provides improved protection from soiling. A need exists for a sanitary napkin that is provided with an improved mechanism for maintaining the sanitary napkin in contact with the wearer's body and which provides improved coverage of the wearer's undergarments.

It is, therefore, an object of the present invention to provide an absorbent article, such as a sanitary napkin, that fits closer to the wearer's body in a comfortable manner especially in the areas where menses originate and provides improved coverage of the wearer's undergarments and which retains its ability to cover a given area of the wearer's undergarments when in use.

These and other objects of the present invention will be more readily apparent when considered in reference to the following description and when taken in conjunction with the accompanying drawings.

SUMMARY OF THE INVENTION

The present invention provides an absorbent article, such as a sanitary napkin, pantiliner, or incontinence pad, in accordance with the claim. More particularly, the present invention is directed to a sanitary napkin that is provided with improved mechanisms for maintaining the sanitary napkin in contact with the wearer's body and mechanisms which provide the sanitary napkin with improved ability to cover and, thus protect the wearer's undergarments, clothing, bedding, etc. from soiling.

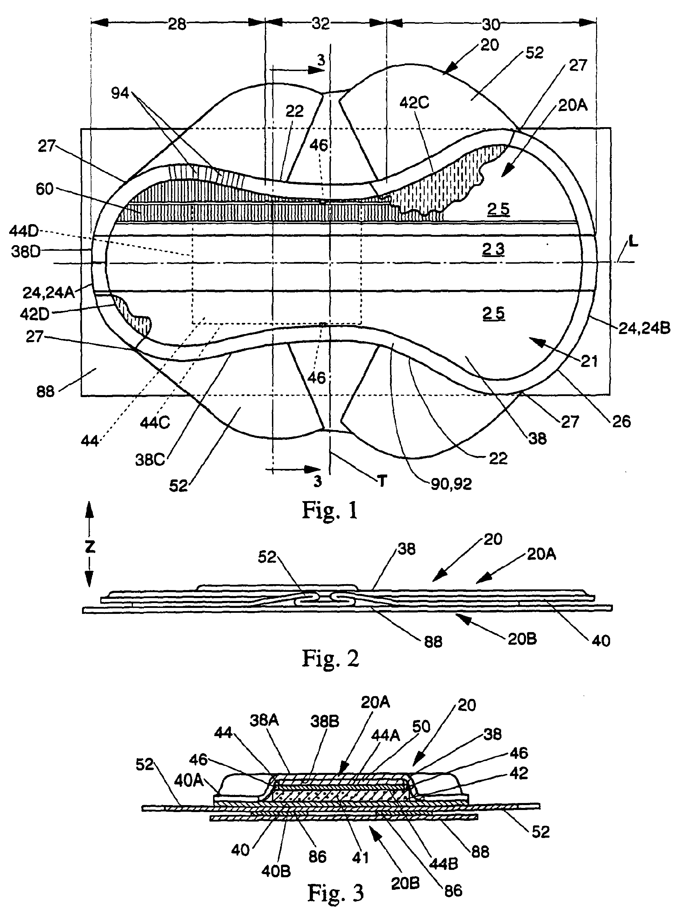

It is to be understood that the present invention specifically refers to the disclosure in respect to Section 2.F of the description and illustrated in Figures 1-5 and 13 of the drawings.

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which:

FIG. 1 is a top plan view of a preferred embodiment of the sanitary napkin of the present invention.

FIG. 2 is a side elevational view of the sanitary napkin shown in FIG. 1.

FIG. 3 is a schematic sectional view taken along line 3-3 of the sanitary napkin shown in FIG. 1.

FIG. 4 is a bottom plan view of the sanitary napkin shown in FIG. 1 shown with the release paper removed from the adhesive on the backsheet.

FIG. 5 is a perspective view of the sanitary napkin shown in FIGS. 1-4 in an in-use configuration.

FIG. 6 is a simplified plan view showing the extensibility of a sanitary napkin of a more conventional shape which has extensible components.

FIG. 7 is a table which shows preferred relationships between the magnitude of stretching forces applied to the sanitary napkin and the amount the sanitary napkin stretches in response to such forces.

FIG. 8 is a plan view photograph of a preferred embodiment of a polymeric web material having a strainable network which is used in the panty covering component of the present invention (shown with the deformations facing toward the viewer).

FIG. 9 is a segmented, perspective illustration of the polymeric web material of FIG. 8 in an untensioned condition.

FIG. 10 is a segmented, perspective illustration of a polymeric web material of FIG. 8 in a tensioned condition corresponding to stage I on the force-elongation curve depicted in FIG. 12.

FIG. 11 is a segmented perspective illustration of the polymeric web material of FIG. 8 in a tensioned condition corresponding to stage II on the force-elongation curve depicted in FIG. 12.

FIG. 12 is a graph of the resistive force versus percent elongation comparing the strainable web material shown in FIG. 8 with an otherwise identical, planar, base polymeric web material.

FIG. 13 is an enlarged plan view of the area where the topsheet and backsheet are mechanically bonded together in a manner which provides for extensibility.

FIG. 14 is a plan view of a modified crescent-shaped web that is attached to the sanitary napkin to form one of the pleated side wrapping elements.

FIG. 15 is a plan view of an alternative web of material having ring rolled sections that can be attached to the sanitary napkin to form one of the side wrapping elements.

FIG. 16 is a plan view of a sanitary napkin in which the side wrapping elements comprise more than one overlapping element on each side of the napkin.

FIG. 17 is a graph which depicts the stress-strain curves for some absorbent articles that are extensible and some absorbent articles that are relatively inextensible under low forces.

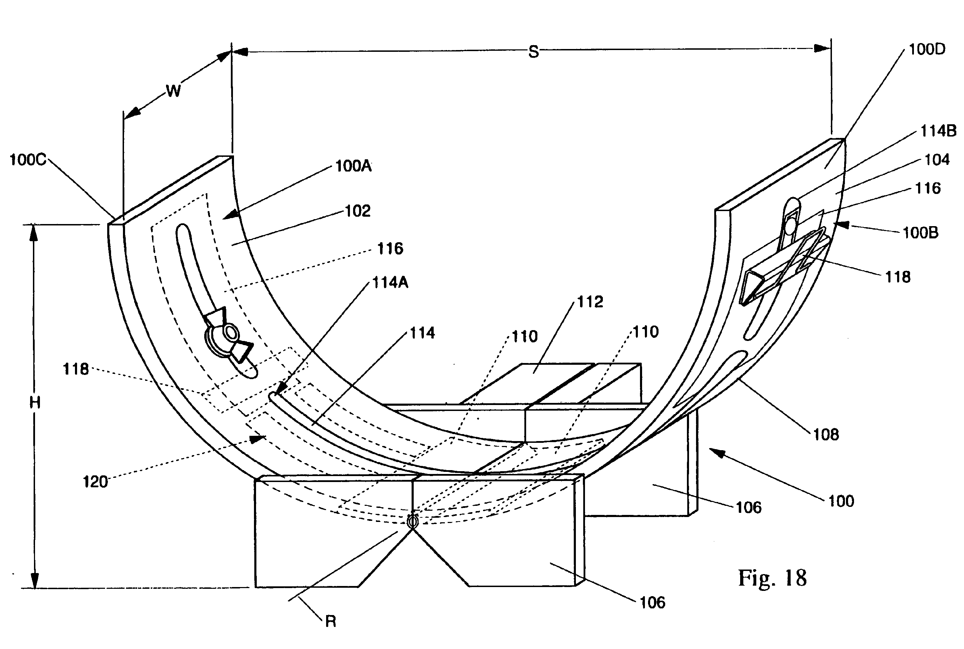

FIG. 18 is a perspective view of the Lift Test apparatus.

FIG. 19 is a bottom view of the Lift Test apparatus.

FIG. 20 is a side view of the Lift Test apparatus.

FIG. 21 is a cross-sectional view of one of the PLEXIGLAS plates used in the Lift Test apparatus as taken along line 21-21 of FIG. 20.

FIG. 21 A is a side view of the calibration of the Lift Test apparatus for a particular absorbent article.

FIG. 22 is a perspective view of the manner in which the tape pieces are attached to the end of an absorbent article (shown partially cut away) in preparation for the Lift Test.

FIG. 22A is a front view of the Pin Chamber caliper measurement device used in the Lift Test

FIGS. 23-25 are graphs which depict the Lift of a stretchable sanitary napkin according to the present invention versus a commercially-available ultra-thin sanitary napkin at the first, second, and third positions, respectively, of the Lift Test apparatus

FIG. 26 is a table of the data in FIGS. 23-25.

FIG. 27 is a graph showing the cycles of elongation of an extensible material from which the "set" of the material can be determined.

FIG. 28 is a plan view of the cardboard cutout used in the Area Coverage Test.

FIG. 29 is a schematic view of the Cantilever Drape Tester.

FIG. 30 is a perspective view of an instrument for measuring the compressive force and resiliency of a catamenial pad.

FIG. 31 is a top plan view of the compression plate assembly used in measuring the compressive force and resiliency of the catamenial pad.

FIG. 32 is a side view of the compression plate assembly shown in FIG. 31.

FIG. 33 is a top plan view of the upper compression plate of the compression plate assembly shown in FIGS. 31 and 32.

FIG. 34 is a side view of the upper compression plate assembly shown in FIG 31.

FIG. 35 is a schematic side view of the Wet Bunch Recoverability Test Apparatus.

## DETAILED DESCRIPTION OF THE INVENTION

### 1. General Characteristics of the Absorbent Article

The overall characteristics of the absorbent article of the present invention will be discussed first.

FIGS. 1-5 show a preferred embodiment of a disposable absorbent article of the present invention 20. The present invention relates to absorbent articles that are provided with an improved mechanism for maintaining the sanitary napkin in contact with the wearer's body and which provide improved coverage of the wearer's undergarments.

The term "absorbent article," as used herein, refers to articles which absorb and contain body exudates. More specifically, the term refers to articles which are placed against or in proximity to the body of the wearer to absorb and contain the various exudates discharged from the body. The term "absorbent article" is intended to include sanitary napkins, pantiliners, and incontinence pads (and other articles worn in the crotch region of a garment). The term "disposable" refers to articles which are intended to be discarded after a single use and preferably recycled, composted, or otherwise disposed of in an environmentally compatible manner. (That is, they are not intended to be laundered or otherwise restored or reused as an absorbent article.) In the preferred embodiment illustrated, the absorbent article is a sanitary napkin designated 20.

The term "sanitary napkin", as used herein, refers to an article which is worn by females adjacent to the pudendal region that is intended to absorb and contain the various exudates which are discharged from the body (e.g., blood, menses, and urine). It should be understood, however, that the present invention is also applicable to other feminine hygiene or catamenial pads such as panty liners, or other absorbent articles such as incontinence pads, and the like.

The sanitary napkin 20 has two surfaces, a liquid pervious body-contacting surface or "body surface" 20A and a liquid impervious garment surface 20B. The sanitary napkin 20 is shown in FIG. 1 as viewed from its body surface 20A. The body surface 20A is intended to be worn adjacent to the body of the wearer. The garment surface 20B of the sanitary napkin 20 (shown in FIG. 2) is on the opposite side and is intended to be placed adjacent to the wearer's undergarments when the sanitary napkin 20 is worn.

The sanitary napkin 20 has two centerlines, a principal longitudinal centerline L and a principal transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn The terms "transverse" or "lateral" used herein, are interchangeable, and refer to a line, axis or direction which lies within the plane of the sanitary napkin 20 that is generally perpendicular to the longitudinal direction. The sanitary napkin 20 has a longitudinal dimension that runs in the general direction of the principal longitudinal centerline L, and a (typically shorter) transverse dimension that runs in the general direction of the principal transverse centerline T.

FIG. 1 shows that the sanitary napkin 20 has a main body portion 21 with two spaced apart longitudinal edges 22, two spaced apart transverse or end edges (or "ends") 24, and four corners 27, which together form the periphery 26 of the main body portion 21 of the sanitary napkin 20. The main body portion 21 also has two end regions, which are designated first end region 28 and second end region 30. A central region 32 is disposed between the end regions 28 and 30. The end regions 28 and 30 extend outwardly from the edges of the central region 32 about 1/8 to about 1/3 of the length of the main body portion. A detailed description of the central region 32 and the two end regions 28 and 30 is contained in U.S. Patent 4,690,680 issued to Higgins on September 1, 1987. The sanitary napkin 20 has a longitudinal central region 23 disposed along the length of at least a portion of the principal longitudinal centerline L (and preferably centered about the same).

The sanitary napkin 20 (or main body portion thereof) can be of any thickness, including relatively thick, relatively thin, or even very thin. The embodiment of the sanitary napkin 20 shown in Figures 1-3 of the drawings is intended to be an example of a relatively thin sanitary napkin (having a caliper of less than or equal to about 5 mm, more preferably less than or equal to about 4 mm), and preferably is an "ultra-thin" sanitary napkin. It should be understood, however, when viewing these figures the number of layers of material shown cause the sanitary napkin 20 to appear much thicker than it actually is. An "ultra-thin" sanitary napkin 20 as described in U.S. Patents 4,950,264 and 5,009,653 issued to Osborn preferably has a caliper of less than about 3 millimeters. The thin sanitary napkin 20 shown should also be preferably relatively flexible, so that it is comfortable for the wearer.

In other embodiments, the longitudinal central region 23 of the sanitary napkin 20 may be provided with an absorbent hump or some other type of raised region or structure (such as those described in the references cited in Section 6 herein) so that a portion of the sanitary napkin has a relatively high caliper, and the surrounding regions 25 have a lesser caliper. Preferably, the surrounding regions, and in particular the end regions, have a caliper of less than or equal to that of the thin sanitary napkins described above. While the longitudinal central region 23 is shown in Fig. 1 as a rectangular strip that extends the length of the sanitary napkin, it should be understood that the raised region, and, thus, the longitudinal central region 23 may be of many other shapes. In such a case, the boundaries of the longitudinal central region 23 will ordinarily coincide with the perimeter defined by the base of the raised region. (The perimeter of the raised region is located where the elevation of the body surface 20A changes due to the presence of the raised region.) The size and shape of longitudinal central region 23 and the surrounding regions 25, therefore, depend on the plan view dimensions of the raised region.

The surrounding regions 25 are the significant absorbent portions of the sanitary napkin that lie outboard of the longitudinal central region 23. The term "outboard" means positioned away from the intersection of the principal longitudinal and transverse centerlines, L and T. The surrounding regions 25 may be referred to as "longitudinal side regions" (or "side regions") 25 because they will typically lie at least transversely outboard of the raised region so that they are on both longitudinal sides of the raised region and the sanitary napkin (hence the name "longitudinal side regions"). The surrounding regions 25 can, however, lie outboard of the longitudinal central region 23 in either a longitudinal direction (i.e., outside the ends of the raised region in the end regions 28 and 30), a transverse direction (i.e., outside the longitudinal edges of the raised region, for instance, if the raised region runs the entire length of the sanitary napkin), or both. The surrounding regions 25, thus, need not completely surround all sides of the raised region.

FIG. 3 shows the individual components of the sanitary napkin 20 of the present invention (without any type of raised structure). The sanitary napkin shown in FIG. 3 generally comprises at least three primary components. These include a liquid pervious topsheet 38, a liquid impervious backsheet 40, and an absorbent core 42 positioned between the topsheet 38 and the backsheet 40. The preferred embodiment shown in FIGS. 1-3 also comprises a less extensible element 44 at least partially located in the central region 32 that deflects in response to stretching (and preferably lifts to provide improved body contact).

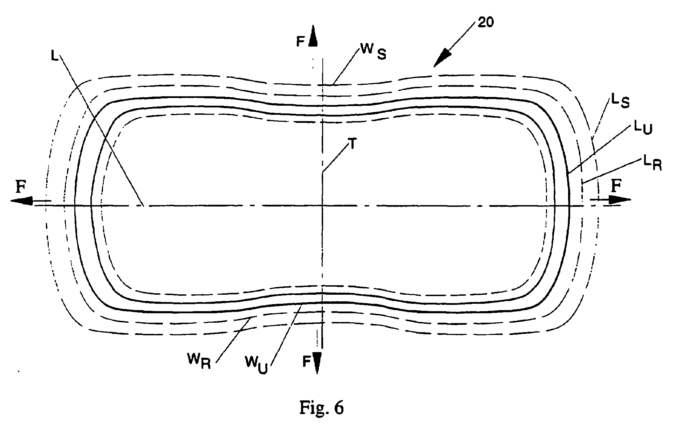

The sanitary napkin may be comprised of several of the basic components described in PCT Publication Nos. WO 93/01785 and 93/01786, and is preferably comprised of one or more extensible components, and more preferably, is comprised of all extensible components (with the exception of the less extensible element), and, thus, has an overall extensibility. The extensibility of the sanitary napkin 20 is shown in a simplified fashion in FIG. 6. The term "extensible", as used herein refers to articles that can increase in at least one of their dimensions in the x-y plane. The x-y plane is a plane generally parallel to the faces of the sanitary napkin 20. The term extensible includes articles that are stretchable and elastically stretchable (defined below). The sanitary napkin 20 shown in Figures 1-5 is preferably extensible both in length and width. In its most preferred embodiments, the sanitary napkin is extensible in all directions in the t-y plane. The sanitary napkin 20, in other embodiments however, may be generally inextensible, only extensible in one of these directions, or extensible in the same direction between the longitudinal and transverse directions. Preferably, the sanitary napkin 20 is extensible at least in the longitudinal direction.

The sanitary napkin 20 may in some preferred embodiments, in addition to being extensible, also be stretchable. The term "stretchable", as used herein, refers to articles that are extensible when stretching forces are applied to the article and offer some resistance to stretching. More preferably still, the sanitary napkin 20 may be elastically stretchable.

The terms "elastically stretchable" or "elastically extensible" are intended to be synonymous. These terms, as used herein, mean that when the stretching forces are removed, the sanitary napkin will tend to return toward its unextended or unstretched (or "original" dimensions). The sanitary napkin 20 need not return all the way to its unstretched dimensions, however, It may, as shown in Figure 6, return to relaxed dimensions (such as $L_R$ and $W_R$) between its unstretched dimensions and extended (or stretched dimensions) $L_S$ and $W_S$. Making the sanitary napkin elastically stretchable will reduce the undesirable tendency of the sanitary napkin to gather longitudinally inward (i.e., bunch longitudinally and become sloppy) when forces which tend to stretch the sanitary napkin are removed such as when the wearer's panties contract.

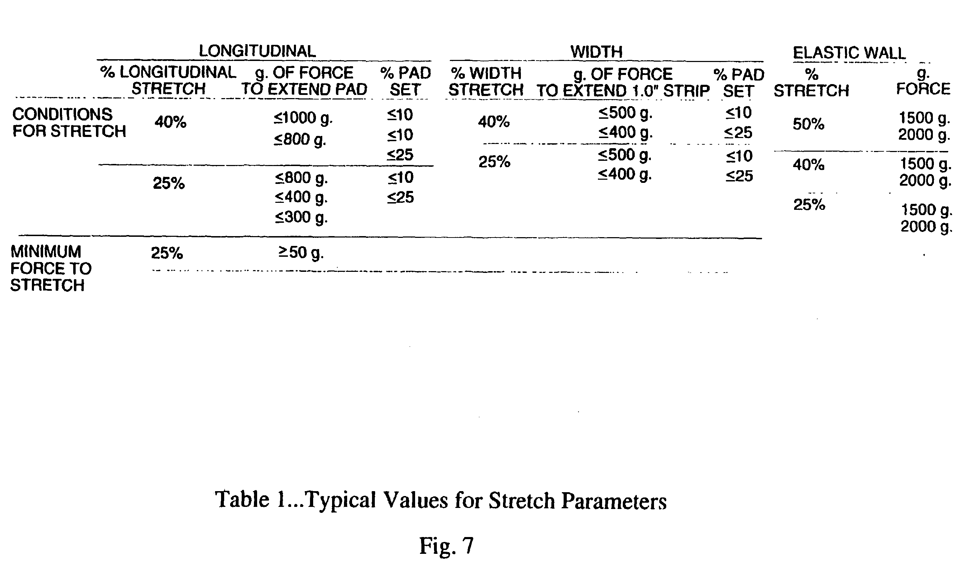

The preferred sanitary napkin 20 embodiment shown in Figs. 1-5 is preferably extensible in the amounts described in PCT Publication Nos. WO 93/01785 and WO 93/01786. To summarize the same, the sanitary napkin is preferably capable of extending about 5% to less than about 50%, more preferably between about 10% and about 40%, and most preferably between about 25% and about 40% under the forces associated with wearing the sanitary napkin in a pair of panties. Preferably, the sanitary napkin is capable of such extension under forces of between about 50 - 100 grams to about 1,000 - 1,500 grams, more preferably under forces of between about 250 grams and about 800 grams. Other preferred amounts of extensibility are set forth in Table 1 (FIG. 7) The sanitary napkin of the present invention can also be provided with any of the other features of the sanitary napkins described in the above publications including, a structure that provides a "force wall" to prevent elongation past a certain amount without substantial increases in the amount of force applied to the sanitary napkin.

In addition, in some especially preferred embodiments described in greater detail herein, various embodiments of the sanitary napkin 20 are capable of smaller amounts extension under forces at the low end of the broadest range set forth above (e.g., forces in the range of about 100 - 200 grams). For instance, in such embodiments, the sanitary napkin is preferably capable of extending about 2.5%, more preferably about 3% at 100 grams force, and about 5%, more preferably about 7.5% at 200 grams force. In absorbent articles with such small amounts of extensibility under low forces, the force wall may also occur at low elongations, such as about 5% elongation, but may occur at elongations up to about 50% elongation.

Further, in embodiments of the sanitary napkin (or other absorbent article) of the present invention which are extensible, the sanitary napkin is extensible in at least the same amounts and under the same forces as the wearer's panties (or other undergarments) so that the panties control the extensibility of the sanitary napkin during wear. In other words, the sanitary napkin preferably has a modulus of elasticity that is close to, and preferably less than or equal to that of the undergarment in which it is placed. For example, if the undergarment requires a force to extend about 5% (or about 10%), the sanitary napkin (that is, the main body portion thereof) requires a force to extend the same amount that is less than or equal to about 1.2 times, preferably less than or equal to about 1 times the force required to extend the undergarment. The force required to extend the crotch region of a typical North American-type woman's panty in the transverse direction (at the narrowest point of the same) is about 135 g/cm. The force required to extend the portions of the back panel of such a panty where the second end region of the sanitary napkin might lie in the longitudinal direction is about 165 g/cm. A typical force to elongate the panty elastics of a North American cotton panty is about 135 g/in. Elastic forces for other types of panties or undergarments may be somewhat higher. The main body portion of the sanitary napkin of the present invention is preferably also extensible at such forces that it can be extended by hand by a consumer in order to increase the absorbent surface area of the napkin at least about 5% without destruction of the main body portion.

Several additional matters should be noted regarding extensibility of the sanitary napkin. The extensibility referred to herein is distinct from mere wrinkling or unfolding of an absorbent article, as well as the straightening of a product curved by elasticity (or by other means), and that any lengthening resulting from such actions is not considered to involve extension of an absorbent article in the X-Y plane. In addition, it is within the scope of the present invention for an absorbent article to have portions or regions that are extensible and portions or regions which are relatively inextensible. If desired, if the properties of such portions of an absorbent article fall within any of the ranges and limits specified herein, the aforementioned ranges and limits can be equally applicable to only portions of an absorbent article, and may be described as such in the appended Claims.

2. The Individual Components of the Sanitary Napkin and the Assembly of the Same.

The individual components which may be suitable for the various embodiments of the sanitary napkin 20 of the present invention will now be looked at in greater detail with reference to FIGS. 1-3.

A. The Topsheet

(1) General Characteristics of Preferred Topsheet

Materials

The topsheet 38 comprises a first liquid pervious component. When the sanitary napkin 20 is in use, the topsheet 38 is in close proximity to the skin of the user. The topsheet 38 may be extensible or inextensible depending on the type of absorbent article it is used with. The topsheet 38 used in the embodiment shown in FIGS. 1-5 is preferably extensible, more preferably elastically extensible, and as compliant, soft feeling, and non-irritating to the user's skin as possible. The topsheet 38 should further exhibit good strikethrough and a reduced tendency to rewet, permitting bodily discharges to rapidly penetrate it and flow toward the core 42, but not allowing such discharges to flow back through the topsheet 38 to the skin of the wearer.

The topsheet 38 has two sides (or faces or surfaces), including a body-facing side 38A and a garment-facing side (or core-facing side) 38B. The body-facing side 38A of the topsheet 38 generally forms at least a portion of the body-contacting surface ("body surface") 20A of the sanitary napkin 20 The topsheet 38 has two longitudinal edges 38C and two end edges 38D

(A similar numbering system applies to the other components of the sanitary napkin. That is, the side of the component facing the wearer's body can be designated by the number of the component and a reference letter "A". The side facing the wearer's undergarments can be designated by the number of the component and the letter "B". The side and end edges can be designated by the number of the component and the reference letters "C" and "D", respectively.)

A suitable topsheet 38 may be manufactured from a wide range of materials including, but not limited to woven and nonwoven materials, apertured formed thermoplastic films, apertured plastic films, hydro-formed films, porous foams, reticulated foams, reticulated thermoplastic films, and thermoplastic scrims. Suitable woven and nonwoven materials can be comprised of natural fibers (e.g., wood or cotton fibers), synthetic or modified natural fibers (e.g., polymeric fibers, such as polyester, polypropylene fibers, and polyethylene, or polyvinylalcohol, starch base resins, polyurethanes, cellulose esters, nylon, and rayon fibers) or from a combination of natural and synthetic fibers. When the topsheet 38 comprises a nonwoven web, the web may be spunbonded, carded, wet-laid, meltblown, hydroentangled, combinations of the above, or the like.

Apertured films are generally preferred for the topsheet 38 because they are pervious to liquids and, if properly apertured, have a reduced tendency to allow liquids to pass back through and rewet the wearer's skin. Suitable apertured films are described in U.S. Patent 3,929,135 issued to Thompson on December 30, 1975, U S. Patent 4,324,426 issued to Mullane et al. on April 13, 1982, U.S. Patent 4,342,314 issued to Radel et al. on August 3, 1982, U.S. Patent 4,463,045 issued to Ahr, et al. on July 31, 1984, and U.S. Patent 5,006,394 issued to Baird on April 9, 1991. A particularly suitable topsheet 38 is made in accordance with U.S. Patent 4,342,314 issued to Radel, et al. and U.S. Patent 4,463,045 issued to Ahr, et al. A topsheet 38 made of model X-3265 or model P1552 apertured formed film sold by Tredegar Corporation of Terre Haute, Indiana has been found to work well.

In embodiments in which the topsheet is extensible, the topsheet 38 preferably has at least some portion with a secant modulus in the longitudinal direction of greater than about 0.1 g/cm/% to about 6 (g/cm)/% at 25% elongation. The term "secant modulus", as used herein, refers to the slope of a straight line that is drawn from the origin through a particular point on a stress-strain curve. The topsheet preferably also has at least some portion with a combined secant modulii in longitudinal direction and transverse direction is such that the geometric mean (or square root) of the product of the longitudinal and transverse direction secant modulii is less than or equal to about 5 g/cm/%. Such extensible topsheet materials also preferably exhibit a set of greater than or equal to about 10%, more preferably about 15%, and even more preferably about 20%.

(2) Alternative Ways of Providing The Topsheet With Extensibility

The topsheet 38 can be made extensible by performing a mechanical operation, such as pleating, corrugating, or ring rolling on the topsheet material to provide folds in the topsheet that are able to open when the topsheet is stretched. Such a process can be performed on many of the topsheet materials described above. In one preferred embodiment of the present invention, the topsheet 38 is made in accordance with U.S. Patent 4,463,045 and ring rolled to provide it with a degree of longitudinal extensibility. Such a topsheet is described in U.S. Patent 5 364 782 entitled "Polymeric Web Having Deformed Sections Which Provide a Substantially Increased. Elasticity To The Web",

Suitable processes for ring rolling or "pre-corrugating" are described in U.S. Patent 4,107,364 issued to Sisson on August 15, 1978, U.S. Patent 4,834,741 issued to Sabee on May 30, 1989, and in co-pending, commonly assigned U.S. Patent 5167897, U.S. Patent 5156793, and U.S. Patent 5143679.

The fold lines in the corrugations of a ring rolled topsheet should run in the transverse direction so the topsheet is longitudinally extensible. In other embodiments, the fold lines could run in the longitudinal direction, both directions, and/or other directions The topsheet 38 will be extensible in directions perpendicular to the fold lines.

Description of SELF Material

In the particularly preferred embodiment shown in Figs. 1-5, the topsheet 38 comprises an apertured web material with a strainable network which exhibits elastic-like behavior without added elastic materials. A web material with such a strainable network may be referred to herein as a "strainable apertured web material" or, for brevity, as a "strainable web material" or simply as the "web material". (It is noted that these latter two terms may include web materials that are apertured or nonapertured.) This type of material is also referred to herein as a structural elastic-like film or "SELF" material.

The strainable apertured web material can, in a preferred embodiment, comprise an apertured formed polymeric film such as a film which is made in accordance with the aforementioned patents issued to Radel, et al. and Ahr, et al. In one highly preferred embodiment, the starting material for the topsheet comprises an apertured film has an absorbent component integrally formed with it which defines its garment-facing side and that it could be any of the types of materials described in Section 2E below. The following description of the material with the strainable network, will be applicable to both apertured materials and non-apertured materials since the SELF technology is also useful in forming preferred components for the backsheet. For clarity in seeing the other components of the sanitary napkin, only a portion of the topsheet 38 is shown in Fig. 1 as being SELFed.

The strainable web material can be made of a base material that has a relatively low extensibility under the forces the sanitary napkin is normally subjected to when worn. When formed into the strainable web material as described herein, however, the base material, thus formed, will be extensible under these forces. The strainable web material can also be formed into a structure that provides a "force wall" to be created at specific, pre-selected elongations and forces. The strainable web material is preferably comprised substantially of linear low density polyethylene (LLDPE). The strainable web material may also be comprised of other polyolefins such as polyethylenes, including low density polyethylene (LDPE), ultra low density polyethylene (ULDPE), high density polyethylene (HDPE), or polypropylene and blends thereof with the above and other materials. Examples of other suitable polymeric materials which may also be used include, but are not limited to polyester, polyurethanes, compostable or biodegradable polymers, heat shrink polymers, thermoplastic elastomers, and breathable polymeric structures.

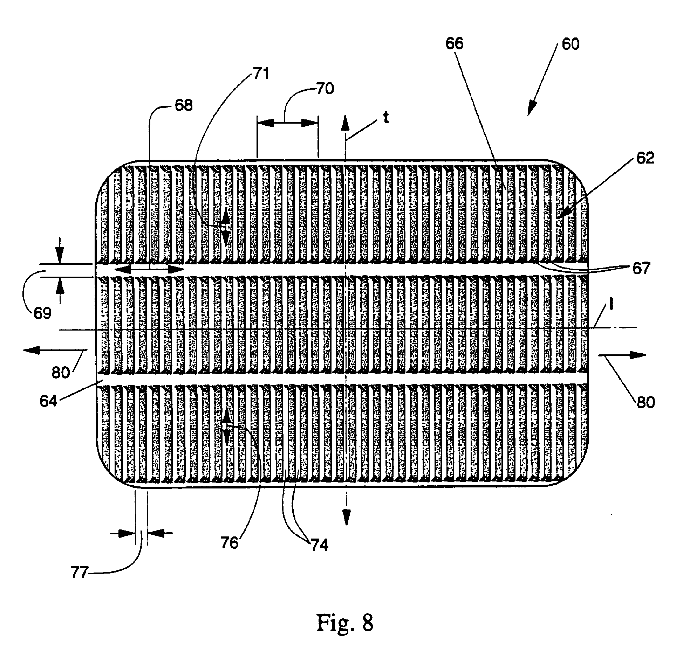

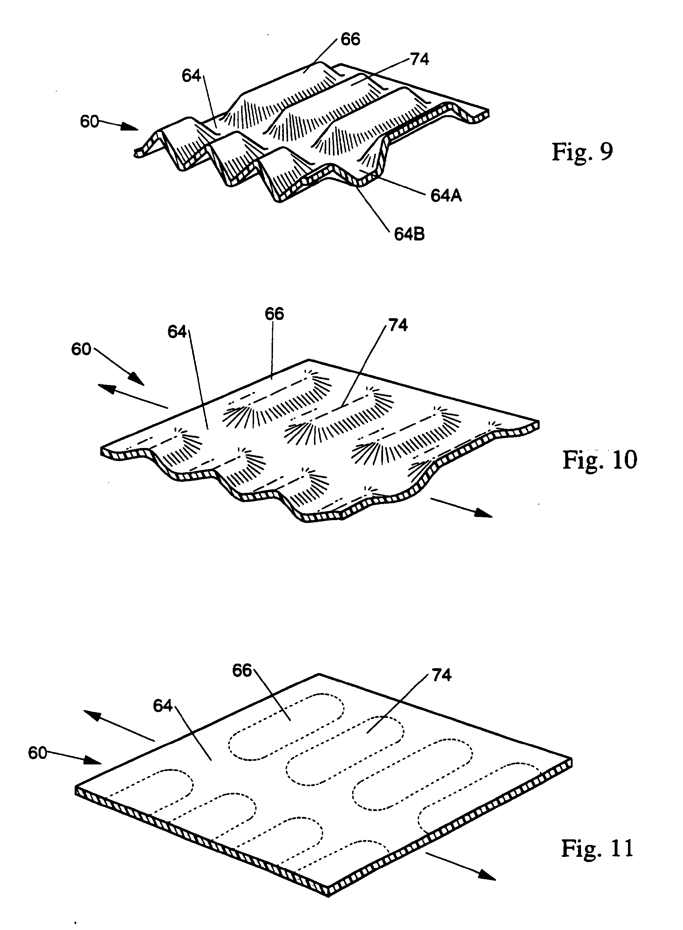

The strainable web material 60 is shown in Figs. 8 and 9 without apertures for simplicity. Figs. 8 and 9 show the strainable web material 60 in its substantially untensioned condition. The strainable web material has two centerlines, a longitudinal centerline, which is also referred to hereinafter as an axis or direction "l" and a transverse or lateral centerline, which is also referred to hereinafter as an axis or direction "t" The lateral centerline "t" is generally perpendicular to the longitudinal centerline "1". In a preferred embodiment, the longitudinal centerline l of the strainable web material is aligned with the principal longitudinal centerline L of the sanitary napkin. In other embodiments, however, the longitudinal centerline 1 of the web material can be oriented in other directions, depending on the direction of extensibility desired.

As shown in Figs. 8 and 9, strainable web material 60 includes a "strainable network" of distinct and dissimilar regions. As used herein, the term "strainable network" refers to an interconnected and interrelated group of regions which are able to be extended to some useful degree in a predetermined direction providing the strainable web material with an elastic-like, relatively low resistive force stage and a relatively high resistive force stage. The strainable network includes at least a first region 64 and a second region 66. The first and second regions each have a first surface and an opposing second surface. In the preferred embodiment shown in Figs. 8 and 9, the strainable network includes a plurality of first regions 64 and a plurality of second regions 66. The first regions 64 have a first axis 68 and a second axis 69, wherein the first axis 68 is preferably longer than the second axis 69. The first axis 68 of the first region 64 is substantially parallel to the longitudinal axis, 1, of the strainable web material 60 while the second axis 69 is substantially parallel to the transverse axis, t, of the strainable web material 60. The second regions 66 have a first axis 70 and a second axis 71. The first axis 70 is substantially parallel to the longitudinal axis of the strainable web material, while the second axis 71 is substantially parallel to the transverse axis of the strainable web material. In the preferred embodiment of Fig. 8, the first regions 64 and the second regions 66 are substantially linear, extending continuously in a direction substantially parallel to the longitudinal axis of the strainable web material.

The first region 64 of the strainable network has an elastic modulus $E1$ and a cross-sectional area $A1$. The second region 66 of the strainable network has a modulus $E2$ and a cross-sectional area $A2$.

In the embodiment illustrated, a portion of the strainable web material 60 has been "formed" such that the entire strainable web material exhibits a controlled resistive force along a predetermined axis when subjected to an applied axial elongation in a direction substantially parallel to such an axis In the case of the embodiment illustrated, the predetermined axis is substantially parallel to the longitudinal axis of the web material. As used herein, the term "formed" refers to the creation of a desired structure or geometry upon the web material that will substantially retain the desired structure or geometry when it is not subjected to any externally applied elongations or forces. As used herein, the term "formed portion" refers to the portion of the material which is comprised of the desired structure or geometry of the strainable network. Suitable methods for forming a material such as the strainable web material described herein include, but are not limited to embossing by mating plates or rolls, thermoforming, high pressure hydraulic forming, or casting

The web material used in the present invention is comprised of a strainable network of contiguous, "distinct", and "dissimilar" regions, wherein the strainable network includes at least a first region and a second region, where the first region has a "surface-pathlength" less than that of the second region. The surface pathlength is measured parallel to a predetermined axis when the material is in an untensioned state. As used herein, the term "surface-pathlength" refers to a measurement along the topographic surface of the region in question in a direction parallel to the predetermined axis. As used herein, the term "distinct" or "dissimilar" when referring to regions, refers to regions within the strainable network having measurably different surface-pathlengths as measured parallel to a predetermined axis while the web material is in an untensioned condition.

In the preferred embodiment shown in Figs. 8 and 9, the first regions 64 comprise a substantially planar region. That is, the material within the first region 64 is in substantially the same condition before and after the formation step undergone by strainable web material. The second regions 66 include a plurality of continuous, interconnected, deformations 74 which extend alternately beyond the plane of both the first and second surfaces (64A and 64B, respectively) of first region 64. In other embodiments, the deformations 74 may extend beyond the plane of only one of either the first or the second surfaces of the first region.

The deformations 74 have a first axis 76 which is substantially parallel to the transverse axis of the web material and a second axis 77 which is substantially parallel to the longitudinal axis of the strainable web material. The first axis 76 of the deformations 74 is at least equal to, and preferably longer than the second axis 77 To enhance the two-stage resistive force versus elongation behavior characteristics of the strainable web material 60 used in the sanitary napkin 20 of the present invention, the ratio of the first axis 76 to the second axis 77 is at least 1.1, and preferably at least 2:1 or greater. In general, the greater this ratio, the more pronounced will be the two-stage resistive force versus elongation characteristic of the web material.

The first region 64 and the second region 66 each have a "projected pathlength". As used herein the term "projected pathlength" refers to length of a region as viewed perpendicularly to the surface of the web material measured parallel to the pre-determined axis (i.e., parallel to the longitudinal axis) of the strainable web material 60. The projected pathlength of the first region 64 and the projected pathlength of the second region 66 are equal to one another.

However, the first region 64 has a surface-pathlength, L1, less than the surface-pathlength, L2, of the second region 66 as measured topographically parallel to the longitudinal axis of the web material while the web material is in an untensioned condition. To enhance the two-stage resistive force versus elongation behavior characteristic of the strainable web material 60, the surface-pathlength of the second region 66 is at least about 15 percent greater than that of the first region, more preferably about 30 percent greater than that of the first region, and most preferably at least about 70 percent greater than that of the first region.

The web material 60 exhibits a modified "Poisson lateral contraction effect" substantially less than that of an otherwise identical unformed web material of the prior art. As used herein, the term "Poisson lateral contraction effect" describes the lateral contraction behavior of a material which is being subjected to an applied elongation. Preferably the Poisson lateral contraction effect of the web material of the present invention is less than about 0.4 when the web is subjected to about 20 percent elongation. Preferably, the web material exhibits a Poisson lateral contraction effect less than about 0.4 when the web material is subjected to about 40, 50, or even 60 percent elongation. More preferably, the Poisson lateral contraction effect is less than about 0.3 when the web material is subjected to 20, 40, 50, or 60 percent elongation.

For the strainable web material, the direction of applied axial elongation, indicated by arrows 80, is substantially perpendicular to the first axis 76 of the deformations 74. (The amount of axial elongation is distance, D.) As the deformations 74 are able to extend in a direction substantially perpendicular to their first axis 76, the direction of applied axial elongation to cause extension in strainable web material is also substantially perpendicular to the first axis 76 of the deformations 74.

While the direction of applied axial elongation, indicated by arrows 80, is substantially perpendicular to the first axis 76 of the deformations 74, an applied axial elongation having a longitudinal component will cause the strainable web material to extend in the direction of applied axial elongation.

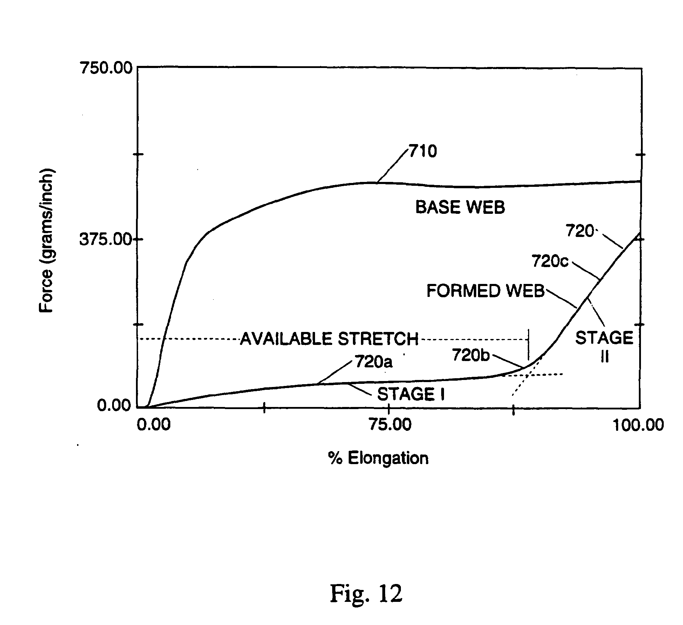

Fig. 12 is an exemplary graph of a resistive force-elongation curve 720 of a formed polymeric web material of the present invention along with a similar curve 710 for a planar, base polymeric film from which the web material is formed. Referring now to the force-elongation curve 720, there is an initial substantially linear, lower force versus elongation stage I designated 720a, a transition zone designated 720b, and a substantially linear stage II designated 720c which displays substantially higher force versus elongation behavior, corresponding to a resistive force wall beyond which the web material may undergo additional permanent deformation.

As seen in Fig. 12 a formed web material having a strainable network exhibits a controlled multi-stage behavior when subjected to an applied elongation in a direction parallel to the longitudinal axis of the web material. The resistive force to the applied elongation is significantly different between stage I (720a) and stage II (720c) of curve 720 as compared to curve 710 which does not exhibit this behavior. Referring now to Fig. 10, as the web material is subjected to an applied axial elongation indicated by arrows 80 in Fig. 8, the first region 64 having the shorter surface-pathlength, L1, provides most of the initial resistive force, P1, to the applied elongation which corresponds to stage I. While in stage I, the deformations 74 in the second region 66 are mostly out of the plane of applied elongation and offer minimal resistance to the applied elongation. In the transition zone between stages I and II, the deformations 74 are becoming aligned with the applied elongation. In stage II, as seen in Fig. 11, the deformations 74 in the second region 66 have become substantially aligned with the plane of applied elongation and begin to resist further elongation. The second region 66 now contributes a second resistive force, P2, to further elongation. The first and second resistive forces to elongation provide a total resistive force, PT, which is greater than the resistive force provided by the first region 64. Accordingly, the general slope of the force-elongation curve in stage II displays the characteristics of a force wall that is significantly greater than the general slope of the force-elongation curve in stage I.

The resistive force P1 is substantially greater than the resistive force P2 when (L1+D) is less than L2 While (L1+D) is less than L2 the first region 64 provides an initial resistive force, P1, generally satisfying the equation:

$$P1 = \frac{(A1*E1*D)}{L1}$$

When (L1+D) is greater than L2 the first and second regions provide a combined total resistive force, PT, to the applied elongation D, generally satisfying the equation:

$$PT = \frac{(A1*E1*D)}{L1} + \frac{(A2*E2*|L1+D-L2|)}{L2}$$

(Where "*" represents a multiplication sign.)

The maximum elongation occurring while in stage I is considered to be the "available stretch" of the web material. The available stretch can be effectively determined by inspection of the force-elongation curve 720, the approximate point at which there is an inflection in the transition zone between stage I and stage II is the percent elongation point of "available stretch". The range of available stretch can be varied from about 10% to 100% or more, this range of elastic-like response is often found to be of interest in disposable absorbent articles, and can be largely controlled by the extent to which surface-pathlength L2 in the second region 66 exceeds surface-pathlength L1 in the first region 64 and the properties of the base film. Significantly higher forces are required to achieve percent elongations in the base film equivalent to those percent elongations in the web 60. The approximate extent of stage I can be controlled as desired by adjusting the pathlengths, L1 and L2 in an untensioned condition. The force-elongation behavior of stage I can be controlled by adjusting the width, thickness, and spacing of first region 64 and the properties of the base film.

When the web material of Fig. 8 is subjected to an applied elongation, the web material exhibits an elastic-like behavior as it extends in the direction of applied elongation and retracts to its substantially untensioned condition once the applied force is removed, unless extended to the point of yielding. The web material is able to undergo multiple applications of applied elongation without losing its ability to substantial recover. Accordingly, the web material is able to retract to its substantially untensioned condition once the applied elongation or force is removed.

While the web material may be easily and reversibly extended in the direction of applied axial elongation, in a direction substantially perpendicular to the first axis 76 of the deformations 74, web material is relatively non-extensible in a direction substantially parallel to the first axis 76 of the deformations 74. The plastic deformation imparted upon the deformations 74 allows the deformations to be extended in one direction, in a direction substantially perpendicular to the first axis of the deformations, while being relatively non-extensible in a direction substantially perpendicular to the direction of extension, in a direction substantially parallel to the first axis of the deformations. In other embodiments, however, the strainable web material 60 can be provided with first regions 64 that extend in different directions to make the strainable web material 60 extensible in more than one direction. For example, the strainable web material 60 can be provided with first regions 64 that extend outward from a center and second regions 66 that are disposed in concentric circles around the center to make the strainable web material 60 extensible in all directions in the x-y plane.

The amount of applied force required to extend the web material is dependent upon the inherent properties of the base material forming the web material and the width and spacing of the undeformed regions 64, with narrower and more widely spaced undeformed regions 64 requiring lower extensional forces to achieve the desired elongation. The first axis 68, (i.e., the length) of the undeformed regions 64 is preferably greater than the second axis 69, (i.e., the width) with a preferred length to width ratio of between 5:1 and 300:1.

The depth and number of deformations 74 can also be varied to control the applied force or elongation required to extend the web material of the present invention. In one preferred embodiment, the deformations are formed by two rigid plates having outer dimensions of 12.7 cm by 30.5 cm by 1.9 cm (5.0" by 12" by 0.75"). On one surface of each plate are a series of meshing teeth which are substantially triangular in cross section and measure 0.76 mm (0.030") at their bases and taper to a vertex with a radius of 0.20 mm (0.008") at the top The centerlines of the teeth are spaced evenly and at 0,76 mm (0.030") increments. On the "toothed" side of one plate, a series of grooves are cut which are parallel to each other and perpendicular to the evenly spaced teeth. These grooves measure 0,79 mm (0.031") wide and are continuous over the entire length of the plate, and are spaced at a distance of 6,35 mm (0.25") on center. These grooves correspond to the undeformed regions of the deformed web of material.

The preferred LLDPE base material is placed between the plates in a hydraulic press having platens larger than the plates to evenly distribute pressure. The plates are compressed under a load of at least 4,000 pounds. The formed web material is then removed from between the plates. The available stretch or elongation is increased if for a given number of deformations, the height or degree of deformation imparted on the deformations is increased. Similarly, the available stretch or elongation is increased if for a given height or degree of deformation, the number or frequency of deformations is increased.

(3) Additional Steps

In preferred embodiments, the topsheet 38 is rendered hydrophilic so that liquids will transfer through the topsheet 38 faster. This will diminish the likelihood that body exudates will flow off the topsheet rather than being drawn through the topsheet and being absorbed by the absorbent core. The topsheet can be rendered hydrophilic by treating it with surfactants. Suitable methods of applying surfactants are described in U.S. Patents 4,950,254 and 5,009,653 issued to Osborn (which include incorporating the surfactant into the polymeric material of a formed film topsheet) as well as treating the surface of the component underlying the topsheet with a surfactant.

In addition, in preferred embodiments, the inner surface 38B of topsheet 38 is secured in contacting relation with an underlying absorbent layer. This contacting relationship results in liquid penetrating topsheet 38 faster. The topsheet 38 may be kept in a contacting relationship with an underlying layer by bonding the topsheet to the underlying layer. However, it is not absolutely necessary to bond the face of the topsheet 38 to the face of the underlying layer. The topsheet 38 can be maintained in contact with an underlying absorbent component by applying adhesives between the topsheet and the underlying component, by entangling the fibers of the underlying layer with the topsheet, by fusing the topsheet 38 to an underlying absorbent layer by a plurality of discrete individual fusion bonds, or by any means known in the art.

B. The Absorbent Core

(1) General Characteristics of Preferred Absorbent Core Materials

The absorbent core 42 is positioned between the topsheet 38 and the backsheet 40. The absorbent core 42 provides the means for absorbing exudates such as menses and other body fluids. The absorbent core 42 need not have an absorbent capacity much greater than the total amount of body fluids anticipated to be absorbed. In fact, the absorbent core 42 may have a capacity that is substantially less than the amount of bodily exudates required to be absorbed if the less extensible element 44 has a capacity sufficient to absorb most or all of such bodily exudates. The absorbent core 42 is generally compressible, conformable, and non-irritating to the user's skin.

In the embodiment shown in Figs. 1-5, the absorbent core 42 is preferably extensible. The absorbent core 42, however, need not be extensible in all embodiments to provide a benefit. For example, a relatively inextensible core can be used in an embodiment in which the topsheet together with an underlying absorbent component (or integral absorbent component) is extensible and the topsheet and such absorbent component are not attached to the face of the core so that they are able to separate from (or "decouple" from) the core. The concept of decoupling (in general) is described in U.S. Patent 5,007,906 issued to Osborn on April 16, 1991. Such an embodiment is useful if the topsheet 38 is capable of extending independently of the absorbent core and any other underlying components which are relatively inextensible In such an embodiment, the topsheet can play a large part in providing the desired body conformity and comfort for the wearer notwithstanding the inextensibility of the underlying components.

The absorbent core 42 can comprise any material used in the art for such purpose including natural materials and synthetic materials. Non-limiting examples of such materials include natural materials such as comminuted wood pulp (which is generally referred to as airfelt), creped cellulose wadding, hydrogel-forming polymer gelling agents, creped tissues or creped nonwovens containing fibers comprised of absorbent or superabsorbent polymers, modified cross-linked cellulose fibers (such as those described in U.S. Patent 5,217,445 issued to Young, et al. on June 8, 1993), capillary channel fibers (that is, fibers having intra-fiber capillary channels such as those described in U.S. Patent 5,200,248 issued to Thompson, et al. on April 6. 1993), absorbent foams (such as those described in U.S. Patent 5,260,345, issued to DesMarais, et al. on November 9, 1993, and U.S. Patent 5,268,224 issued to DesMarais, et al. on December 7, 1993), thermally bonded airlaid materials (such as those materials described in Patent Application WO 95/10996 entitled "Catamenial Absorbent Structures Having Thermally Bonded Layers for Improved Handling of Menstrual Fluids, and Their Use in Catamenial Pads Having Improved Fit and Comfort", absorbent sponges, synthetic staple fibers, polymeric fibers, peat moss, or any equivalent material or combinations of materials.

The polymeric gelling agents listed above may also be referred to as "absorbent gelling materials" ("AGM"), or "superabsorbent materials". Polymeric gelling agents are those materials which, upon contact with liquids such as water or other body liquids, imbibe such liquids and thereby form hydrogels. In this manner, liquids discharged into the absorbent core 42 can be acquired and held by the polymeric gelling agent, thereby providing the articles herein with enhanced absorbent capacity and/or improved liquid retention performance. The polymeric gelling agent which is employed in the absorbent core 42 will generally comprise particles of a substantially water-insoluble, slightly cross-linked, partially neutralized, hydrogel-forming polymer material. The polymeric gelling agent can be in many forms, including in the form of particles, flakes, or fibers.

In one preferred embodiment, the absorbent core 42 is a laminate. The laminate is comprised of a layer of superabsorbent polymer material, such as in the form of particles 41, disposed between two air-laid tissues, first and second tissue layers. The first and second tissue layers provide containment of the superabsorbent polymer material, improve lateral wicking of the absorbed exudates throughout the absorbent core 42 and provide a degree of absorbency. The tissue layers can be comprised of a single tissue web which is folded with the superabsorbent material particles between, or two separate sheets of the same (or different) tissue:

A suitable laminate is a superabsorbent laminate known as WATER-LOCK L-535 available from the Grain Processing Corporation of Muscatine, Iowa (WATER-LOCK registered TM by Grain Processing Corporation). Such superabsorbent laminates are disclosed in U.S. Patent 4,467,012, issued to Pedersen et al. on August 21, 1984, U.S. Patent 4,260,443, issued to Lindsay et al. on April 7, 1981, and U.S. Patent 4.578.068 issued to Kramer, et al. on March 25, 1986.

(2) Alternate Ways of Providing The Absorbent Core With Extensibility

The absorbent core materials described above can be made extensible in many different ways, including by cutting or slitting the same. Figure 1 shows an embodiment in which the topsheet 38 is partially cut away and the absorbent core 42 is a laminate as described above which is slitted or partially slitted with transverse slits for longitudinal extensibility.

The entire plan view area of the absorbent core 42 can be provided with slits of the type shown in Fig. 1, or only a portion of the core can be provided with slits. Preferably, in the embodiment shown in Fig. 1, the entire core is slit. In alternative embodiments, the portions of the absorbent core 42 that lie beyond the less extensible element 44 can be slit and the portion of the core that lie below the less extensible element 44 can be unslit, or vice versa.

In alternative embodiments, the absorbent core 42 can be made extensible by making the same from tissue paper having between 20% and 200% stretch (i.e., capable of extending to an extended dimension that is between about 1.2 and 3 times its unextended dimension). Such tissue sheets can be made by a number of processes. The tissue paper may in one embodiment, be conventionally creped tissue. For example, the tissue paper may be a BOUNTY tissue that is taken directly after it has been creped off of a Yankee dryer before any crepe is pulled out of the tissue. A process for making such a tissue is described in U.S. Patent 5,098,522 issued to Smurkoski, et al. on March 24, 1992.

In alternative embodiments, the absorbent core 42 can be made from a tissue with no or very low initial crepe that is creped after lamination. The creping process in this case could occur by passing the laminate through two matched rolls such that they will yield a corrugated laminate tissue with extensibility in the range of 20% to 200%. The corrugations should be perpendicular to the direction of a desired stretch. Thus, in this and the aforementioned embodiment, a laminate can be formed that comprises two creped nonwoven layers with a superabsorbent polymeric material disposed between the layers of nonwoven material. In still other embodiments, the entire main body portion 21 of the sanitary napkin (or the entire sanitary napkin) can be creped.

In other alternative embodiments, the absorbent core 42 can comprise an absorbent strainable web material (that is, a "SELFed" web). A SELFed absorbent web material can be prepared in a number of ways. The "SELFing" process described above with relation to providing the topsheet with extensibility can be performed on an absorbent starting material such as a tissue layer or tissue laminate or on one of the other types of absorbent material described herein as being suitable for use in the absorbent core 42. It should be understood that while such an operation will provide an extensible absorbent material, when many types of absorbent material (such as tissue layers) are SELFed, they may not be provided with the same type of magnitude of multi-stage behavior in response to applied elongation because such starting materials may initially have very little inherent resistance to elongation. In other alternative embodiments, a material that has greater inherent resistance to elongation can be used as a starting material, and such a material can be combined with an absorbent material either before or after the SELFing process. For instance, the starting material may comprise a laminate of a tissue layer on top of a film (which is either apertured or non-apertured) that is subjected to a SELFing process. An example of a material which is first SELFed and then combined with an absorbent material is a film which has particles of absorbent gelling material adhered to its surface after the film is subjected to a SELFing process. In still other embodiments, the entire main body portion 21 of the sanitary napkin (or the entire sanitary napkin) can be SELFed after it is assembled.

In still other alternative embodiments, rather than comprising an extensible component, the absorbent core 42 can comprise a transversely segmented structure such as is described in P&G Case 4310, U.S. Patent Application Serial No. 07/630,451 filed in the name of Osborn on December 19, 1990, (PCT Patent Publication No. WO 10984, published July 9, 1992).

(3) Additional Steps

The longitudinal and end edges 22 and 24 of the main body portion 21 are preferably sealed to prevent the wicking and expulsion of liquid or any liquid-containing superabsorbent material from the napkin when it is extended. Alternatively, the longitudinal edges 42C and edges 42D of the absorbent core 42 may be sealed rather than sealing the edges of the entire main body portion. The edges of the core 42 may, for example, be wrapped or covered by a tissue layer. In other alternative embodiments, the edges of the tissue may be folded, or otherwise manipulated to prevent the wicking and expulsion of liquid or liquid-containing superabsorbent material particles 41 from the core 42. All permanent seals around the perimeter of the main body portion should not break upon lengthening (i.e., any seal is intended to remain for the duration of the use of the sanitary napkin).

The absorbent core 42 may be made elastically extensible even though it does not have elastic properties of its own. The absorbent core 42 can be made elastically extensible by attaching it to an elastic backsheet or topsheet so that the absorbent core 42 will extend and retract with the elastic topsheet or backsheet.

C The Backsheet

(1) General Characteristics of Preferred Backsheet Materials

The backsheet 40 prevents the exudates absorbed and contained in the absorbent core 42 from wetting articles which contact the sanitary napkin 20 such as pants, pajamas and undergarments. The backsheet 40 should be flexible and impervious to liquids (e.g., menses and/or urine).

The backsheet 40 may comprise a woven or nonwoven material, polymeric films such as thermoplastic films of polyethylene or polypropylene, or composite materials such as a film-coated nonwoven material. Preferably, the backsheet 40 is a thin plastic film, such as a polyethylene film having a thickness of from about 0.012 mm (0.5 mil) to about 0.051 mm (2.0 mils). Exemplary polyethylene films are manufactured by Clopay Corporation of Cincinnati, Ohio, under the designation P18-1401 and by Tredegar Film Products of Terre Haute, Indiana, under the designation XP-39385.

The backsheet 40 is preferably embossed and/or matte finished to provide a more clothlike appearance. Further, the backsheet 40 may permit vapors to escape from the absorbent core 42 (i.e., the backsheet 40 may be breathable) while still preventing exudates from passing through the backsheet 40. Flushable or biodegradable backsheets can also be used, e.g., such as with the pantiliner devices described herein. Another suitable backsheet material is nonwoven/ film laminate described in U.S. Patent 5,007,906 issued to Osborn April 16, 1991.

(2) Alternate Ways of Providing The Backsheet With Extensibility

The backsheet 40 (and any attached overlying component) may have the same extensibility characteristics as the topsheet (e.g., secant modulus, etc.). The backsheet 40 can be made extensible by forming it from an elastomeric film such as the film described in U.S. Patent 4,476,180 issued to Wnuk on October 9, 1984. Such a film is obtained from Exxon Chemical Company of Lake Zurich, IL as Exxon film EXX-500 (formerly EXX-7).

Another preferred extensible backsheet 40 is an extensible adhesive film Formula #198-338 manufactured by the Findley Adhesives Company of Wauwatosa, Wisconsin The Findley adhesive film is a fluid impervious film capable of extending 200 - 300%. The Findley adhesive film is preferred because it is also elastically extensible. At least one side of this film can be used with the adhesive "as is" in the sanitary napkin 20. For example, this side of the adhesive film can be adhered to the garment-facing side 42B of the absorbent core 42. The other side of the adhesive film that forms the garment-facing side 40B of the backsheet 40 may have its adhesive surface at least partially covered (or "blocked" to eliminate its adhesive characteristics). The adhesive defining the body-facing side 40A of the backsheet can also be at least partially blocked. The exposed adhesive can be blocked in a number of suitable ways. These include, but are not limited to attaching a layer of nonadhesive material to cover the exposed adhesive, brushing or sprinkling a powdered material such as talcum powder or corn starch on at least part of the exposed adhesive, and covering the exposed adhesive with a creped nonwoven material and/or a nonwoven material that is oriented in a direction which allows the backsheet to extend (such as with most of its fibers running perpendicular to the desired direction of the stretch). The partial blocking of the exposed adhesive on the garment-facing side 40B of the backsheet 40 can be used with the remaining exposed adhesive to create particular adhesive patterns for fastening the backsheet to the panty covering component.

In still other embodiments, an adhesive film can be created with one side that has adhesive tack, and one side without tack. One suitable adhesive film having these characteristics is a composite structure comprising a nonwoven elastomeric film with a low modulus pressure sensitive adhesive, such as adhesive film Formula #198-338 which is available with a blocking film such as film Formula H2031 from the Findley Adhesives Company. Such materials are further described (and used for other purposes) in U.S. Patent 5,032,120 issued to Freeland, et al. on July 16, 1991, and U.S. Patent 5,037,416 issued to Allen, et al. on August 6, 1991.

In other preferred embodiments, the backsheet 40 may comprise an extensible laminate structure comprised of two or more layers. The laminate can be comprised of layers which are each capable of different extensibility. For instance, a backsheet 40 can comprise a laminate formed of a layer of Findley adhesive film that is covered on one or both sides by an extensible nonwoven web or by an extensible film.

In still other embodiments, the backsheet 40 can be made extensible by performing a mechanical operation, such as pleating, corrugating, ring rolling, or SELFing the backsheet material. In the preferred embodiment shown in Figs 1-5, the backsheet 40 is formed by SELFing one of the exemplary polymeric films described above. Such a SELFed backsheet material is preferred over many of the elastomeric films described above because of its relatively low cost.

D. The Less Extensible Element

The sanitary napkin 20 is also preferably provided with an optional less extensible or non-extensible region or less extensible element (or "insert") 44. Such a less extensible region can comprise a part of one or more of the components of the sanitary napkin, or it can comprise a separate element (e.g. an insert) that is placed between or within the various components of the sanitary napkin.

For example, the less extensible region can comprise part of the absorbent core, or a separate element positioned above the absorbent core, below the absorbent core, or within the absorbent core (e.g., such as between layers of a laminate absorbent core). Figs. 1-5 show an embodiment in which the less extensible region is provided by an insert 44 that is positioned between the topsheet 38 and the absorbent core 42.

The insert 44 is preferably located approximately in the central region 32 of the sanitary napkin 20, but as shown in Fig. 1, the insert 44 may also lie at least partially in the first end region 28 of the sanitary napkin. In the particularly preferred embodiment shown, the insert 44 is activated in response to forces from at least two different sources. The insert 44 preferably both deflects in response to stretching of the rest of the sanitary napkin (and preferably deflects upward in the center or lifts to provide improved body contact) and also preferably deflects upward in response to inward compressive forces exerted by the wearer's thighs during use.

The insert 44 has a body-facing side 44A, a garment-facing side 44B, a pair of longitudinal edges 44C, and a pair of end edges 44D. The insert 44 can generally be any type of component that is less extensible than at least some of the other parts of the main body portion 21 of the sanitary napkin 20. The insert 44 may be relatively inextensible. In other embodiments, the less extensible element may have a degree of extensibility, albeit a lesser degree of extensibility than the other parts of the main body portion 21.

The insert 44 should preferably be flexible. The flexibility of the insert 44 should be enough that the sanitary napkin is comfortable to wear The flexibility of the insert 44 is not unlimited, however, since the less extensible element 44 must resist flexibility enough to maintain the sanitary napkin in the desired in use configurations without collapsing under the forces associated with wearing the napkin. The structure of the insert 44 is preferably rigid enough, however, to allow bowing or buckling to occur when inwardly-oriented lateral compressive forces are applied to the longitudinal edges 44C of the less extensible element. The insert 44 should preferably not collapse inward (i.e., "squash" like a sponge) without providing any z-direction lift in response to the lateral compressive forces exerted on the sanitary napkin 20 during use. The insert 44 can be in the form of a layer of material, or in some other suitable form. The insert 44 will preferably maintain sufficient rigidity when it is both dry and after it has become wet (such as by body exudates).

The insert 44 is preferably also flexure-resistant, reformable, and moisture stable as these terms are defined in the U.S. Patent 5,171,302 issued to Buell. The insert 44 and the sanitary napkin may also be made to assume many of the cross-sectional configurations described in the aforementioned patent. More particularly, the insert described herein has an improved structure which is designed to achieve the desired in-use configurations even more efficiently in thinner, more consumer-friendly executions.

The insert 44 should be laterally compressible under relatively low forces so that the sanitary napkin is comfortable to wear. When worn, sanitary napkin and other related catamenial products are subjected to lateral compression forces. When these compressive forces are released, the sanitary napkin may rebound from its compressed state. The insert 44 may be resilient enough that it moves back to its uncompressed configuration in use after the laterally inward compressive forces exerted by the wearer's thighs are removed. Alternatively, if the insert is joined to the longitudinal side edges of the absorbent core, the insert may passively expand with the panty crotch when the compressive forces exerted by the wearer's legs are removed. This ensures that the insert 44 will enable the sanitary napkin 20 to cover a large portion of the wearer's panties during use and to minimize lateral leakage of exudates around the sides of the sanitary napkin which leads to staining of the wearer's panties (which may be referred to as "side soiling"). The manner in which the sanitary napkin reacts to these compressive forces is also important since it affects the visual appearance of the sanitary napkin after use.

The insert 44 may be made from any suitable material. The material should be soft, flexible, and absorbent, but rigid enough to bow or buckle. The insert 44 may be made from many of the basic types of absorbent core materials specified herein. These core materials, however, preferably should not be subjected to any process (such as SELFing, ring rolling, pleating, corrugating, or slitting) to provide the material with extensibility. FIGS. 1-5 show an embodiment in which the insert 44 comprises an unslit absorbent laminate. The insert 44 is preferably also designed to provide for controlled buckling such as by folding it to create a medial longitudinally-oriented fold line in the same.

The insert 44 may be simply placed on top of the core 42 and held in place by fitting snugly against the surrounding components of the sanitary napkin 20. In the embodiment shown in FIGS. 1-5, the insert 44 is affixed at a single point 46 on each of its longitudinal side edges.

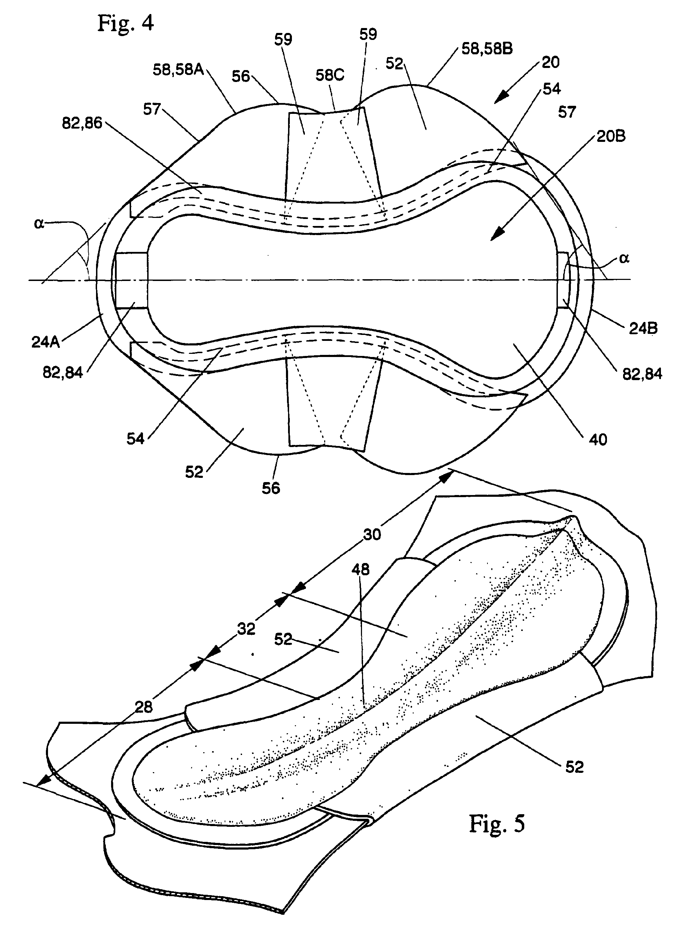

When the sanitary napkin 20 is elongated, the central region 32 of the napkin narrows. This causes the insert 44 to bow or buckle and form a ridge 48 (shown in FIG. 5) along the principal longitudinal centerline L of the sanitary napkin 20.

The insert 44 can be formed from many of the types of materials used in the various components of the sanitary napkin (such as the types of materials used in the absorbent core and as the backsheet, or combinations thereof). For instance, the insert can be formed from webs or laminates of absorbent material (with or without absorbent gelling materials), or from impervious materials. Examples of suitable absorbent materials include webs of cross-linked cellulosic fibers and meltblown webs. It is preferred, however, to form the insert from an absorbent material, and a material such as a thermally bonded airlaid web (which may be referred to herein as "TBAL" for brevity) that has some resiliency. Examples of some suitable nonabsorbent materials which may be placed under the absorbent core (or slit, or otherwise adapted to provide liquid passageways to the core) include thermoplastic polyethylene, polypropylene, synthetic foams, films or suitable blends of the types of materials described herein. On preferred foam material for use in the insert is a polyethylene foam known as VOLARA 2a obtained from Voltek Corp., Lawrence. Mass.

The insert 44 preferably contains at least some thermoplastic material. Upon melting, at least a portion of this thermoplastic material migrates to the intersections of the fibers, typically due to interfiber capillary gradients. These intersections become bond sites for the thermoplastic material. When cooled, the thermoplastic material at these intersections solidifies to form the bond sites that hold the web or matrix of fibers. Bonding at these fiber intersections increases the overall compressive modulus and strength of the resulting matrix. This matrix preferably contains from about 10 to 90% cellulosic fibers and from about 10 to about 90% thermoplastic fibers or material.

In a particularly preferred embodiment, the insert 44 is made of a thermally bonded absorbent material fabricated from a blend of cellulose and synthetic fibers. Such a preferred material for the insert 44 is described in the aforementioned Patent Application WO 95/10996, entitled, "Catamenial Absorbent Structures Having Thermally Bonded Layers for Improved Handling of Menstrual Fluids, and Their Use in Sanitary napkins Having Improved Fit and Comfort". Such a material is preferred because, unlike many foam materials, it is absorbent, and it has inherent resiliency, and it can be formed into resilient structures without having cells that are crushed in the process (which often happens with foam materials).

Such particularly preferred thermally bonded absorbent material is obtained as DANWEB material #1079-2338 and 2339 from Dan Web of Aarhus, Denmark. DANWEB material #1079-2338 comprises a homogeneous blend of about 70% Flint River fluff (cellulose), 15% DANAKLON ES-C 1.7 dtex x 6 mm bicomponent fibers, and about 15% Nalco 1180 absorbent gelling material particles. DANWEB material #1079-2338 is formed into a web having a basis weight of about 152 grams/m$^2$, a caliper of about 1.2 mm measured under a load of (0.2 psi) 1.38 kPa and a density of about 0.13 g/cc. DANWEB material #1079-2339 comprises a two layer composite. The first layer has the same composition as material #1079-2338. The second layer comprises a homogeneous blend of about 85% Flint River fluff and 15% DANAKLON ES-C 1.7 dtex x 6 mm bicomponent fibers. The composite web has a basis weight of about 290 grams/m$^2$, a caliper of about 2.3 mm, and a density of about 0.13 g/cc.

The insert 44 can also comprise a laminate of a thermally bonded absorbent material and other materials. One particularly preferred laminate comprises a laminate of DANWEB #1079-2338 material and one or more layers of an 18 g/yd$^2$ (21.5 g/m$^2$) spunbonded polypropylene nonwoven material known as CELESTRA available from Fiberweb, North America of Simpsonville, SC, which is then embossed with the pattern described in U.S. Patent 4,781,710 issued to Megison, et al. on November 1, 1988, and referred to internally at P&G as P-9. Both layers of P-9 material are preferably melted to the back of the DANWEB material.

The thermally bonded airlaid material can be formed by metering an airflow containing the fibers and thermoplastic material, in substantially dry condition, onto a typically horizontally moving wire forming screen. Suitable systems and apparatus for air-laying mixtures of fibers and thermoplastic material are disclosed in, for example, U.S. Patent 4,157,724 (Persson), issued June 12, 1979, and reissued December 25, 1984 as Re. 31,775; U.S. Patent 4,278,113 (Persson), issued July 14, 1981; U.S. Patent 4,264,289 (Day), issued April 28, 1981; U.S. Patent 4,352,649 (Jacobsen et al), issued October 5, 1982; U.S. Patent 4,353,687 (Hosler, et al), issued October 12, 1982; U.S. Patent 4,494,278 (Kroyer, et al), issued January 22, 1985; U.S. Patent 4,627,806 (Johnson), issued December 9, 1986; U.S. Patent 4,650,409 (Nistri, et al), issued March 17, 1987; and U.S. Patent 4,724,980 (Farley), issued February 16, 1988. A particularly desirable system for air-laying mixtures of fibers and thermoplastic material according to the present invention is disclosed in U.S. Patent 4,640,810 (Laursen et al), issued February 3, 1987.

In the preferred embodiment shown, the topsheet 38, backsheet 40, and absorbent core 42 are extensible, and the insert 44 is positioned on top of the absorbent core and is less extensible than these other components. The lamination of the P-9 material on the bottom of the insert 44, in this embodiment provides the insert 44 with a smooth bottom surface (that is, one that has a lower coefficient of friction) so that when the underlying absorbent core 42 is stretched, the insert 44 will be able to more easily slide relative to the core 42. This is believed to improve the ability of the core 42 to stretch and cause this less extensible insert 44 to pop up

In such an embodiment, since the insert 44 has a plastic layer for its bottom surface, it is important for the insert 44 to avoid interfering with the absorbent function of the sanitary napkin. There are several ways this can be done. In one embodiment, the top surface 44A of the insert 44 can comprise sufficient absorbent material that the insert 44 serves as the primary absorbent component of the sanitary napkin and the absorbent core in the remaining portions of the sanitary napkin can serve as a "drop cloth" for exudates that are not deposited on the insert 44. In other versions of such an embodiment, the insert 44 can be made semi-liquid impervious or liquid impervious. For instance, the insert 44 can be formed of a laminate of TBAL material and a single layer of P-9 material and/or the P-9 material is not heated completely until the insert is totally impervious. Alternatively, the insert 44, or the P-9 material may be slitted with slits, or have holes punched in it or a window cut out therein so that liquids may travel through the insert 44 to the underlying absorbent core 42.

E. Other Optional Components

The sanitary napkin 20 of the present invention may be provided with optional additional components.

The sanitary napkin 20 of the present invention can be provided with one or more additional pervious or absorbent portions or layers. The additional pervious or absorbent portions or layers may be an integral part of one of the components of the sanitary napkin, such as the topsheet or absorbent core, or they may be a separate layer positioned between the absorbent core 42 and either the topsheet 38, the backsheet 40, or both.

FIG. 3 shows that in the preferred sanitary napkin embodiment shown in Figs. 1-5, an underlying absorbent portion, such as wicking material (or acquisition material) 50 forms the garment-facing side of the topsheet 38. In the embodiment shown in Figure 3, the acquisition component 50 is a nonwoven material having the same configuration as the absorbent core 42 that is attached to and integrated with the formed film portion of the topsheet and is SELFed along with the formed film prior to the assembly of the sanitary napkin.

The terms "layer" or "web", as used herein to describe the acquisition component, include, but are not limited to single unfolded sheets, folded sheets, strips of material, loose or bonded fibers, multiple layers or laminates of material, or other combinations of such materials The terms layers and webs are thus, not limited to single unfolded layers or sheets of material. Various folded arrangements are described in greater detail in PCT Patent Application Publication No. WO 92/07535 published in the name of Visscher, et al. on May 14, 1992.

The acquisition component 50 serves to improve wicking of exudates over and into the absorbent core 42. There are several reasons why the improved wicking of exudates is important. The improved wicking provides a more even distribution of the exudates throughout the absorbent core. The improved wicking also allows the sanitary napkin 20 of the present invention to be made relatively thin. The acquisition component 50 is capable of dispersing exudates over a large surface area of the absorbent core 42. The acquisition component 50 thus allows the sanitary napkin 20 to absorb relatively large amounts of exudates. Bulky prior art sanitary napkins relied on a high degree of vertical absorption at the point where exudates are initially deposited. Because the absorbent cores of these prior napkins were fairly thick, they could absorb a large volume of exudates while utilizing only a small degree of the surface area or lateral absorption capacity. The thin versions of the sanitary napkins 20 of the present invention may absorb relatively large amounts of exudates because the wicking disperses the exudates over a large surface area of the absorbent core 42 where the exudates can better and faster be vertically absorbed into the absorbent core 42.

The acquisition component 50 may also be used to direct exudates toward particular portions of the core such as the ends of the core 42D. Liquid exudates that are deposited on the core 42 will tend to be distributed radially outward from the place where they are deposited. Since the core 42 of the sanitary napkin 20 is relatively narrow in comparison to its length, liquid exudates will reach the longitudinal edges 42C of the core 42C much sooner than they will reach the ends 42D of the absorbent core. The acquisition component 50 can be used to longitudinally wick and direct exudates toward the ends 42D of the core 42. This more effectively utilizes the capacity of the core, and reduces the possibility of leakage caused by exudates prematurely reaching the longitudinal edges 42C of the core.

The characteristics of the acquisition component 50 are as follows. The acquisition component 50 should be liquid permeable and is preferably hydrophilic. The acquisition component 50 is also preferably compliant, soft feeling, and non-irritating to the user's skin. It can be made from any materials that are capable of dispersing exudates in the preferred manner described above. The materials are preferably also be capable of having the topsheet 38 fused to them. The acquisition component 50 is preferably provided with stretch properties. (If desired, these additional components may be provided with extensibility in any of the manners described herein.)

The fibers or other structural elements comprising the acquisition component 50 may be inherently hydrophilic. Alternatively, they may be treated to render them hydrophilic. Suitable methods for rendering fibers hydrophilic include treating them with a surfactant. The fibers can be treated by spraying the material comprising the acquisition component with a surfactant or immersing the material into the surfactant A more detailed discussion of such a treatment and hydrophilicity is contained in U S Patents 4,988,344 and 4,988,345 issued to Reising, et al. and to Reising, respectively. The hydrophilicity of these fibers allows the acquisition component 50 to draw liquid exudates through the body-facing surface 38A of the topsheet 38 from below.

The acquisition component 50 may be comprised of many of the same materials as the absorbent core. The acquisition component 50 may be comprised of woven or nonwoven materials. These materials may be synthetic, or partially synthetic and partially natural materials. Suitable synthetic fibers include polyester, polypropylene, polyethylene, nylon, viscous rayon fibers, or cellulose acetate, with polyester fibers being preferred. Suitable natural fibers include cotton, cellulose, or other natural fibers. The acquisition component 50 may also be at least partially comprised of cross-linked cellulose fibers, capillary channel fibers and fibrous superabsorbent material such as FIBERSORB manufactured by Camelot of Leominster, MA.

The fibers of the acquisition component 50 may be oriented primarily in a single direction to cause liquid exudates deposited on the acquisition component 50 to preferentially wick and be distributed toward the ends 42D of the absorbent core 42.

The acquisition component 50 may be any suitable size. The acquisition component 50 need not extend the full width of the absorbent core 42. The acquisition component 50 could, for instance, be in the form of a strip.

The acquisition component 50, if nonwoven, can be made by a number of different processes. These include, but are not limited to the following in order of preference from least to most preferred: meltblown, spunbonded, carded, the latter including, in order of preference, thermally-bonded, air-through bonded, powder bonded, latex bonded, solvent bonded, or most preferably, spunlaced. The latter processes are more preferred because it is easier to orient the fibers in a single direction in such processes.

Suitable commercially available products for use as the acquisition component of the topsheet 50 include a 70%/30% rayon/polyester fabric known as SONTARA The SONTARA fabric is described in greater detail in U.S. Patents 4,950,264 and 5,009,653 issued to Osborn. Another material suitable for use as the acquisition component of the topsheet is an air-through bonded nonwoven material comprised of bi-component fibers which is manufactured under the tradename HAVIX S2146 by the Havix Corporation, Gifu-City, Japan (formerly known as Fukamura).

F. <u>Combinations of Topsheet. Backsheet, and Core Materials and Assembly of the Same Into the Main Body Portion of the Sanitary Napkin.</u>

The main body portion 21 of the sanitary napkin 20 of the present invention can be comprised of any combinations of the different topsheet, backsheet, and core materials described herein. The main body portion 21 may, as noted above, be comprised of all extensible components. The main body portion may also be comprised of any of the other types or combinations of extensible or inextensible topsheets, backsheets and absorbent cores that are described in PCT Publication Nos. WO 93/01785 and 93/01786.

The components of the main body portion described above (the topsheet, backsheet, and absorbent core) can be assembled in any suitable manner. In the preferred embodiment shown in Figures 1-3, the components of the main body portion are assembled in a "sandwich" configuration with the components sized so that the edges of the topsheet 38 and backsheet 40 extend outward beyond the edges of the absorbent core 42. The topsheet 38 and backsheet 40 are preferably at least partially peripherally joined using known techniques. As shown in Figure 1, the topsheet 38 is preferably secured to backsheet 40 along a seam, such as seam 90. Seam 90 is preferably liquid impervious. The seam 90 can be formed by any means commonly used in the art for this purpose such as by gluing, crimping, or heat-sealing.

The term "joined", as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element.

The components of the sanitary napkin 20 can be joined together by adhesives, stitching, heat and/or pressure bonds, dynamic mechanical bonds, ultrasonic bonds, intermingling or entanglement of the fibers or other structural elements comprising the components of the sanitary napkin, such as by meltblowing the fibers comprising one component onto another component, extruding one component onto another, or by any other means known in the art. Suitable means for attaching the components of the sanitary napkin are described in U.S. Patent Application Serial No. 07/810,744 filed in the name of Cree, et al. on December 17, 1991 (PCT Patent Publication No WO 93/11725 published on June 24, 1993).

When the main body portion is comprised of extensible components, the components can be joined together in any suitable manner that allows the main body portion to extend. The combining of topsheet and backsheets in extensible products cannot always be accomplished by traditional sealing methods or materials used for nonstretchable products. Bonds formed by traditional heat and pressure methods often do not stretch or are embrittled at the seals so that they easily rip or tear when the product is stretched. This is particularly a problem when the topsheet and the backsheet have different elastic properties, or melting points, or are sufficiently different in composition that sealing is difficult even when these components do not stretch.

Another difficulty is associated with making the perimeter seals liquid impermeable when the extensible components are highly textured or corrugated and receive their extensibility from their corrugations. These corrugated structures often have very small regions that are not sealed due to their surface texture. This may allow the leakage of menses through the corrugations. In addition, the corrugations may be destroyed when the edges are sealed together, destroying their stretch properties.

Backsheets made via SELF technology present unique problems in that the caliper of the backsheet can vary by a factor of 4 over very short distances due to the deep embossing pattern. Conventional thermal bonding may cause brittleness that destroys the stretch leading rips and tears in the edge seal. In addition, differences in the caliper of the SELFed film causes non-uniform heating and melting resulting in holes along the seal or in the backsheet.

There are several approaches that can be used to produce fluid impermeable, stretchable edge seals between the topsheet and the backsheet. In one example, the backsheet 40 comprises a stretchable adhesive film. The core 42 is placed on top of the backsheet 40. The topsheet 38 is then placed on top of the core 42. The portions of the edges of the topsheet 38 that extend outward beyond those of the core 38 are secured to those of the backsheet 40 using the adhesive around the perimeter of the backsheet film. It has been found that such a construction adequately secures the components of the sanitary napkin without further securing the faces of the adjacent components to each other. Although, as noted above, it is often preferred to secure some of the components at their faces, as well.

A liquid impermeable seal with proper stretch and integrity can be formed in an absorbent article comprised of one or more SELFed components when the edges of the product are heat sealed prior to subjecting the edge region to the SELFing process. This can be accomplished in several ways.

One suitable method is to SELF the component parts of the product leaving the edge regions undeformed, then combine the materials, and thermally seal or otherwise crimp the edges. As a separate process, the sealed edges are then "SELFed" resulting in a stretchable product with fluid impermeable, stretchable edges seals than have good integrity.

Alternatively, the process can be accomplished in a single SELFing process. In this case the product is combined with conventional heat seals along the product edges. The total product is the "SELFed" in a die where the SELF patterns for the main body portion are recessed so that the thin edge regions of the pad receive the proper degree of embossing and the thicker central regions of the pad are also adequately embossed resulting in a product that has both extensible edge seals and an extensible main body portion.

It is further possible to have different degrees of embossing for the outer edges of the pad and the interior regions of the pad, so that the pad when stretched deforms in a manner that provides configurations in use that enhance body fit, and/or panty coverage.

The topsheet and the backsheet of a stretchable product can be combined with a stretchable adhesives such as Fuller 4031 or Fuller 2352. However, to prevent the problems of the adhesive bleeding through the topsheet causing the topsheet to have a low level of tack which become very uncomfortable during wear, an elastic barrier film (such as EXX500 made by Exxon) may be bonded on top of the edge seal. This technique is particularly useful when the topography of the materials (e.g., prestretched tri-laminates, ring rolled materials, and materials with fibrous surfaces) is such that bonded areas formed in the same would often leave small pin hole leaks. The elastic barrier film is heated so that its adhesive fills the valleys blocking the flow of fluid. Heat and pressure are required to force the hot molten glue into capillaries or small valleys that would transport fluid. A related approach is to create a product where the backsheet extends outward further than the topsheet and to adhesively bond the topsheet to the backsheet as above and then turn the edges of the backsheet film over the sealed area.

The components of the products can also be prestretched prior to sealing with either adhesive and/or thermal bonds to provide a pleated edge seal pattern (and a softer corrugated surface). This approach has the added advantage of providing a "stop" in the amount of stretch available in the product. This approach also provides a puckered or pleated outer edge seal which may be preferred for a soft edge. In addition, special sealing patterns may be used to help provide stretchable seals, such as, pleated edge seal, a zig-zag seal and intermittent dot patterns. Heat seal bonds can be combined with the adhesive approach.

In other alternate embodiments, a suitable seal can be created by extrusion of a stretchable film (e.g., EVA, EX500) onto the core or onto a nonwoven material covering the back of the core layer where the extruded film extends onto the edges of the core-facing side of the stretchable topsheet.

In a particularly preferred extensible sanitary napkin embodiment shown in FIGS. 1-5, the portions of the topsheet and backsheet at the edges of the topsheet and backsheet are secured together using an extensible adhesive 92 around the perimeter of the sanitary napkin and in addition, a preferred distribution of mechanical bonds 94 in the perimeter area. The extensible adhesive 92 provides an impervious extensible seal around the perimeter of the sanitary napkin. The mechanical bonds 94 (only a portion of which are shown in Fig. 1) provide added strength. The mechanical bonds 94 are arranged in intermittent zones (or regions) of bonded and nonbonded areas.

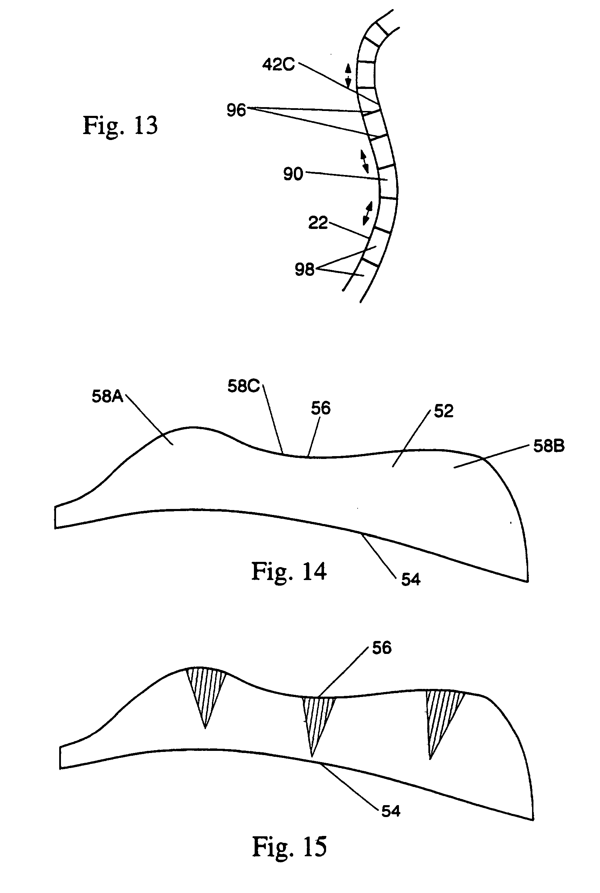

FIG. 13 shows the details of one preferred pattern of bonded and unbonded areas 96 and 98. In the embodiment shown in FIG. 13, the bonded areas comprise a plurality of spaced apart line segments that are approximately perpendicular to the longitudinal edges 42C of the absorbent core 42 at each bond. The bonded areas 96, thus, typically have at least a component that is oriented in the transverse direction.

The bonded areas 96 can, in other embodiments, be in many suitable patterns, including but not limited to geometric shapes, such as circles or squares or other suitable configurations or in continuous or intermittent lines. Regardless of the exact pattern which is used, the bonded areas should be spaced apart in the desired direction of extensibility for the completely assembled absorbent article so they are separated by unbonded areas. The bonded areas 96 are not extensible and the unbonded areas 98 are extensible.

The bonded areas 96 are preferably formed by a heat and pressure process in which the application of temperature and pressure are carefully controlled so that neither the material comprising the topsheet nor the material comprising the backsheet are melted in the process. If either the temperature or the pressure is too high, and the topsheet, the backsheet, or both are melted, the bond formed will become brittle which may result in the topsheet and/or backsheet material tearing at the bond site. A suitable seal of a SELFed polyethylene formed film topsheet and a SELFed polyethylene film backsheet can be formed by a heated plate having bonded areas as shown in Fig. 1 which are approximately 6 mm x 2 mm which are spaced 5 mm apart. The plate is heated to 77°C (170°F) for 5 seconds and the seal is formed using hand pressure.

The above manners of joining the components are preferred for ease of construction. (Other means of uniting the various components can be used.) For instance, the present invention also includes so-called "tube" products. In these products, a liquid pervious cover material (such as topsheet material) can be wrapped completely around the absorbent core and the backsheet, and then the components can be secured together. In alternative arrangements, the topsheet could be wrapped around the core, and the wrapped core could be placed on and secured to the backsheet.

G. Side Wrapping Elements

The sanitary napkin 20 preferably also comprises side wrapping elements (or "panty covering components") 52 located along each longitudinal side edge 22 of the main body portion 21. The side wrapping elements 52 preferably automatically wrap around the elasticized side edges of the wearer's panties when the sanitary napkin is placed in the wearer's panties and the panties are pulled up by the wearer.

The description herein of the side wrapping elements 52 being "located along" (or "associated with") the longitudinal side edges 22 of the main body portion 21 is intended to include embodiments in which the side wrapping elements 52 are integral with the main body portion 21 (and comprise extensions of the components thereof, such as the topsheet and the backsheet) as well as embodiments in which the side wrapping elements 52 comprise separate elements that are attached to the main body portion 21 either at or inboard of the longitudinal side edges 22 of the main body portion 21 If the side wrapping elements 52 comprise separate elements, they may be attached to any portion of the main body portion 21, such as the body-facing side, the garment-facing side, or some point in between these two sides

The side wrapping elements 52 are preferably extensible. The side wrapping elements may be extensible generally in the longitudinal direction, generally in the transverse direction, or in any direction between the longitudinal and transverse directions. Preferably the side wrapping elements are at least extensible in the longitudinal direction.

In the preferred embodiment shown in FIGS. 1-5, the side wrapping elements 52 comprise a pair of separate elements, one of which is attached to the garment-facing side of the napkin along each longitudinal side edge 22 of the main body portion 21. Each side wrapping element preferably comprises a modified crescent-shaped piece or web of extensible material. The crescent-shaped webs each have a proximal edge 54 adjacent to the place where the side wrapping elements are attached to (or extend from) the main body portion 21, and each side wrapping element extends outward to a distal edge 56.

The webs of material (one of which is shown in FIG. 14) are described as being in a "modified" crescent-shape prior to attachment to the main body portion. The inside edge of the web of material is concave inward prior to attachment along the longitudinal side edge of the main body portion where it forms the proximal edge of the side wrapping element. The outside edge of the web of material is convex outward. The outside edge has a shape that defines two rounded portions (or lobes) 58A and 58B in plan view. These rounded portions (referred to together by reference number 58) are located near the ends of the web of material. The rounded portion 58A that is to be attached to the front end of the main body portion may, as shown in FIG. 14, be provided with a different radius of curvature than the rounded portion 58B at the end of the web that is to be attached to the back end of the main body portion. This may be done so the curvature of the distal edge of the side wrapping elements will follow the curvature of the longitudinal side edges of the main body portion when the web is attached to the main body portion. It should be noted that in other embodiments, the curvature of the two rounded portions may be the same.

The rounded portions 58A and 58B on the distal edges of the side wrapping elements are separated by an intermediate section 58C of the distal edge. The intermediate section 58C of the distal edge is disposed closer to the longitudinal centerline of the sanitary napkin than the outermost points of the rounded portions. In the embodiment shown in FIGS. 1-5, the intermediate section 58C is given this configuration by spreading open the inside edge of the crescent-shaped pieces and attaching them so that they follow the curvature of the longitudinal side edges of the main body portion. In the process of attaching the side wrapping elements, at least one, and preferably two, pleats 59 are formed in the side wrapping element 52 where a first portion of the side wrapping element overlaps a second portion of the side wrapping element.

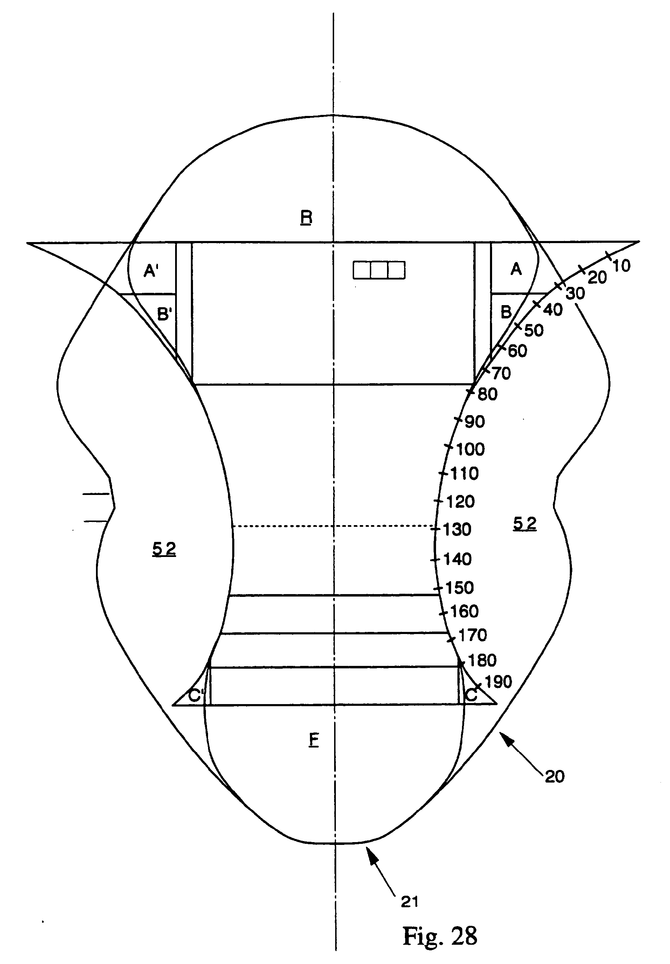

FIG. 4 shows that the end edges 57 of the side wrapping elements 52 preferably form an angle $\alpha$ of less than or equal to about 90° with the longitudinal centerline. The angle $\alpha$ becomes more important as the length of the side wrapping elements 52 increases, particularly when the side wrapping elements are greater than or equal to about 170 mm long (as measured parallel to the longitudinal centerline along the proximal edge of the side wrapping elements). Keeping the angle $\alpha$ under the angles specified above assists the side wrapping elements in folding under the wearer's panties. In particular, the proper angle $\alpha$ prevents the undesirable tendency for the side wrapping elements 52 to flip the ends of the main body portion back on themselves when the sanitary napkin is placed in the wearer's panties. This is particularly important in the areas where the side wrapping elements align with the regions of the panty leg openings that have the greatest curvature (i.e., the smallest radii of curvature). The regions of the panty leg openings that have the greatest curvature (as shown in. Fig. 28) are typically found furthest longitudinally outward from the narrowest portion of the panty crotch.

The side wrapping elements 52 may be provided with any of the properties described in U.S. Patent 5584829 entitled "Absorbent Articles Having Panty Covering Components That Naturally Wrap the Sides of Panties" filed in the name of Lavash, et al. on July 22, 1993, and Patent Application WO 95/07675 entitled "Absorbent Articles Having Panty Covering Components Comprising Extensible Web Materials Which Exhibit Elastic-Like Behavior" filed in the name of Mansfield, et al. on September 20, 1993. Such properties include, but are not limited to a resistance to edge compression of greater than or equal to 5 grams force so that the side wrapping elements 52 are substantial enough that, they will not bunch up when forces are applied by the wearer's panty elastics to the side wrapping elements during wear.

Various alternative embodiments of the side wrapping elements 52 are also possible. For instance, as shown in FIG. 15, in an another embodiment, instead of providing the side wrapping elements 52 with pleats to allow the side wrapping elements to expand when they are folded under the wearer's panties, the side wrapping elements 52 can be provided with ring rolled sections, SELFed sections, or corrugations at various portions, such as at selected portions near the distal edges of the same, preferably at the flap transverse centerline in the regions adjacent the flap transverse centerline, or both.

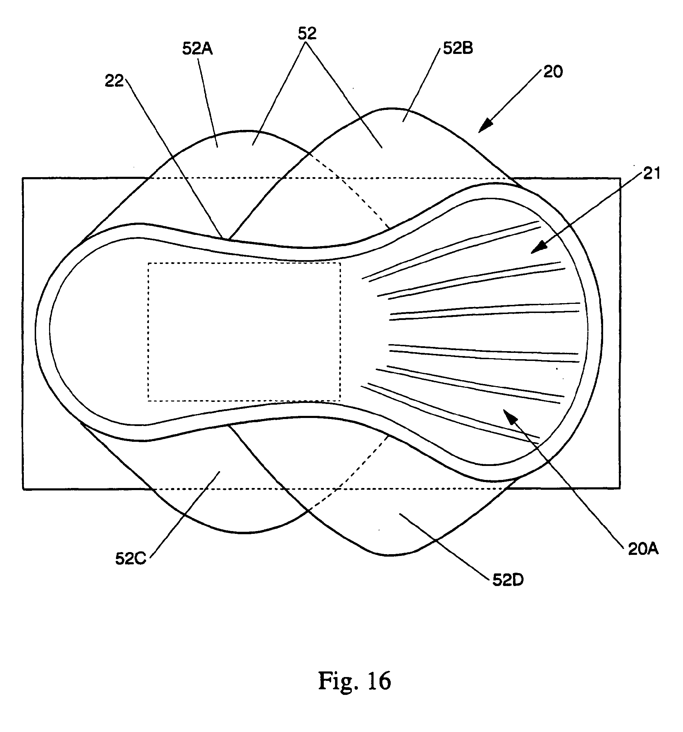

FIG. 16 shows that in another alternative embodiment, the side wrapping elements 52 can comprise more than one overlapping element, such as 52A, 52B, 52C, and 52D. That is, there is more than one overlapping side wrapping element associated with the main body portion 21 along each longitudinal side edge 22 of the main body portion. The overlapping side wrapping elements preferably have at least some portions that overlap at least 5 mm, more preferably at least about 1/4 inch (about 0.6 cm), more preferably at least about 10 mm, more preferably at least about 1/2 inch (about 1.3 cm), more preferably still at least about 3/4 inch (about 2 cm), and most preferably about 1 inch (about 2.5 cm).

The advantage of the alternative embodiment shown in FIG. 16 is that the multiple side wrapping elements such as 52A and 52B can function independently of each other in fitting around the curvature of the panty crotch, yet since they overlap, they will still completely cover the side edges of the panty crotch to prevent soiling of the panty. This embodiment also provides the advantage that the different side wrapping elements, such as 52A and 52B, can each be provided with different properties that allow them to better fit around the wearer's panty crotch. For example, each of the side wrapping elements can be provided with extensibility in different directions (e.g., longitudinal, transverse, or at different angles relative to longitudinal or transverse directions) Such a construction would be difficult to create in a side wrapping element that comprised a single component using conventional technologies.

In less preferred alternative embodiments of the present invention, the sanitary napkin 20 may be provided with flaps that extend outwardly from each longitudinal edge 22 of the sanitary napkin 20. The flaps may be in any suitable configuration Suitable flaps may, for example, be made in accordance with the teachings of U.S Patents 4,589,876, issued May 20, 1986 to Van Tilburg and 4,687,478, issued August 18, 1987 to Van Tilburg, U.S. Patent Application Serial No. 07/769,891 entitled "Absorbent Article Having Flaps and Zones of Differential Extensibility" filed-October 1, 1991 in the name of Lavash, et al. (PCT Publication No. WO 93/06805 published. April 15, 1993), Patent Application CA 2079557 entitled "Absorbent Article Having Inwardly-Folded Pleated Flaps, US 5346486 entitled "Sanitary Napkin Having Laterally Extensible Means for Attachment to the Undergarment of the Wearer", and U.S. Patent 5,267,992 issued to Van Tilburg on December, 1993. Alternatively, the sanitary napkin can be provided with side wrapping elements that have one or more features of the flaps described in the aforementioned patents and patent applications.

H. Fasteners for Attaching the Sanitary Napkin to the Wearer's Panties.

The garment surface 20B of the sanitary napkin 20 and the garment surface of the side wrapping elements 52 may include fasteners for attaching the sanitary napkin to the undergarment of the wearer 82.

FIG 4 shows that in the particularly preferred embodiment shown in the drawings, the sanitary napkin is provided with two end fasteners 84 and perimeter fastener 86, a fastener that is disposed around the perimeter of the main body portion 21, which are adapted to secure the portion of the sanitary napkin underlying the main body portion 21 to the crotch region of an undergarment. The end fasteners 84 are preferably inextensible fasteners, such as inextensible adhesive patches. The end fasteners 84 serve to firmly anchor the ends of the main body portion in the wearer's panties. The perimeter fastener 86 is preferably an extensible fastener, such as an extensible adhesive. The extensibility of the perimeter fastener 86 assists the portions of the sanitary napkin between the ends of the main body portion 21 in extending during use.

The inextensible end fasteners 84 can comprise any adhesive or glue used in the art for such purposes can be used, with pressure-sensitive adhesives being preferred: Suitable adhesives are Century A-305-IV manufactured by the Century Adhesives Corporation, Instant Lock 34-2823 manufactured by the National Starch Company, 3 Sigma 3153 manufactured by 3 Sigma, and Fuller H-2238ZP manufactured by the H.B. Fuller Co. Suitable adhesive fasteners are also described in U.S. Patent 4,917,697.

Suitable extensible adhesives for use as the perimeter fastener 86 include extensible adhesives, per se, and extensible adhesive/backsheet combinations. Any extensible adhesives known in the art can be used. Suitable extensible adhesive/backsheet combinations include, but are not limited to non-extensible adhesive used on an extensible backsheet material such as 3 Sigma 2474 available from Anchor Continental, Inc., 3 Sigma Division, of Covington, Ohio; elastically stretchable adhesive films such as Findley adhesive 198-338, or an elastically stretchable adhesive film known as 3M XPO-0-014 available from the Minnesota Mining and Manufacturing Company of St. Paul, Minnesota; or spray adhesives such as 3M adhesive 1442 on a low modulus elastic film.

Other suitable fastener configurations are shown in PCT International Patent Publication No. WO 92/04000 entitled "Shape and Adhesive Fastening Means for an Absorbent Article" published in the name of Papa, et al. on March 19, 1992; PCT Publication No. WO 93/01783 published in the name of Olsen, et al., and in PCT Publication No. WO 93/01785 published in the name of Osborn, et al.

It should be understood that if it is desired to make the component that forms the garment surface of the sanitary napkin (and any overlying components) extensible in the wearer's panties, the particular adhesive configurations that can be used depend on whether extensible or inextensible adhesives are used. The portion of the sanitary napkin on which extensible adhesives are located will be extensible. Sanitary napkins containing inextensible adhesives will typically only be capable of extension between the inextensible adhesive patches. Therefore, if inextensible adhesives are used, they are preferably applied in intermittent patterns, including but not limited to intermittent dots, intermittent strips, and the like, to permit the sanitary napkin to extend between adhesive patches. If, on the other hand, the adhesive is extensible, the adhesive can be applied in continuous or intermittent patterns in the above configurations (and other configurations). If the adhesives are extensible, they preferably extend approximately the same amounts as the sanitary napkin as set forth in Table 1.

In addition, other types of fasteners can be used instead of, or in addition to adhesives. These other types of fasteners are preferably arranged in patterns similar to those in the patent publications referred to above. Such fasteners include, but are not limited to conventional VELCRO hook material, the fasteners described in: U. S Patent 4,946,527 issued to Battrell on August 7, 1990; U.S. Patents 5,058,247 and 5,116,563 issued to Thomas, et al. on October 22, 1991 and May 26, 1992, respectively; and EPO Patent Application Publication No. 0 381 087 published August 8, 1990; or, high coefficient of friction foams and other high coefficient of friction materials in the same category as those described in U.S. Patent 4,166,464 issued to Korpman, U.S. Patent 4,834,739 issued to Linker, III, et al., and U.S Patent 5,011,480 issued to Gossens, et al. These fasteners may also be made extensible as described in U.S Patent Application Serial No. 07/915,133 (PCT Publication No. 93/01785).

The side wrapping elements 52, as noted above, should wrap and stay without being provided with fasteners to secure the same to the panties. However, embodiments of the present invention may have optional fasteners thereon for additional security. The optional side wrapping element fasteners 45 can be any of the types of fastening materials specified herein The optional side wrapping element fasteners assist the side wrapping elements 52 in staying in position after they are wrapped around the edges of the crotch portion of the panty. The side wrapping element fasteners may be located on the garment surface of side wrapping elements 52, adjacent the distal edges 56 of the side wrapping elements 52, or at various other locations on the side wrapping elements.

3. Properties of the Absorbent Article

The properties of the absorbent article are, unless otherwise stated, described below in terms of those of the preferred sanitary napkin shown in the drawings. The other types of absorbent articles, such as pantiliners and incontinence products, may (but are not required to) have the same or similar properties.

A. Size and Shape

The sanitary napkin has a first end region, a second end region (or "second region"), and a central region disposed between the end regions. The first end region and the central region can be considered to comprise a first region of the sanitary napkin. The sanitary napkin is asymmetrically shaped so that the second end region is larger than the first end region. The ratio of the width of the second end region to the first end region is between greater than about 1:1 up to about 2.7:1 or even 3:1. Preferably, the ratio of the width of the second end region to the first end region is about 1:5:1, more preferably about 1.7:1. Although the sanitary napkin can be worn with either end at the front and rear of the wearer's body, the sanitary napkin is preferably worn by the wearer so that the second end region is placed to the rear of the wearer's body.

The body-facing side 20A of the main body portion 21 of the sanitary napkin preferably defines an absorbent surface area of greater than or equal to: about 60 cm$^2$, and more preferably about 120 cm$^2$, 130 cm$^2$, 140 cm$^2$, 150 cm$^2$, and most preferably about 200 cm$^2$. If the absorbent article is a pantiliner, it preferably has an absorbent surface area of greater than or equal to about 60 cm$^2$ and is less than or equal to about 100 cm$^2$.

The term "absorbent surface area", as used herein, refers to a measurement of the portion of the surface area of the main body portion of the absorbent article which has some underlying absorbent material. Thus, the absorbent surface area of an absorbent article having a perimeter seal in which only topsheet and backsheet material are joined (i.e., no absorbent material in the sealed area), would comprise the total surface area less the portion of the total surface area that was comprised by the perimeter seal.

B. Body Contact

The sanitary napkin 20 of the present invention, as discussed above, preferably conforms closely to the wearer's body during use, and has a large area that is in actual contact with the wearer's body during use.

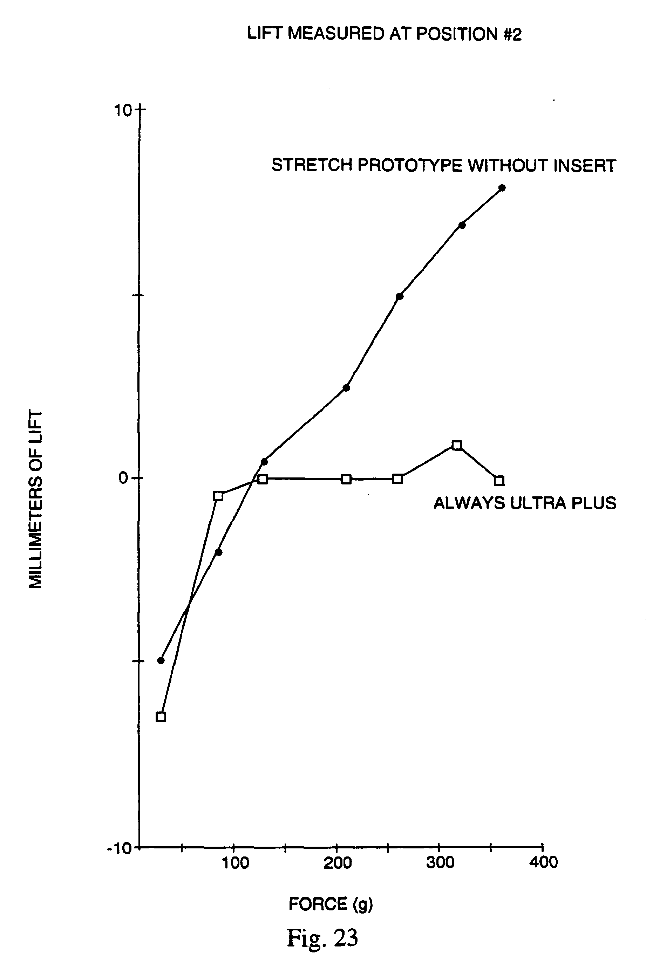

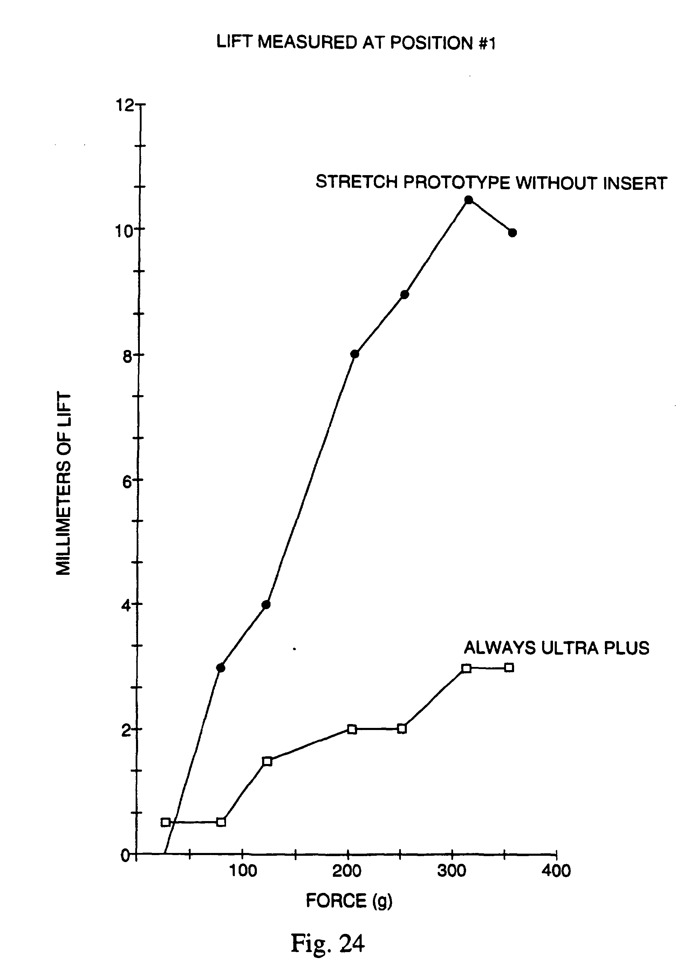

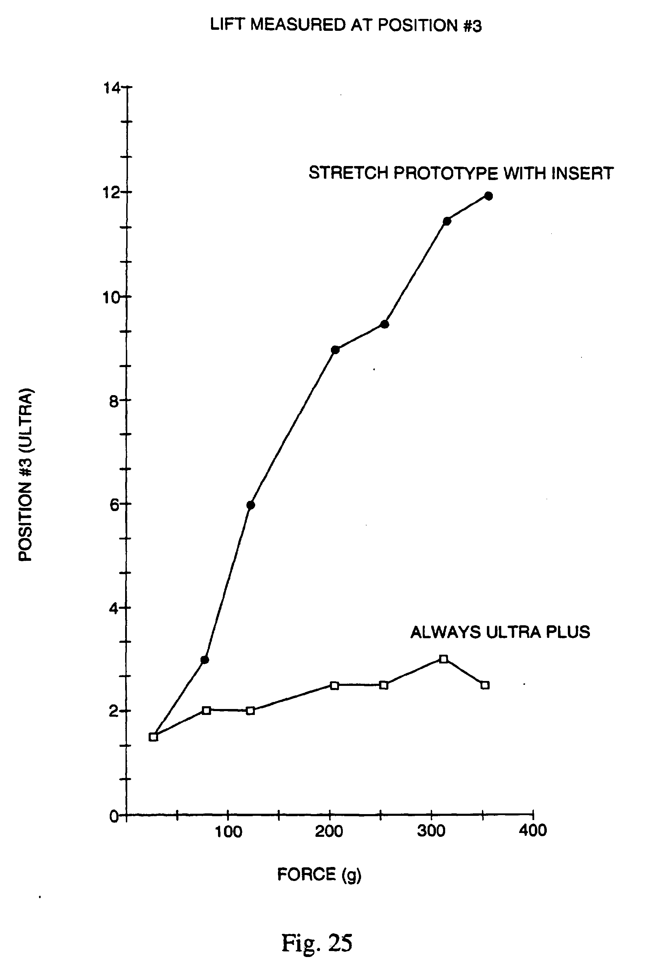

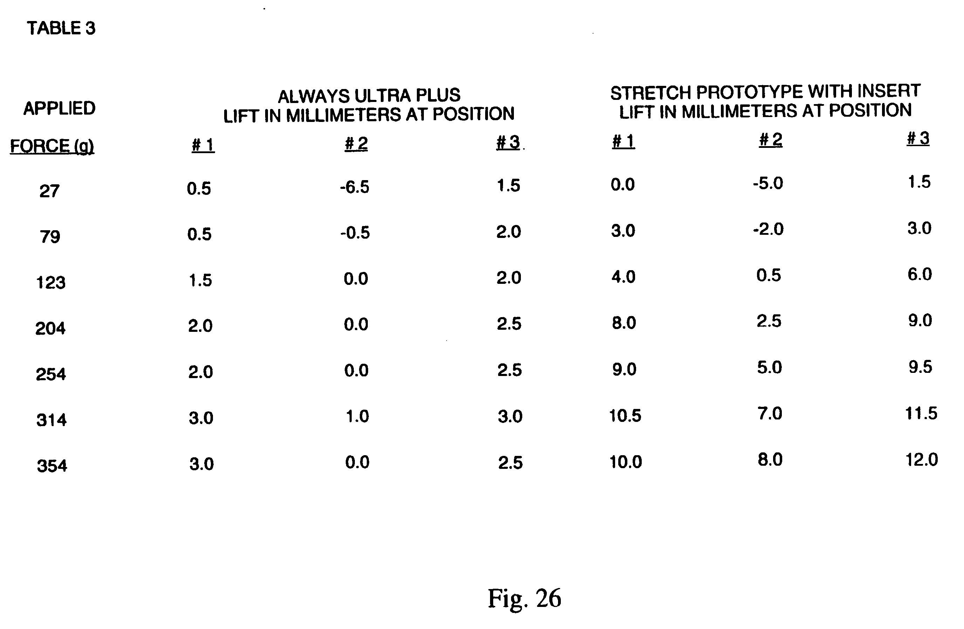

The Lift Test is a laboratory method for approximating the potential of an absorbent article to achieve good body contact. The procedure for measuring Lift is set forth in Section 5 below. The Lift can be measured at several different points in the Lift Test apparatus. These points can be thought of as representing a wearer's introitus, perineum, and the crevice between the wearer's buttocks (or "gluteal groove"). It should be understood, however, that the points on the test apparatus are intended to provide a consistent basis for comparing the Lift of different absorbent articles. The test apparatus and the points thereon are by no means intended to conform identically to a woman wearer's anatomy. FIGS. 23-25 are graphs which depict the Lift at the corresponding first, second, and third positions in the Lift Test apparatus, respectively, of a stretchable sanitary napkin according to the present invention versus a commercially-available ultra-thin sanitary napkin. FIG. 26 is a table of the data in FIGS. 23-25.

The sanitary napkin of the present invention preferably has a Lift at the first position in the Lift Test apparatus of greater than about 3 mm, more preferably greater than or equal to about 4 mm, 5 mm, 6 mm, etc., a Lift at the second position of greater than about 1 mm, more preferably greater than or equal to about 2 mm. and a Lift at the third position of greater than about 3 mm, preferably greater than or equal to about 4 mm, 5 mm, 6 mm, etc.

The extensibility of the topsheet and any underlying absorbent or previous component that is attached to the garment-facing face of the topsheet can also be examined as another laboratory method for approximating the potential for an absorbent article to achieve body contact. The reason for focusing on the extensibility of the topsheet and underlying absorbent component is that it is believed that since these components are closest to the wearer's body, if these components have characteristics such as extensibility it will allow them to extend and conform to the wearer's body, the absorbent article will also likely provide good body contact. The extensibility of the topsheet and any attached underlying component also has the effect of allowing movement between the wearer's body and the body-facing side of the absorbent article which results in transferring the frictional forces from the wearer's skin to the absorbent article, providing improved comfort for the wearer.

The extensible topsheet and any attached underlying component preferably have a relatively low modulus of extensibility in both the longitudinal and transverse directions. The geometric mean of the longitudinal and transverse secant modulus at 25% elongation of the topsheet and any attached component (or components) is preferably less than or equal to about 8 (g/cm)/%, more preferably less than or equal to about 6 (g/cm)/%, still more preferably less than or equal to about 5 (g/cm)/%, still more preferably less than or equal to about 4 (g/cm)/%, and most preferably less than or equal to about 1 (g/cm)/%. When the stretching forces are removed from the topsheet and underlying component, the topsheet and underlying component have less than or equal to about 20% set, and more preferably less than or equal to about 10% set. Similar values are possible for the backsheet and any attached overlying component. The procedures for measuring the extensibility and set of the topsheet and underlying absorbent component and the backsheet and any attached overlying component is set forth in Section 5 below. (Although the procedure is described in terms of only the topsheet for simplicity, it is understood that the same procedure can be performed with respect to the backsheet and any attached overlying components.)

In particularly preferred embodiments, the entire absorbent article is extensible at least about 3% at 100 grams of force and about 7% at 200 grams of force. These forces are low enough to allow the absorbent article to be easily extended by the wearer prior to the placement of the same in his or her undergarments if it is desired to increase the absorbent surface area provided by the absorbent article The extensibility of the absorbent article can be measured in several ways.

One simple way of measuring the extensibility of the absorbent article during wear is simply to lay the absorbent article out flat on a flat surface face down, make two heavy black or red marks which can be seen through a white panty on the backsheet of the absorbent article on opposite ends of the absorbent article (or on opposite sides of the absorbent article) which overlie some absorbent material when the absorbent article is face down, and then to place the article in a wearer's undergarment, and measure the distance between the marks at various times during wear.

Another method for measuring the extensibility of the absorbent article at the low forces described above is to clamp both ends (or both edges) of the absorbent article in the region of the absorbent article which is most extensible in an INSTRON tensile tester using one inch clamps set so that the ends of the jaws of the clamps are 1/2 inch inward from the outer edge of any fastener on the garment side of the absorbent article. Clamping the absorbent article in this manner ensures that the portion of the absorbent article between the places where the absorbent article is affixed to the wearer's undergarments (the extensibility of which portion of the absorbent article is key to the fit and comfort of the absorbent article) will extend under the applied forces. The gauge length is the distance between clamps before the absorbent article stretches. The cross-head speed is set at 1 inch (2.54 cm)/minute. The elongation is continued until a force of 200 grams is reached. The extension of the sample at 100 grams and 200 grams are then plotted versus force. The slope of the stress-strain curve from the origin to the specified forces for various different types of absorbent articles can be taken from the origin through the 100 gram point and through the 200 gram point.

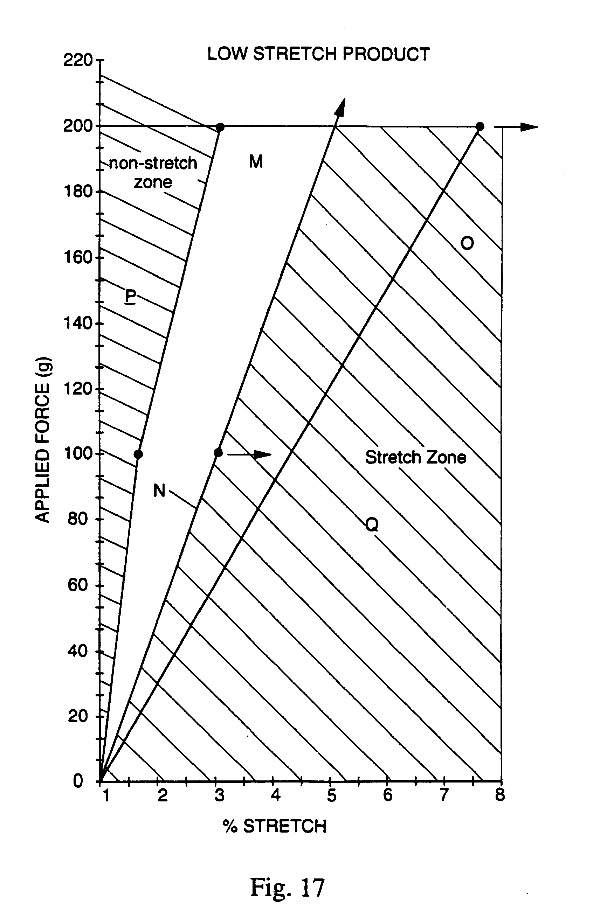

Fig. 17 is a graph which represents some possible results of such a procedure In Fig. 17, the results for several different absorbent articles are depicted. Three lines and two main areas are depicted in Fig. 17. Line M represents the stress-strain curve of the most easily elongated conventional non-stretchable (or minimal stretch) absorbent articles. Line N represents the stress-strain curve of some of the less extensible absorbent articles according to the present invention under low forces Line O is the stress-strain curve for an extensible absorbent article according to the present invention which has a preferred amount of extensibility under low forces

Fig. 17 shows that the sanitary napkin is preferably capable of extending about 2.5%, more preferably about 3% at 100 grams force, and about 5%, more preferably about 7.5% at 200 grams force. In absorbent articles with such small amounts of extensibility under low forces, the force wall may also occur at low elongations, such as about 5% elongation, but may occur at elongations up to about 50% elongation.

The shaded area P in Fig. 17 represents the area of the graph occupied by conventional absorbent articles, that are not generally considered herein to be extensible. The shaded area Q represents the area of the graph occupied by the extensible absorbent articles of the present invention which are extensible under low forces. The unshaded area between areas P and Q represents a transition zone between absorbent articles that are considered herein to generally be extensible for all practical purposes, and extensible absorbent articles according to the present invention.

The methods of measuring extensibility described above are believed to be somewhat simpler alternatives to the methods described in U.S. Patent 5824004. The test methods in US 5824004 are, however, incorporated by reference herein as additional ways of quantifying the desired extensibility of the sanitary napkin of the present invention.

C. Area Coverage

The sanitary napkin, as noted above, is intended to cover a relatively large portion of the wearer's panties when worn and the sanitary napkin is preferably capable of sustaining such area coverage for the duration that the sanitary napkin is worn.

The portion of the wearer's panties covered by the sanitary napkin is often expressed herein in terms of the length of the elasticized side portions of the wearer's panties (or panty elastics) that is covered by the side extensions. This is done for convenience since covering the panty elastics will prevent soiling of the sides of the panties ("side soiling") and it has been found that if the side extensions cover a given length of the panty elastics, the portion of the panties that lies between the panty elastics will ordinarily also be covered by the sanitary napkin.

The side extensions 52 preferably cover a minimum of at least about 30 mm of the wearer's panty elastics and sustain such coverage during use. The side extensions 52 may cover portions of the panty elastics that range in length up to the length of the sanitary napkin when it is in a fully extended condition. In preferred embodiments, the side extensions 52 cover at least about 130 mm of the wearer's panty elastics, and more preferably, the side extensions 52 cover at least about 140, 150, 160, 170, 180, 190, 200 ..., etc. mm of the wearer's panty elastics.

In order to achieve and sustain this area coverage, the side extensions preferably are preferably extensible under lesser forces than the elasticized sides of the panties and in amounts that are equal to or greater than the panty elastics. These properties allow the panty fabric and elastic to extend during wear without constraint. The side extensions 52 preferably have several other properties which contribute to their ability to cover the wearer's panties. The side extensions 52 are preferably more flexible than the fabric of the wearer's panties and preferably have a caliper of less than or equal to about 2 mm.

D. Drapability/Flexibility

(1) Drapability

The drapability of the sanitary napkin is expressed in terms of a measurement known as Flexural Rigidity. Flexural Rigidity is measured by the Drape Test described in Section 5 below. The sanitary napkin preferably has a Flexural Rigidity of greater than or equal to about 100 mg-cm, more preferably greater than or equal to about 300 mg-cm, and less than or equal to about 30,000 mg-cm, more preferably less than or equal to about 25,000 mg-cm, even more preferably less than or equal to about 20,000, more preferably less than or equal to about 10,000 mg-cm, more preferably still less than or equal to about 5,000 mg-cm, and most preferably less than or equal to about 3,000 mg-cm.

If, on the other hand, the absorbent article comprises a pantiliner (for example, one having a sample size of about 5 cm x 15 cm), it preferably has a Flexural Rigidity of greater than or equal to about 150 mg-cm in the transverse direction and greater than or equal to about 400 mg-cm in the longitudinal direction and less than or equal to about 1,600 mg-cm, more preferably between about 500 mg-cm and about 1,600 mg-cm, and most preferably between about 600 mg-cm and about 1,000 mg-cm. Pantiliners may have somewhat different drapability and flexibility ranges than sanitary napkins due to their smaller size.

The drapability (and flexibility) of the absorbent article may also be expressed as a ratio in which the length of the absorbent article including any peripherally sealed area is the denominator. This is useful because it provides a way to quantify the ease with which the absorbent article can be handled when it becomes highly drapable. If the absorbent article is both long and highly drapable, it may become excessively "floppy" and difficult to handle, particularly once the release paper is removed from the adhesive fastener on the garment side of the article. The absorbent article can bend over on itself and need special adjustment by the wearer to place it into her panties. Also too high a flexibility can cause the backsheet of the absorbent article to fold over on itself causing the adhesive fastener on the backsheet to stick to itself and form irreversible, wrinkles in the absorbent article. This may happen when the product is placed in the wearer's panties as well as during wear. These wrinkles can cause discomfort and reduce panty soiling protection because they will make the surface of the absorbent article smaller.

Expressed in this alternative manner, the sanitary napkin described herein preferably has a Flexural Rigidity/ Length of greater than or equal to about 1, more preferably greater than or equal to about 1.5 mg-cm/cm and less than or equal to about 30 mg-cm, more preferably less than or equal, to about 25 mg-cm, more preferably less than or equal to about 20 mg-cm, more preferably still less than or equal to about 15 mg-cm, and most preferably less than or equal to about 10 mg-cm/cm.

(2) <u>Flexibility</u>

The extensible embodiments of the sanitary napkin of the present invention can be somewhat stiffer than the sanitary napkin described in U.S. Patent 5.009.653 issued to Osborn, but preferably have flexure resistances equal to or less than the sanitary napkin described in the Osborn patent.

The flexure-resistance of a sanitary napkin is measured by peak bending stiffness. Peak bending stiffness is determined by a test which is modeled after the ASTM D 4032.82 CIRCULAR BEND PROCEDURE, the procedure being considerably modified and performed as follows. The CIRCULAR BEND PROCEDURE is a simultaneous multi-directional deformation of a material in which one face of a specimen becomes concave and the other face becomes convex. The CIRCULAR BEND PROCEDURE gives a force value related to flexure-resistance, simultaneously averaging stiffness in all directions.

**APPARATUS**

The apparatus necessary for the CIRCULAR BEND PROCEDURE is a modified Circular Bend Stiffness Tester, having the following parts:

A smooth-polished steel plate platform which is $102.0 \times 102.0 \times 6.35$ millimeters having an 18.75 millimeter diameter orifice. The lap edge of the orifice should be at a 45 degree angle to a depth of 4.75 millimeters.

A plunger having an overall length of 72.2 millimeters, a diameter of 6.25 millimeters, a ball nose having a radius of 2.97 millimeters and a needle-point extending 0.88 millimeter therefrom having a 0.33 millimeter base diameter and a point having a radius of less than 0.5 millimeter, the plunger being mounted concentric with the orifice and having equal clearance on all sides. Note that the needle-point is merely to prevent lateral movement of the test specimen during testing. Therefore, if the needle-point significantly adversely affects the test specimen (for example, punctures an inflatable structure), than the needle-point should not be used. The bottom of the plunger should be set well above the top of the orifice plate. From this position, the downward stroke of the ball nose is to the be exact bottom of the plate orifice.

A force-measurement gauge and more specifically an Instron inverted compression load cell. The load cell has a load range of from about 0.0 to about 2000.0 grams.

An actuator, and more specifically the Instron Model No. 1122 having an inverted compression load cell. The Instron 1122 is made by the Instron Engineering Corporation, Canton, Mass.

**NUMBER AND PREPARATION OF SPECIMENS**

In order to perform the procedure for this test, as explained below, five representative sanitary napkins are necessary. From one of the five napkins having, of course, any panty adhesive release paper removed and adhesive blocked, to be tested, some number "Y" of $37.5 \times 37.5$ millimeter test specimens are cut. Specimens having portions in which a topsheet is joined directly to a barrier sheet or which are a laminate of a topsheet, two or less tissue sheets and a barrier sheet, should not be tested. The reason that these specimens are not tested is due to the realization that prior art napkins exist in which a topsheet is joined to a barrier sheet beyond the edges of an absorbent core in the periphery of the napkin, such portions of which are highly flexible. However, the present invention is more concerned with the overall flexibility of the sanitary napkin and not merely the peripheral portions thereof and, therefore, the flexibility of the present invention is with the flexibility of the significant absorbent portions of the sanitary napkin. If any of these significant absorbent portions of the sanitary napkin meet the parameters of this test, then the sanitary napkin satisfies the test. Therefore, a number of different specimens should be tested from each sanitary napkin. Certainly, the structurally most flexible portion of the sanitary napkin should be tested, excluding those portions excluded above. The test specimens should not be folded or bent by the test person, and the handling of specimens must be kept to a minimum and to the edges to avoid affecting, flexural-resistance properties. From the four remaining sanitary napkins, an equal number "Y" of $37.5 \times 37.5$ millimeter specimens, identical to the specimens cut from the first napkin, are cut. Thus, the test person should have "Y" number of sets of five identical specimens.

The procedure for the CIRCULAR BEND PROCEDURE is as follows. The specimens are conditioned by leaving them in a room which is $21 \pm 1°C$, and $50 \pm 2\%$ relative humidity for a period of two hours. The test plate is leveled. The plunger speed is set at 50.0 centimeters per minute per full stroke length. A specimen is centered on the orifice below the plunger such that the body surface 26 of the specimen is facing the plunger and the garment surface 17 of the specimen is facing the platform. Of course, any panty adhesive release paper (if present) is removed, to simulate in-use conditions. Any panty adhesive (if present) should be blocked, using means well known to those skilled in the art, such as glycerin and/or powder, to prevent the specimen from adhering to the platform and an artificially high peak bending stiffness being obtained. If desired, the specimen may be centered over the orifice with the body surface 26 facing the platform and the garment surface 12 facing the plunger to obviate the need for blocking any adhesive which may be present. The indicator zero is checked and adjusted, if necessary. The plunger is actuated. Touching the specimen during the testing should be avoided. The maximum force reading to the nearest gram is recorded. The above steps are repeated until all five of the identical, specimens have been tested.

**CALCULATIONS**

The peak bending stiffness for each specimen is the maximum force reading for that specimen. Remember that "Y" number of sets of five identical specimens were cut. Each set of five identical specimens is tested and the five values received, for that set are averaged. Thus, the test person now has an average value for each of the "Y" sets tested. Remember, if any of the significantly absorbent portions of the sanitary napkin have the requisite flexure-resistance, then the napkin satisfies the parameters of this test. Therefore, the flexure-resistance for a particularly designed sanitary napkin is the greatest flexibility these average peak bending stiffnesses.

The sanitary napkin may have a flexure resistance of less than or equal to about 700 grams, but more preferably has a flexure resistance of less than or equal to about 500 grams, more preferably less than or equal to about 400 grams, and most preferably less than or equal to about 250 grams. In cases where the sanitary napkin is provided with a raised or stiffened region, it preferably has the flexure resistances set forth in the patent applications incorporated by reference in Section 6 below.

The sanitary napkin 20 is, thus, highly drapable and highly flexible and very clothlike. If the sanitary napkin becomes too flexible, the napkin can be provided with optional stiffening zones (as shown in Fig. 16) that give the sanitary napkin, or portions thereof, additional stability to prevent it from becoming difficult to handle, particularly after removal of the release paper. Such optional stiffening zones can be made of foam, high loft strips, stretchable scrim, or other suitable materials. The materials comprising the optional stiffening zones can be extensible or inextensible. If the stiffening materials are inextensible, they can be incorporated into the sanitary napkin in such a manner that permits the surrounding portions of the napkin to stretch. Alternatively, such inextensible stiffening materials may be completely unconnected to other components of the sanitary napkin. If the sanitary napkin contains areas which are inextensible due to the presence of inextensible stiffening materials, in determining the amount that the sanitary napkin is capable of extending, the napkin should be clamped in the test apparatus between such inextensible structures, rather than on such structures so as not to distort the results of the test.

E. <u>Compressive Force and Resiliency</u>

The compressive forces are preferably measured as the amount of force necessary to hold the central portion of the sanitary napkin compressed in the cross direction (i.e., width) in both the dry and wet states. The resiliency of the sanitary napkin can be measured as both the percent recovery of the initial width of the sanitary napkin and the absolute width recovered in the central portion of the sanitary napkin after it has been subjected to cross-directional compression. The absolute width recovered after compression relates to the ability of the sanitary napkin to sufficiently cover the panty to protect it from soiling. The percent recovery of the sanitary napkin after compression has been found to correlate to the visual appearance of the product after use. Sanitary napkin users have evaluated sanitary napkins that have considerably narrower widths at the time of removal (relative to the sanitary napkin before it is worn) as being poor for bunching. Although compressive forces and recoveries are measured in both the dry and wet states, many of the wearer's perceptions as to comfort appear to be formulated as the sanitary napkin is first being worn. This means that compressive forces and recoveries in the dry state may be more relevant to the wearer's perceptions of comfort than are those in the wet state. It has been found that thin sanitary napkins having compressive force values of about 300 g or less, preferably about 200 g or less, in the dry state are considered to be comfortable when worn. Preferably, sanitary napkins according to the present invention have compressive force values in the dry state in the range of from about 50 to about 300 g, and more typically from about 100 to about 200 g.

Most sanitary napkins suffer a loss in their properties for recovery as they become wet. This means the wet state of the sanitary napkin is more critical to sustained area coverage of the panty than is the dry state. Sanitary napkins which have an absolute width after wet compression of at least about 48 mm (preferably, at least about 55 mm) sufficiently cover the panty area to have an impact on the prevention of panty soiling. Typically, sanitary napkins according to the present invention have a width (or compression recovery value) at the center after wet compression in the range of from about 48 to about 70 mm, and are more typically in the range of from about 55 to about 65 mm.

Similarly, since many sanitary napkin users make a visual assessment of the sanitary napkin after it has been worn for a period of time (i.e., when checking or removing the sanitary napkin), the sanitary napkin is more than likely to contain some amount of liquid body exudates. Thus, the wet state is important to the visual appearance of the product after use. Sanitary napkins which recover (at the center) from the wet compressed state at least about 65% (preferably at least about 75%) of their initial width appeal to catamenial users for their visual appearance after use. Sanitary napkins according to the present invention preferably recover after wet compression from at least about 65 to at least about 90%, and more preferably from about 80% to 100% (more typically from about 75 to about 85%) of the initial sanitary napkin width.

The procedure for measuring the compressive force values on the sanitary napkin in the dry state, and the absolute and relative recovery from compression (i.e., resiliency) in the wet state are set forth in the Test Methods section of this specification.

### F. Wet Bunch Recoverability

The sanitary napkin preferably also exhibits a high degree of recovery when wet after it has been bunched (or "Wet Bunch Recovery"). This ensures that the sanitary napkin will continue to cover a relatively large area of the wearer's undergarments after bunching under in use conditions. Wet Bunch Recovery is typically a panty-assisted (or undergarment-assisted) type of recovery in which the sanitary napkin returns toward its original configuration when the panties stretch since the sanitary napkin (and particularly the edges of the same) should remain attached to the wearer's panties and be stretched back with the panties.

Sanitary napkins with an absorbent core comprised of a batt of airfelt that wads up when wetted and slumps inside the topsheet and backsheet display undesirable low Wet Bunch Recovery values and poor area coverage properties. The sanitary napkin of the present invention are distinguishable in that they exhibit high Wet Bunch Recovery values. Preferably the absorbent core of the sanitary napkin of the present invention returns to at least 80%, more preferably at least about 90% of its original width after wet bunching (i.e. Wet Bunch Recovery of greater than or equal to about 80% and 90%, respectively).

### G. Test Capacity and Total Capacity

The sanitary napkin may have the same Test Capacity and Total Capacity of the sanitary napkins described in U.S. Patent 5,009,653 issued to Osborn. Preferably, the sanitary napkin of the present invention has a Total Capacity of greater than or equal to about 10 grams, more preferably greater than or equal to about 20 grams, and most preferably greater than or equal to about 40 grams.

If, on the other hand, the absorbent article comprises a pantiliner, it preferably has a Total Capacity of greater than or equal to about 2 grams and less than or equal to about 10 grams.

The following Examples further illustrate several embodiments of the present invention. The following Examples, however, are not intended to limit the scope of the absorbent articles encompassed therein.

### 4. Examples

### Example 1 - Sanitary Napkin

The following is an Example of a construction of the sanitary napkin shown in Figs. 1-5 of the drawings.

### COMPONENTS

The components for the sanitary napkin are as follows:

| | |
|---|---|
| Absorbent Core - | DANWEB #1079-2338 TBAL, slit with a die |
| Insert - | DANWEB #1079-2338 TBAL with a layer of P-9 nonwoven material melted on the back using iron at 149°C (300°F) (Insert is 8,6 cm x 6,35 cm (3 3/8" x 2 1/2") in size) |
| Topsheet - | Composite of an Apertured Formed film #5780 made under U.S. Patent 4,463,045 by Tredegar Film Products and HAVIX S2146 air-through bonded nonwoven which is cut to shape of core (slightly smaller on all sides, e.g., 5-6 mm than formed film) oriented so that extensibility is lengthwise and fuzzy side is toward film which are adhesively bonded together using core-shaped pattern application of Fuller adhesive #2031 H. B. fuller Company of St. Paul, MN and SELFed. |
| Backsheet - | SELFed Polyethylene film P18-1401 (Clopay) |
| Surfactant - | PEGOSPERSE obtained from Lonza, Inc., Williamsport. PA (0.02 grams). |
| Side Wrapping Elements | Laminate of a 21.5 - 23.9 g/m$^2$ (18-20g/yd$^2$) spunlace nonwoven web comprised of 1.5 denier polyester fibers manufactured as product HEF 44330ES4 by Hercules. Inc. (Oxford, GA) and an elastomeric film (the complete laminate being obtained from Clopay as product # P18-2147) |

(continued)

| | |
|---|---|
| Panty fastener - | Extensible - Fuller 2238 hot melt adhesive (spiral application) |
| | Inextensible - 2 rectangular patches (both conventional pressure sensitive adhesives. e.g., 3 Sigma 3153): |
| | Front of pad- 2,5 x 4,4 cm (1" x 13/4") |
| | Back of pad - 4,4 cm x 1,3 cm (1 3/4" x 1/2") |
| Construction adhesive- | Web of Fuller HL 2352 with release paper |
| Release paper - | Conventional (14 cm (5 1/2") wide) |

ASSEMBLY

The assembly of the main body portion of the sanitary napkin is as follows:

The components are prepared in the form set forth above. The insert is impressed on both sides with intermittent lines so that it will deform into a preferred configuration in use. The P-9 side of the insert is impressed with three spaced apart dashed lines that are centered about the longitudinal centerline of the insert. The TBAL side of the insert is impressed with two concave inwardly-oriented curved dashed lines that lie laterally outboard of the three lines impressed into the other side of the insert. The insert is then bent slightly along all of the dashed lines. A small 3,2 mm x 12,7 mm (1/8" x 1/2") piece of 3M double-sided adhesive tape is centered along each longitudinal edge of the insert and adhered to the P-9 side of the insert. The insert is then placed on top of the absorbent core 4,1 cm (1 5/8") from the front end edge of the core with its P-9 side down.

The SELFed poly backsheet is taped to a glass plate. A sheet of Fuller 2352 construction adhesive with a piece cut out of the center is placed over the SELFed backsheet material. The Fuller construction adhesive is used to seal the perimeter portions of the core to the backsheet material. The central portion of the core is not sealed so that it will be able to decouple from the backsheet. The Fuller construction adhesive also extends beyond the edges of the core so that it is available to form a perimeter seal to the topsheet. The underside of the topsheet sprayed with a surfactant using a template which will keep the surfactant at least 9,5 mm (3/8") from the edge of the absorbent core. The surfactant is permitted to dry. The topsheet is placed on top of the core with the formed film side up. The edges of the assembly of the main body portion are then rolled to seal the components together.

The preparation of and addition of the side wrapping elements to the main body portion and remaining steps are as follows.

The material for the side wrapping elements is cut to the configuration shown in Fig. 14 of the drawings. The side wrapping elements are pleated by folding the same around a trapezoidal-shaped template. The side wrapping element material is folded so that the portion of the pleats at the distal edge of the side wrapping elements overlaps 1.2 cm. An 20,3 cm (8 inch) long strip of Fuller 2352 construction adhesive is placed on the backsheet of the assembled main body portion parallel to and 3,2 mm (1/8 inch) inward of the longitudinal side edges of the absorbent core. The pleated side wrapping elements are placed over the adhesive. The side wrapping elements are rolled to secure the same to the main body portion.

The fasteners are then applied to the garment-facing side of the sanitary napkin. The Fuller hot melt adhesive is applied in a spiral pattern around the perimeter of the main body portion as shown in Fig. 4. The end fastener adhesive patches are then applied so that they are 3,2 mm (1/8 inch) inward from the end of the core. Release paper is applied, and the assembly of the product is completed.

The specifications of the finished product are as follows:

Main Body Portion

| Parameters of Main Body Portion | Specifications |
|---|---|
| Pad length (mm) | 260 ± 4 |
| Core length (mm) laminate | 248 ± 1 |
| Pad width at widest part of first end region (mm) | 94 |
| Pad width in central region (mm) (narrowest portion | 80 ± 1 |
| Pad width at widest part of second end region (mm) | 140 |
| Core width at center (mm) | 65 ± 1 |
| Pad caliper (mm at (0.25 psi) 1,72 kPa) | |

(continued)

| Parameters of Main Body Portion | Specifications |
|---|---|
| At center region | 3.01 ± 0.07 |
| At end regions | 2.07 ± 0.12 |
| Insert length (mm) | 86 |
| Insert width (nun) | 67 |

Side Wrapping Elements

| Parameters | Specifications |
|---|---|
| Overall length at proximal edge (mm) | 220 ± 5 |
| Overall width at narrowest point (mm) | 46 ± 2 |
| Width of pleat at distal edge (mm) | 10 ± 1 |

Example 2 - Panty Liner or Light Day Pad

This Example describes a comfortable, highly absorbent, panty-conforming liner or light day pad for light menstrual use, tampon back-up, mid-cycle discharge or light incontinent use. The comfort is achieved from a combination of stretching with the panty, flexibility, and dryness.

The basic design includes an absorbent core comprising a carded nonwoven web comprised of a blend of 50% FIBERSORB fibrous superabsorbent material and 50% Hercules T-196 polypropylene fibers (the latter obtained from Hercules Company of Oxford, GA.). The core is covered with a carded nonwoven polypropylene web as a topsheet. The topsheet and core are then thermally bonded using the pattern described in U.S. Patent 4,781,710 issued to Megison, et al The topsheet/core structure is then laminated to a polyethylene film backsheet approximately 1 mil in caliper using a stretchable water-insoluble pressure sensitive adhesive or hot melt adhesive.(An alternately preferred method of applying the backsheet to the absorbent structure is to extrude a polyethylene backsheet film directly onto the back of the absorbent structure.) The combined laminate structure is then SELFed or ring rolled to provide the product with extensibility. Preferably, the perimeter of the product is not SELFed or ringrolled so that the product maintains a higher level of integrity during wear. For example, a 1/8" perimeter that is not "SELFed" provides integrity during wear and still allows the total product to stretch. A suitable stretchable fastener, preferably a pressure sensitive adhesive fastener is then applied to the backside of the product, such that it does not appreciably bridge the female portions of the SELFed or ringrolled pattern. The pressure sensitive adhesive can be applied so that it penetrates the female or valley portions of the SELFed or ringrolled pattern and does not appreciably bridge the female portions of the pattern by spraying spiral hot melt adhesives onto the backsheet and then applying pressure. A combination of viscosity and pressures force the adhesive into the recessed areas of the SELFed or ringrolled pattern. In other alternative embodiments, the adhesive can be restricted only to the land or male areas or undeformed regions of the SELFed or ringrolled patterns. A suitable method of applying adhesive only to the surface of the male portions of the pattern on the backsheet is a gravure printing of the adhesive. The caliper of the finished product may be between about < 1 mm and about 4 mm. The flexibility of the product may be between about < 50 g and about 700 grams. The product preferably has a Flexural Rigidity of between about 100 mg-cm to about 3,500 mg-cm and a Flexural Rigidity/Length ratio of from about 2 (mg-cm)/cm to about 20 (mg-cm)/cm.

Such a product construction provides several advantages. The incorporation of fibrous AGM into the product rather than particulate AGM allows AGM containing products to be ringrolled or SELFed without the AGM puncturing the backsheet attempting to perform such operations on products containing particulate AGM.

Standard methods of applying pressure sensitive adhesive fasteners to stretchable products with highly corrugated or textured backsheets have often been unsatisfactory because the adhesive tends to bridge the female portions of the SELFed or ringrolled pattern. This often results in large amounts of adhesive from the fastener transferring to the panty upon removal of the product from the panties. The product described above overcomes this problem.

5. Test Methods

The following are the procedures for determining whether the sanitary napkin (or other absorbent article) falls within the scope of those appended claims that require measurement of certain properties of the article.

All measurements are made on newly packed, and unless otherwise stated, dry (i.e., not wetted or soiled) absorbent articles. The articles should be removed from their package for at least 30 minutes and handled carefully to avoid compressing, or otherwise affecting the properties of the same. Unless otherwise stated, all tests are performed at 50% humidity and at 23°C (73°F), unless otherwise specified on samples that have been conditioned by leaving them in a room at 50% relative humidity and at 23°C (73° F) for a period of two hours prior to the tests. The samples are carefully handled to avoid any stretching or bending of the same prior to performing the tests.

A. Body Contact

Several procedures for estimating the relative amount of the surface area of the body-facing side of the absorbent article that is in direct contact with the wearer's body at a given time are provided below:

(1) The Lift Measuring Test

INTRODUCTION

This test involves the use of a lift measuring test apparatus that is shaped to roughly approximate the various areas of a female body that the absorbent article must fit adjacent in order to achieve close body contact. The lift measuring test apparatus comprises two curved PLEXIGLAS pieces that are intended to approximate the portions of the wearer's body that the crotch of the wearer's undergarments contact during wear. The apparatus contains longitudinally-oriented slit-like opening that is intended to approximate the space between the wearer's labia and the crevice between the wearer's buttocks (the "gluteal groove"). The ends of the absorbent article are attached to clamps which are adjusted to simulate the forces exerted when a woman's panties are pulled up to the wearer's body. The distance that the middle of the absorbent article vertically intrudes into the slit-like opening is measured to provide a relative measurement of body contact.

APPARATUS

| Lift Measuring Apparatus | The lift measuring test apparatus comprises six pieces of PLEXIGLAS arranged as shown in FIGS. 18-22. The Lift Test apparatus 100 has an inside surface 100A, an outside surface 100B, a front portion 100C, and a rear portion 100D. |
| --- | --- |
| | The PLEXIGLAS pieces include two identical 1/4" thick arcuate pieces 102 and 104 which have a height H of 150 mm, a width W of 135 mm, a length S of 300 mm when assembled in an abutting relationship as shown in Figs. 18-20, and a radius of curvature of the inner surface of the arcuate pieces, J (as shown in FIG. 20), of 150 mm. A pair of rectangular PLEXIGLAS support legs 106 are mounted on the sides of the arcuate PLEXIGLAS pieces as shown in Fig. 18. The support legs 106 are mounted perpendicularly to the arcuate pieces so that the bottom 108 of the arcuate pieces is held at least 20 mm above the table on which the test apparatus 100 is placed. |
| | The arcuate pieces 102 and 104 are connected by a pair of hinges 110 that allow the arcuate pieces to open 90°. The arcuate pieces 102 and 104 are held together at the transverse centerline R of the test apparatus by a magnet 112 when closed. The arcuate pieces have an 8 3/8 inch (212 mm) long central longitudinally-oriented slit-like opening (or "slit") 114 (as measured along the curvature of the outside surface 100B of the arcuate pieces) that varies linearly in width from 6 mm at the portion 114A of the slit located nearest to the front of the portion 100C of the test apparatus (the |
| | portion of the apparatus that is intended to represent the front of the wearer's body) to 19 mm at the portion 114B of the slit located nearest to the rear 100D of the apparatus The portions of the PLEXIGLAS surrounding the slit 114 are beveled at a 45° angle so that the slit is wider on the bottom surface 100B than on the top surface of the arcuate pieces. Both ends of the slit 114 are rounded. |

(continued)

The arcuate pieces have additional channels to the front and rear of the slit 114 which are oriented along the longitudinal centerline of the slit. These channels provide a mechanism within which the bolts holding the clamps 118 can slide to adjust the position of the clamps relative to the slit. The arcuate pieces 102 and 104 are provided with tape 116 which can be marked with indicia to indicate the proper position for clamping the ends of the absorbent article in clamps 118.

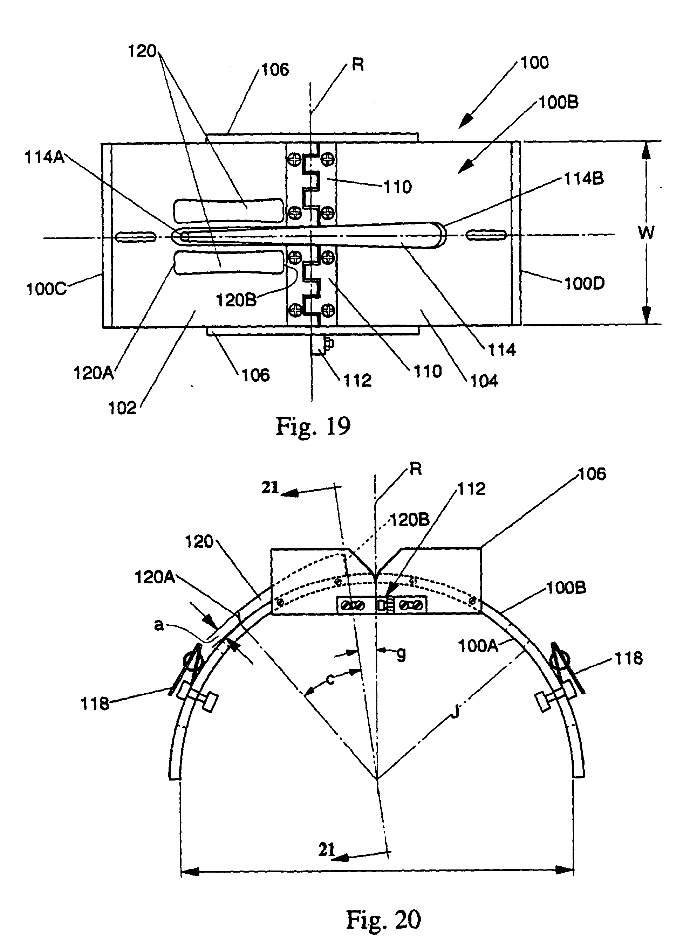

The front arcuate piece 102 of the test apparatus is also provided with a pair of three-dimensionally curved PLEXIGLAS pieces 120 that are intended to represent the wearer's labia majora. The curved pieces 120 have the configuration shown in Figs. 19-21 and the dimensions shown in Table 2 below. The curved pieces are centered about the slit and are spaced 36 mm apart (on center) as described in Table 2 and their rear end edges 120B are spaced from the rear end edge of the first arcuate plate 102 that is defined by the 8° angle g described in Table 2.

TABLE 2 - Dimensions of Curved Pieces

| Dimension | Size (in mm) |
| --- | --- |
| a | 7 mm |
| b | 16 mm |
| c | 33 degrees |
| d | 16 mm |
| e | 6 mm (radius) |
| f | 36 mm |
| g | 8 degrees |

| | |
| --- | --- |
| Weights | Sufficient weight to place total weight of 314 grams on the sample (including weight of clamps (described below)). |
| Clamps | 2 spring-loaded, finger-operated 2 inch (5 cm) wide clamps (Boston No. 2 clips manufactured by Hunt Manufacturing Co., Statesville, N.C.) for securing the absorbent article in the test apparatus using bolts with wing nuts. |
| Pin Chamber Caliper Measurement Fig. Device | Constructed according to attached drawing 22A. |

## PROCEDURE

The release paper covering any adhesive fastener on the garment-facing side of the absorbent article is removed prior to the beginning of the test and any fastener is covered such as by sprinkling talc on adhesive fasteners to minimize the effect of the fastener on the test results. (Unless otherwise stated, this applies to all test procedures described in this specification.)

The caliper of the absorbent article is first measured in accordance with the caliper test procedure set forth in Section D below. Two caliper measurements are taken. The caliper of the point of maximum amplitude of the absorbent article is measured. A measurement of the portion of the absorbent article having the smallest caliper, such as the end regions 28 and 30, which contains absorbent material is also taken (i.e., the minimum caliper). The caliper of the end regions is of interest because these are the regions of the sanitary napkin that typically come into contact with sensitive areas of the wearer's body (such as the gluteal groove). Making these regions of the sanitary napkin thin has been found to be very important to the wearer's comfort. The maximum calipers are determined by taking a sufficient number of caliper measurements at different places over the absorbent article to ensure that the caliper of the portion of the absorbent article with the largest and smallest calipers are measured.

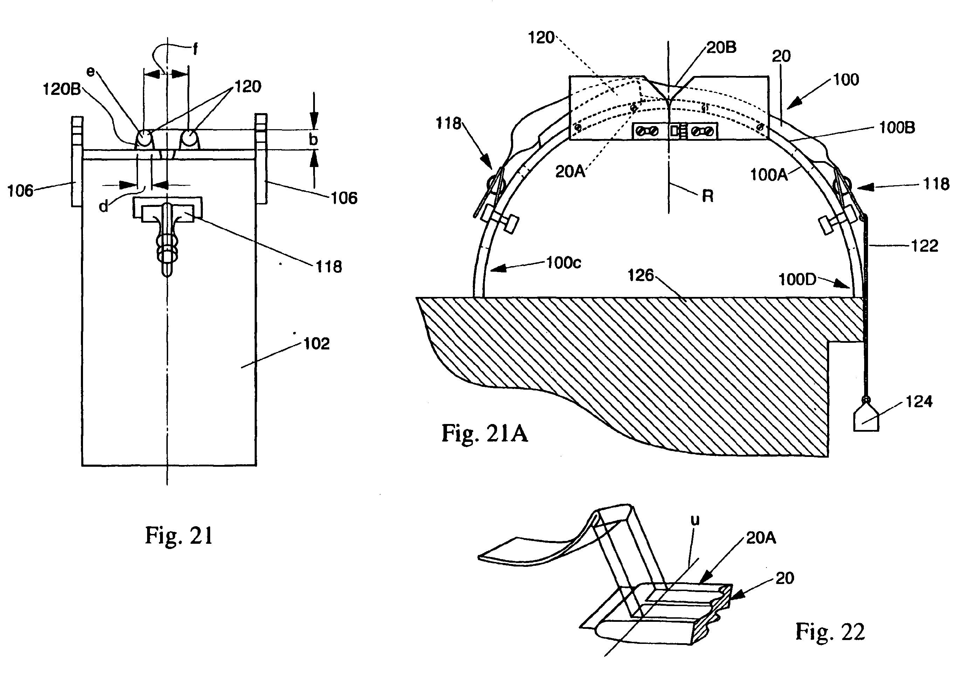

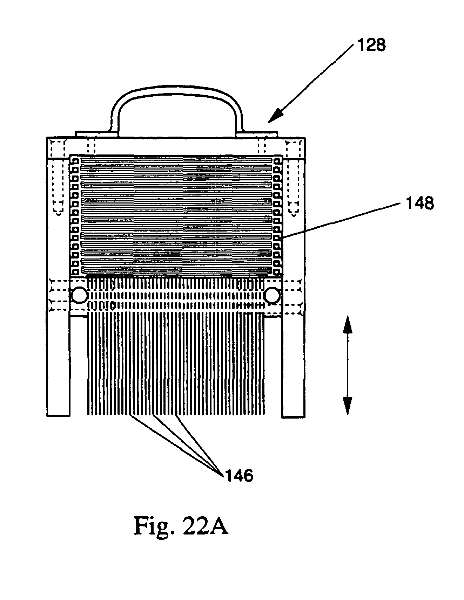

The amount that the absorbent article will extend under a 314 gram force is then measured. This is done to calibrate the test apparatus for each different type of absorbent article being tested. The absorbent article used for the caliper measurements is discarded and a separate absorbent article is used for this measurement. The second absorbent article sample is placed with the body-facing side 20A of the absorbent article 20 adjacent to the outside surface 100B of the arcuate PLEXIGLAS plates of the testing apparatus as shown in Fig. 21 A. The absorbent article, unless it is provided with flaps or side wrapping elements that are offset forward or rearward of the main body of the article, is centered longitudinally with respect to the transverse centerline R of the test apparatus. If the absorbent article has flaps which are offset, the absorbent article is centered on the arcuate plates so that the flap (or side wrapping elements) transverse centerline coincides with the transverse centerline R of the test apparatus.

The clamp 118 at the front portion 100C of the test apparatus is set so that the edge of the jaw of the clamp is spaced 150 mm from the transverse centerline R of the test apparatus (as measured along the curvature of the inside surface 100A of the test apparatus. The clamp 118 at the rear portion 100D of the test apparatus is initially spaced the same distance from the transverse centerline R of the apparatus (toward the rear portion of the test apparatus).

A piece of tape is prepared for each end of the absorbent article. The tape comprises a folded one inch wide strip of 3M masking tape available from the Minnesota Mining and Manufacturing Company of St. Paul, Minn. The strip is folded lengthwise as shown in Fig. 22 so that it extends the desired overall length and all but a one inch (2.54 cm) by 1/2 inch (1.3 cm) area of the adhesive is covered at one end. The tape is used to equalize the results for absorbent articles having different lengths and to negate the effect of the different length and position of the fasteners on the garment side of the article.

A first folded piece of tape is made a length that is long enough so that its exposed adhesive section can be affixed to the front end of the absorbent article in the manner described below and the other end of the tape can be gripped by the clamp at the front portion of the test apparatus while the absorbent article is centered over the test apparatus.

The end of the first folded piece of tape is adhered to the garment-facing side of the absorbent article. The end of the tape with the exposed adhesive is adhered to the absorbent article so that it is centered along the longitudinal centerline of the absorbent article The end of the exposed adhesive section nearest the end of the tape that will go in the clamps aligns with the most longitudinally remote edge of the fastener on the garment-facing side of the absorbent article as shown in Fig. 22. If the absorbent article is not provided with a fastener on its garment-facing side, then the exposed adhesive section of the first piece of tape is adhered to the garment-facing side of the absorbent article a similar distance inward from the portion of the garment-facing side that overlays the end edge of the absorbent material of the absorbent article. Thus, in Fig. 22, the line U denotes either the end edge of a fastener or the end edge of the absorbent material of the absorbent article.

The second folded piece of tape is adhered to the garment-facing side of the absorbent article and clamped in the same manner as the first piece of tape.. The other end of the piece of tape is gripped in the clamp 118 at the adjacent end of the test apparatus. The nut on the clamp 118 at the front portion 100C of the test apparatus is tightened. The nut on the clamp 118 at the rear portion 100D of the test apparatus is left loose so that it can slide freely.

Weights 124 are hung from the clamp 118 at the end of the absorbent article at the rear portion of the test apparatus (where the nut has been loosened). The weight is allowed to hang freely (although a short length of the wire 122 holding the weight 124 may contact part of the rear portion 100D of the test apparatus as shown in Fig. 21 A). The weight should not be dropped, nor should a sudden force be applied with the weight when the pad is hanging freely. After a period of 5 seconds the nut on the clamp at the rear portion 100D of the test apparatus is tightened.

The weight on the end of the absorbent article at the rear portion of the test device places a stretching force on the absorbent article so that the absorbent article tends to want to form a straight path between the clamps. At this point, the absorbent article will move into as close contact within the slit as the absorbent article is capable of achieving under the test conditions.

The test apparatus 100 is turned right side up so that it rests on its support legs 106. The Pin Chamber caliper measurement device is then used to measure the distance the absorbent article rises within the slit from the outside surface 100B of the arcuate plates (the baseline).

The Pin Chamber 128 comprises a case with a plurality of narrow (1.1 mm diameter), spaced apart, vertically-oriented, lightweight (28.4 mg) pins 146 arranged in a row across the device. The pins are movable in the vertical direction. The Pin Chamber case has a glass window in the front and back so that the height of the pins can be observed when the Pin Chamber is in use. A ruler 148 marked in millimeter increments is provided along side of the pins prior to the placement of the absorbent article on the test apparatus. The Pin Chamber is positioned over the test apparatus so that it straddles the test apparatus. A measurement to determine the distance the pins drop to the bottom surface of the arcuate plates is taken at each of the desired locations. These measurements serve as the baseline values for the test. The distance the pins drop above or below the baseline is then measured by gently lowering the pins with the absorbent article in place. It should be noted that the slit is wide enough that several pins may drop between the edges of the slit at various locations. If that occurs, the reading taken is that of the highest pin.

The first measurement is taken at a point that is spaced 47 mm forward of the transverse centerline R of the test apparatus. This distance is intended to correspond with the labia area of wearer's body. (This 47 mm distance, and the following two distance measurements are measured along the curvature of the inside surface 100A of the test apparatus.) The second measurement is taken at a point that is spaced 17 mm to the rear of the transverse centerline of the test apparatus. This is intended to correspond with the wearer's perineum. The third measurement is taken at a point that is spaced 70 mm to the rear of the transverse centerline of the test apparatus. This is intended to correspond with the wearer's "gluteal groove". These values are recorded. The foregoing procedure is repeated for a total of three representative absorbent articles. The three measurements obtained are then averaged to provide a value for the Lift of the absorbent article at each of the locations.

(2) Extensibility and "Set" of the Topsheet

The procedures for measuring the extensibility and "set" of the topsheet and any attached underlying component are as follows.

To measure the extensibility of the topsheet, a one inch (2.54 cm) wide strip sample is cut out of the absorbent article parallel to the principal longitudinal centerline of the absorbent article. A second one inch wide strip sample is cut out of an identical absorbent article parallel to the principal transverse centerline of the absorbent article. The samples should run the full length and width of the absorbent article. Any perimeter seal, such as a peripheral crimped area, where the components of the absorbent article are peripherally joined together is cut away and removed. The remaining samples should then each comprise a strip which includes a strip of the topsheet material, possibly one or more components that are attached to the underside of the topsheet material in a face-to-face relationship, and possibly some strips of underlying components that no longer remain attached to the strip of topsheet material either directly or indirectly after the perimeter seal is removed. Any strips that are no longer joined to the strip of topsheet material either directly or indirectly are discarded. The samples to be used in the test comprise the one inch wide strips of topsheet material and any underlying component that remain attached to the strips of topsheet material.

The ends of the sample are clamped in an INSTRON model 1122 tensile testing apparatus available from Instron Engineering Corporation of Canton. Mass. using a 1 inch (2.5 cm) wide clamps.

The clamps of the tensile tester are set so that they will be pulled away from each other in opposite directions (that is, they will pull at an angle of 180 degrees). The sample should be centered in the clamps and the clamping pressure should be sufficient to prevent any slippage of the sample in the clamps (this applies to all of the test methods). The sample should be clamped so that the outermost edge (i.e., the free end) of the clamps are 2.0 inches (5 cm) apart.

The gauge length of the tensile tester is set at 5 cm (2.0 inches). The load cell of the testing apparatus is tared to zero. The trigger point to begin collecting data is set. The elongation is initiated using a cross head speed of 1.0 inch per minute (2.54 cm per minute). The force required to extend the samples 25% is recorded, and the test is completed and the secant modulus at 25% elongation (defined as the slope of the line drawn between the origin and the stress at 25% elongation) is calculated.

The term "set", as used herein, refers to the amount of permanent deformation (as a percentage of the original sample length) remaining in a sample after application and removal of the indicated strain.

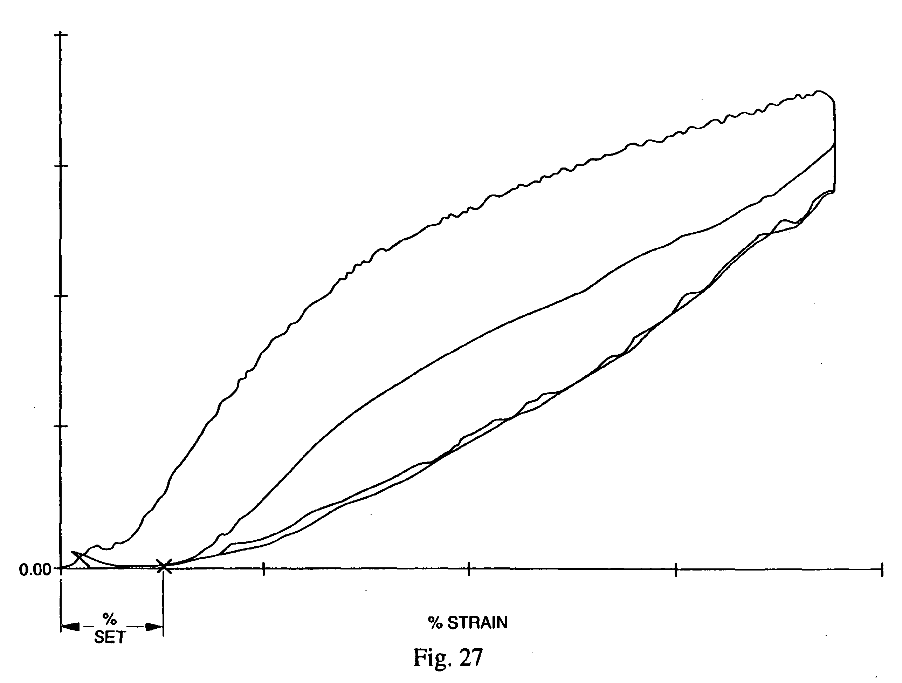

The set of a material is determined by pulling a 2,5 cm (1") wide and greater than 5 cm (2") long (so that a 5 cm (2") gauge length can be used) sample of the material to a given strain in an Instron model 1122 testing apparatus, using a crosshead speed of 1.0 in/min (2.5 cm/min.). The sample is mounted in the Instron grips so that the axis of elongation is parallel to the long dimension of the sample. The gauge length of the sample (the distance between gripping points) is 2" (5 cm). The strain used for this particular test is 25%. That is, a 2" (5 cm) gauge length sample is pulled to 2.5" (6.4 cm) and is held for thirty seconds at that strain. The separation between the grips is then returned to 5 cm (2") (0% strain on the sample) and held at this position for 60 seconds. This cycle is then repeated. The percent set is determined as the first point on the strain axis where the force to elongate is greater than zero during the second cycle. This is illustrated in Figure 27. An average percent set for three samples is reported.

B. Area Coverage

A laboratory method of approximating the amount of panty area (or area of other type of undergarment) covered by an absorbent article requires a stiff cardboard cutout and a sample of the absorbent article to be tested.

The cardboard cutout is in the exact configuration shown in Fig, 28. The cardboard cutout shown in Fig. 28 represents a portion of a woman's panty that a sanitary napkin is generally required to cover during use. The particular outline of the panty crotch shown in Fig. 28 represents the panty when a woman wearer is squatting (on of the more extreme cases encountered during wear in which the panty is stretched to a relatively large size, particularly at the rear portion of the panty). The side edges of the cardboard cutout represent the elasticized side portions of the panty at the leg openings. The measurements of the edges of the panty made along the curvature of the panties on the wearer's body (a three dimensional complex curved surface that is curved from front to back and from side to side).

Fig. 28 is drawn to scale, but is reduced in size from its actual size. The width of the cardboard cutout as measured along the dotted line should be 3 inches (7.6 cm). The space between each numbered line on the side edge of the cardboard cutout should be equal to 10 mm in the full size cutout. Fig. 28 is also covered with a grid of 6,35 cm x 6,35 cm (1/4 inch x 1/4 inch) squares which are useful in determining the area covered by the sanitary napkin 20 which is superimposed over the cardboard cutout. A cardboard cutout for use in the test can be made by enlarging Fig. 28 to the size cutout should be made from cardboard that is stiff enough that it will not bend during the performance of the test. The cardboard cutout provides a consistent undergarment crotch (or "panty crotch") size for comparing the coverage of different absorbent articles.

The absorbent article is centered with its transverse centerline between the two marks on the left side of Fig. 28 if it is a symmetric product. If the absorbent article is asymmetrical, it is generally positioned so that a transverse line running through the narrowest portion of its primarily absorbent component is centered between the two lines on Fig. 28. The absorbent article is affixed to the cardboard cutout in the manner it is intended to be affixed to an undergarment during wear. If the absorbent article has flaps or side wrapping elements, these are wrapped around the sides of the cardboard cutout as they would normally be wrapped around the leg openings in the crotch portion of the undergarment. The transverse centerline of the flaps is positioned between the two marks on the cardboard cutout. If the flaps or side wrapping elements are normally affixed to each other, the normal procedure for affixing them should be followed. However, if the flaps are generally affixed to the underside of the wearer's undergarment, they should be affixed to the bottom of the cardboard cutout.

The distance along the side of the cardboard cutout that is covered by the absorbent article is measured from the scale on the side of the cutout. This method provides a measurement of the relative area coverage that the absorbent article provides in terms of the length of the edge of the undergarment that the absorbent article is capable of covering.

Fig. 28 shows one of the unique features of the preferred sanitary napkin shown in Figs. 1-5. The sanitary napkin 20 is shown as covering the portions of the areas at the rear of the panty (i.e., the cardboard cutout) designated A, A', B, and B'. The sanitary napkin also covers the areas designated C and C' at the front of the cutout. The areas of the cardboard cutout are considered to be "covered" by the surface of an absorbent article if they are covered by either the main body portion 21 of the absorbent article, or if they are covered by any flaps or side wrapping elements 52.

The fact that the sanitary napkin 20 shown in Fig. 28 is capable of covering portions of areas A, A', B, and B' is believed to distinguish it from currently marketed sanitary napkins. Preferably, the sanitary napkin of the present invention is capable of covering at least about 10%, more preferably at least about 20%, 30%, .. , etc. up to 100% of the total area of regions C and C' at the front of the cardboard cutout.

Coverage of the foregoing areas of the cardboard cutout (and, thus, corresponding areas of the wearer's panties) is significant for several reasons. The areas of the cardboard cutout covered by the sanitary napkin of the present invention are believed to be greater than that generally covered by current commercially available sanitary napkins. This provides the desired increased protection from soiling. In addition, the fact that the cardboard cutout is scaled to the dimensions of the panty during wear coupled with the fact that the cutout is substantially covered by the sanitary napkin shows that the napkin has the potential capability of covering the complex curved surface of the panty in use. The fact that the sanitary napkin is highly flexible and drapable and is provided with a perimeter fastener, allows it to actually follow the complex curved shape the panties take during wear and stretch with the panties.

C. Drapability/Flexibility

(1) Drapability

The procedure for measuring the drapability of the absorbent article (expressed in terms of Flexural Rigidity) is as follows:

REFERENCE

ASTM Method B 1388-64: Standard Methods for Test for Stiffness of Fabrics (modified as described herein).

PRINCIPLE

This test is based on the cantilever beam principle. The distance a strip of sample can be extended beyond a flat platform before it bends through a 45° angle is measured. The inter-action between sample weight and sample stiffness measured as the sample bends or drapes under its own weight through the given angle under specified test conditions is used to calculate the Flexural Rigidity.

GENERAL COMMENTS

The drape test is only one way of measuring a sample's stiffness, and one of the components of sample softness. This method should be followed as closely as possible, however, there will be times in which additional testing is necessary and apparatus. For example, testing of samples in only longitudinal and transverse directions is described.

In general, a single sample strip should be tested only <u>one</u> time with the body-facing side of the sample facing upward. Likewise, sample strips for use in this test must be very carefully handled to prevent folds, wrinkles, bends, etc. This test is intended to be used on products before they have been folded or bent for packaging by the manufacturer. If the sample is placed by the manufacturer in a folded configuration, it should be gently unfolded for the test. If only folded products are available, the Flexural Rigidity can be approximated by measuring a sample taken from between the fold lines. If it is impossible to completely remove the folds from a sample, then the measurement may be taken by orienting the sample with the inward portion of the fold facing upward during the test so that the effect of the fold will be minimized.

APPARATUS

| | |
|---|---|
| Cantilever Drape Tester | The tester is constructed according to drawing Fig. 29. |
| 1-inch Wide Sample Cutter | JDC Cutter. Obtain from Thwing-Albert Instrument Company, Philadelphia, Pennsylvania. |
| Paper Cutter | Size convenient for cutting samples to length of (4.0 $\pm$ 0.1 or 6.0 $\pm$ 0.1 inches) 10.2 $\pm$ 0.25 cm or 15.2 $\pm$ 0.25 cm |
| Conditioned Room | A room conditioned to 23 $\pm$ 1°C (73 $\pm$ 2°F), 50 $\pm$ 2% Relative Humidity. |
| Zerostat Anti-Static Pistol (optional) | To eliminate static charge on the drape tester and/or tissue. Distributed in the USA by Discwasher, Inc., Columbia, MO 65201. May be obtained from Morgan Instruments, Inc., P.O. Box 46442, 113 Circle Freeway Dr., Cincinnati, OH 45246. Morgan Catalog No. |
| | 70-35-00. Also available from record shops and photographic supply stores. Use of this pistol is an approved way to remove static charges for this test. <u>Never use fabric softener</u> to remove static charge from a drape test Operate the Anti-Static Pistol according to the manufacturer's instructions. |

SAMPLE PREPARATION

The samples should be placed in an area of the room permitting maximum recirculation of air and maximizing equilibration with the humidity and temperature conditions.

Carefully cut one 1 in. (2.54 cm) wide strip from four representative samples of the absorbent articles to be tested, in both the longitudinal and transverse directions. The strips should initially be cut so that they run in length from one edge of the product to the opposite edge. The sample strips should generally be cut from the most highly drapable portions of the absorbent article. In the case of the strips cut in a transverse direction, this will normally be in the end regions of the absorbent article. The ends of the strips are trimmed so that any portion of the ends of the strips such as the crimped area of the sanitary napkin described herein which does not contain any absorbent material is removed. Mark the direction very lightly on one end with the initials LD or TD.

When trimming any such non-absorbent material from the ends of the strips, carefully make a cut exactly perpendicular to the long dimension of the strip near one end using a paper cutter. It is important that the cut be exactly perpendicular to the long dimension of the strip. As sample strips are cut, the direction of cut is marked lightly on one end.

OPERATION

Using other portions of the samples to be tested, determine the basis weight of the sample in milligrams/cm$^2$.

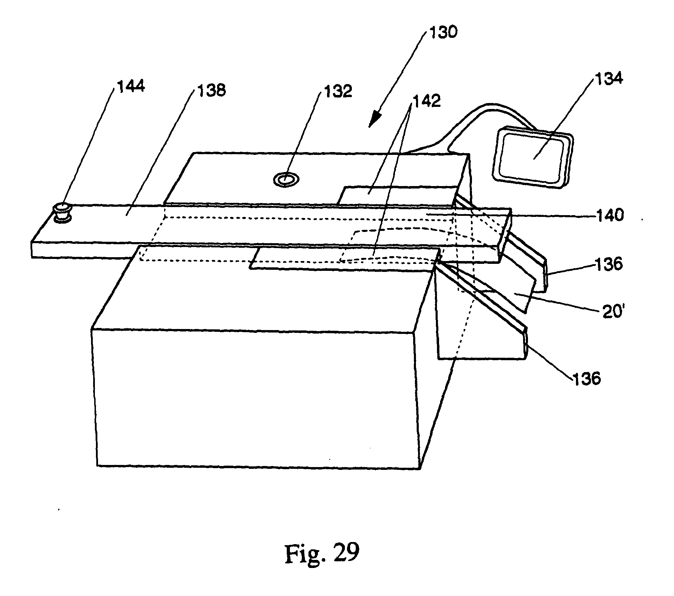

The drape tester 130 (Fig. 29) should be placed on a bench directly in front of the operator. It should be level on the bench as indicated by the small bubble level 132 on the top, and the bench should be relatively free of vibration, excessive heat. and most important - <u>must be free of draft.</u> It is very important that no air current blow directly or indirectly on the tester during operation as this greatly influences the results by causing the sample strip to flutter.

The operator may either sit or stand in front of the tester while it is being used. After the operator has chosen a position in front of the tester which is comfortable, the mirror 134 on the tester must be adjusted so that the upper edge of the two angular plastic fins 136 extending from the one end of the tester appear to be aligned, or coincident, forming a single plane.

Remove the sample slide bar 138 from the sample slot 140 on the top platform of the drape tester. Place one of the test sample strips 20' on the sample slot so that one end of the sample strip is <u>exactly even</u> with the vertical edge of the tester where the two angular fins are attached. Note that the sample slot is slightly wider than the one inch sample.

Place the strip so that it is centered and does not touch the edges of the sample slot. Very carefully place the sample slide bar back on top of the sample strip so that its front edge is also aligned with the edge of the sample strip in the tester. The sample strip will be adjacent to two rulers 142 affixed, one to each side of the sample slot. The sample slide bar 138 must be carefully placed so that the sample is not wrinkled or moved forward, and so that the zero mark on one or the other of the rulers also is lined up exactly with the line on the sample slider.

Using very light, gentle pressure on the knob 144 attached to one end of the sample slide bar, and very gently, steadily, and at a rate of approximately 1/3 - 1/2 in. (0.85 cm - 1.3 cm) per second, move the sample slider forward (toward the end of the tester to which the two fins are attached. As the sample slide bar moves forward, the sample should move at an equal, slow rate. As the sample slider is pushed forward, any tendency of the sample strip to slip can be noted by watching its position with reference to the one of the two indicator lines on the sample slide bar itself. As the sample slide bar and sample strip project over the edge of the tester, the sample strip will immediately or eventually begin to bend, or drape downward. Continue moving the sample slide bar. Stop moving the sample slide bar the instant the leading edge of the absorbent core of the sample strip falls level with the single (coincident and aligned) 45° reference line formed by the two fins as viewed in the mirror. Note: If the sample strip contains a fold line due to the condition which the product was received from the manufacturer, a representative section of the sample strip which does not bend at the fold line should be tested if possible. If it is not possible to eliminate the fold, proceed as described above. Should the sample specimen begin to twist, stop the slide when the mid-point of the leading edge cuts the reference line formed by the two fins. If the twist is greater than 45°, discard the sample and repeat the test on an additional sample strip

After the sample strip has draped to the degree required for the leading edge of the absorbent core to fall level with the 45° reference line and the sample slide bar has been stopped, determine the travel of the sample. Mark the point where the sample draped on the sample.

The length of the portion of the sample that draped is then measured from the point it was marked to the end edge of the absorbent material in the sample. This measurement should not include any portions of the sample that are only comprised of a topsheet and/or backsheet peripheral border. The portion of the sample that draped is then cut at the marked place. Any peripheral border material is also cut away. The sample that remains is then weighed in grams and the basis weight for the sample strip is calculated. The test sequence is performed a total of four times for a particular type of absorbent article in each direction (LD and TD).

CALCULATIONS

The equation used to express Flexural Rigidity is as follows:

$$G = W(L/2)^3$$

Where G equals the Flexural Rigidity, W is the sample basis weight in milligrams/cm$^2$, and L is the length of the overhang in cm excluding the length of any peripheral border. Results are expressed in (milligrams cm) or "milligrams-cm".

(2) Flexibility

The procedure for measuring the flexibility of the absorbent article is the Circular Bend Procedure described in U.S. Patent 5,009,653 issued to Osborn.

D. Caliper

The procedure for measuring the caliper of the absorbent article is set forth in U.S. Patent 5,009,653 issued to Osborn for absorbent articles having substantially uniform caliper. If the absorbent article has a non-uniform caliper (e.g., a hump or other raised portion), the caliper of the raised portion and the surrounding regions should be separately measured using a different shaped comparator foot (e.g., non-circular) such as a rectangular foot having the same surface area so that the desired loading 1,72 kPa (0.25 psi) remains the same. Such a procedure is set out in greater detail in U.S. Patent Application Serial No. 07/874,872 filed April 28, 1992 (PCT Publication WO 93/21879) the disclosure of which is fully incorporated by reference herein. In addition, if the raised portion is more than 1 mm greater in caliper than the surrounding regions when the caliper of the raised portion is measured under no load, the difference in caliper between the raised portion and the surrounding portions is subtracted out from the results in the Lift measurement test.

E. Compressive Force and Resiliency Test

The following test method is used to measure the lateral compressibility properties of a sanitary napkin. The sanitary napkin is compressed by a pair of plates designed to simulate forces and constraints experienced during wear. In this test, the center of a sanitary napkin is subjected to 6 cycles of compression along its width, followed by release of the compressive forces. (This test can also be used with other absorbent articles such as incontinence products and pantiliners). The distance of travel of the plates and resulting force are measured.

(1) Apparatus and Sample Preparation

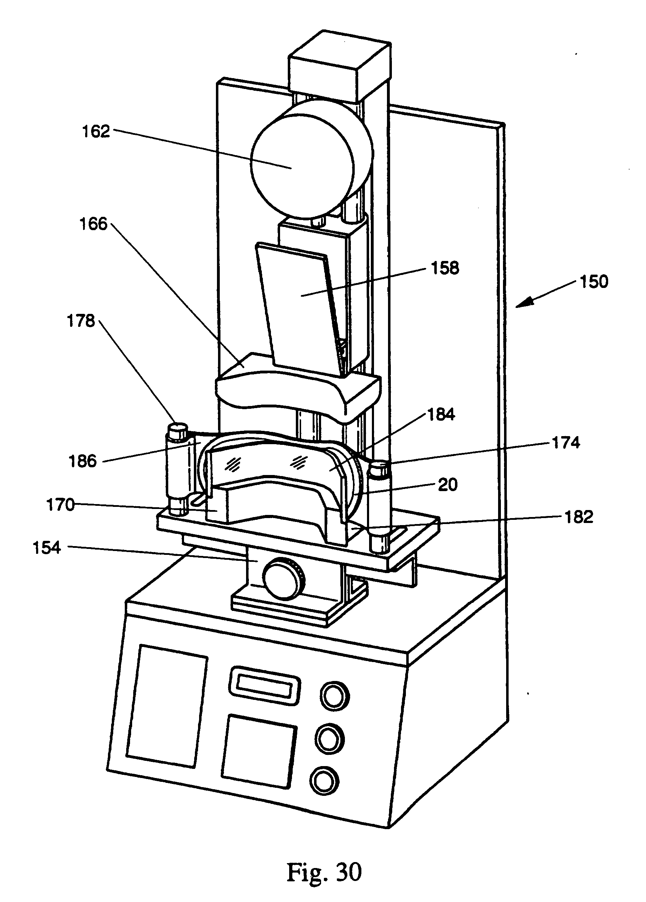

This test requires a constant rate of elongation tensile testing apparatus such as an Instron model #1122 or EME 599A tensile (and compression) testing instruments. FIG. 30 shows this test being performed with an EME 599A instrument obtained from EME Inc., P.O. Box 187, Newbury, OH, indicated generally as 150.

The testing instrument 150 includes a fixed lower clamp 154 for securing one of the compression plates and an upper reciprocating clamp 158 for securing the other compression plate. Instrument 150 also includes a weight (4000 g) indicated generally as 162 for biasing the upper clamp 158 downwardly.

Compressive forces are applied the sanitary napkin 20 by an assembly comprised of a pair of plates 166 and 170. The upper compression plate 166 simulates both the opposite thigh of a wearer and the portion of the body contacting the sanitary napkin 20 during use (perineal area). The lower compression plate 170 also contains two spaced cylindrical posts 174 and 178, one on each side of the lower body portion 182 of the lower plate 170, as well as a PLEXIGLAS viewing screen 186 mounted on body portion 182. These posts 174 and 178 hold the crotch part of a panty 186 for attachment of sanitary napkin 20. (The crotch portion of a suitable panty is cut out from a panty and provided with a sewn tube at either end for attachment to the posts 174 and 178.)

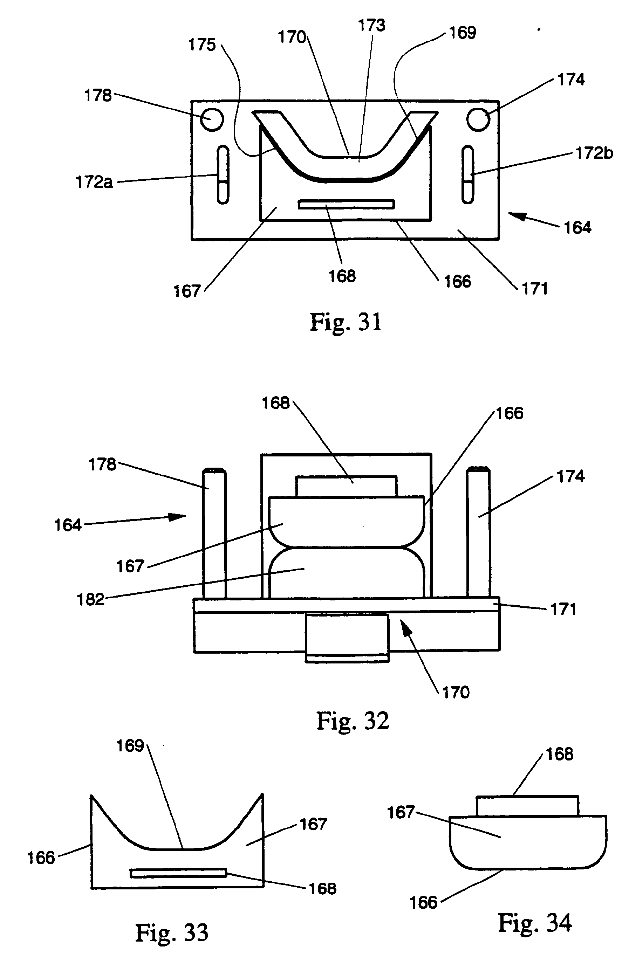

Plates 166 and 170 are shown in greater detail as assembly 164 in FIGS 31 and 32. Referring to these Figures, lower plate 170 comprises a base 171 in which are formed a pair of spaced lots 172a and 172b that are used to secure plate 170 to clamp 154 of instrument 150. As particularly shown in FIG 31, plate 170 has an upper body portion 173 that is provided with a convexly curved face 175.

As particularly shown in FIGS 33 and 34, upper plate 166 has a body portion 167 that is provided with a concave, curved face 169. Attached to body portion 167 is a generally rectangular mounting bracket 168 for securing the upper plate 166 to the reciprocating clamp 158 of instrument 150. As particularly shown in FIG 31, the curved convex face 175 of lower plate 170 forms a matable configuration with curved concave face 169 of upper plate 166. This matable configuration allows upper plate 166 to move past lower plate 170 in close proximity thereof but without contact.

The plates 166 and 170 (and their constituent parts) can be made from any suitable material that can be formed into the required shape (e.g., aluminum. Lexan, Plexiglas). The weight of the plates 166 and 170 together must be significantly lower than the limit of the instrument load cell to allow sufficient range for the force measurement. The plates 166 and 170 should also be centered with the curvatures of the upper and lower pieces lined up opposite one another. When the plates come together during compression, there should be no physical contact between them.

During the test, the crosshead speed is 22 inches/minute (56 cm/minute). The gap between plates 160 and 170 starts at a distance of 10 cm (4 inches), and then narrows to a 1 inch gap distance when sanitary napkin 20 is fully compressed. This equates to an initial cross head setting of 20 cm and a final crosshead setting of 12.5 cm when the dimensions of the apparatus are taken into account.

(2) Test Procedure

A minimum of six samples of each sanitary napkin 20 is required for the test. The release paper is removed from the sanitary napkin 20 and then the sanitary napkin is centered on the panty crotch portion with respect to the seams. The sanitary napkin 20 is then pressed down lightly to ensure it is secured. The sewn tubes on the ends of the panty crotch portion are then slid onto the poles of lower compression plate 170. Sanitary napkin 20 should be in the configuration of an arc with its ends pointing toward the front of instrument 150, and should be loosely confined between the panty crotch and the lower front portion of plate 170. The sanitary napkin 20 is oriented such that it is standing up on one edge. The plates 166 and 170 should now be 4 inches apart.

The plate 166 is then moved towards plate 170 by the downward motion of reciprocating upper clamp 158 until sanitary napkin 20 has been compressed to 1 inch (full compression). Compression is then maintained for 30 seconds. The distance at which the upper compression plate 166 makes contact with the edge of sanitary napkin 20 is determined when a force of 10 g is reached. This is the initial width of the sanitary napkin. The force at the end of the 30 seconds after full compression is reached, and immediately before the compression is released, is recorded as the compression force.

After 30 seconds of full compression, the compressive forces are released by moving plate 166 to its initial position (10 cm (4 inches) apart). Sanitary napkin 20 is left uncompressed for 60 seconds. At the end of 60 seconds, a second compression cycle is started. The same procedure described before is carried out. This procedure is repeated until sanitary napkin 20 has been subjected to 6 compression/release cycles.

Three dry samples of sanitary napkin 20 are tested by this procedure. Three additional samples of sanitary napkin 20 are then tested in the wet state by pouring 7.5 ml of 0.9% saline solution into the center of the samples (allowing the sample to distribute the fluid itself), followed by 10 minutes before testing begins. The wet samples are subjected to the same procedure as the dry samples.

(3) <u>Calculations</u>

After 3 dry samples and wet samples are run, the following values are determined:

(1) The average compression force from cycle 6 on the three dry sanitary napkins;

(2) The average initial sanitary napkin width from cycle 6 on the three wet sanitary napkins:

(3) The average percent width on the three wet sanitary napkins is calculated using the following equation

$$\% \text{ sanitary napkin width} = 100 \text{ x } \frac{\text{(initial sanitary napkin width cycle 6}}{\text{initial sanitary napkin width cycle 1)}}$$

F. <u>Wet Bunch Recoverability Test</u>

The following test is used to measure the amount an absorbent article will tend to recover from bunching after it has been wetted and subjected to transversely-oriented inward forces (i.e., bunched).

The sample used for this test is the entire absorbent article. If the absorbent article is provided with an adhesive fastener, a release paper covering an adhesive fastener on the garment-facing side of the absorbent article is removed and covered with tissue. The same applies to any adhesive on any flaps or side wrapping elements if the absorbent article is provided with side flaps or side wrapping elements. The width of the absorbent element (at its narrowest point if it is hourglass-shaped) is measured. The absorbent article is wetted by dispersing 15 mls of saline solution (available from Baxter Travenol Company of Deerfield, Illinois) uniformly over a portion of the central region of the absorbent article 2 1/2 inches (6.4 cm) in length and the full width of the absorbent element of the absorbent article using a dropper. The absorbent article is allowed to set for 2 minutes so that any superabsorbent material therein may absorb the saline solution.

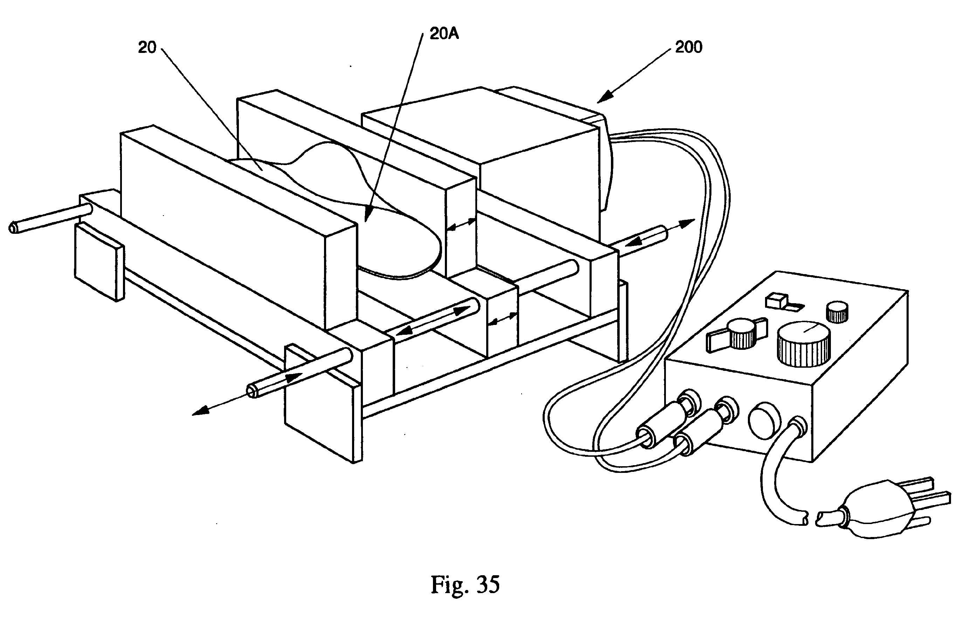

The absorbent article is then placed in the device 200 shown in Fig. 35. The absorbent article 20 is placed in the device 200 with its body-facing side 20A facing upward. If the absorbent article is provided with flaps, the width of the device is set so the flaps are folded upward against the sides of the device. One side of the device is then moved slowly inward until the sides of the device are 20 mm apart. This bunches the absorbent article. After a period of 5 seconds, the side of the device is backed away to the starting position so the absorbent article can be removed.

After the bunching forces are removed, the absorbent article is taken out of the bunching apparatus and the bunching procedure is reversed. The reversal of the bunching procedure can be done by hand or by a suitable apparatus. The bunching of the absorbent article is reversed by gripping the longitudinal side edges of the absorbent article in the central region of the absorbent article and pulling transversely outward in opposite directions on the longitudinal side edges of the absorbent article In the reversal of the bunching, the portions of the absorbent article located outboard of any absorbent material (such as in any crimped area) should be gripped <u>so that no forces are exerted directly on the absorbent material.</u> If the absorbent article is provided with flaps or side wrapping elements, then the outwardly-oriented forces should be applied to the distal edges of the flaps or side wrapping elements. The reversal of the bunching is accomplished by using an amount of force which unbunches or straightens out the non-absorbent portions of the absorbent, article (and the absorbent portions, to the extent this can be done without directly contacting the absorbent portions) in the area where it is gripped. The force applied should not, however, be so high as to destroy the integrity of the absorbent article or any of its components.

The width of the absorbent element is then measured at the same place a width measurement was initially taken. The difference in the two widths can be divided by the initial width to provide a percentage which represents the Wet Bunch Recovery of the absorbent article. This distinguishes the absorbent article from thick airfelt products which have a high degree of irreversible wet bunching (and a low Wet Bunch Recovery). The Wet Bunch Recovery also provides a measurement of panty assisted recovery since during the unbunching portion of the test, the absorbent article is grasped by non-absorbent portions such as the backsheet which is typically the only portion of the absorbent article which is directly linked to the wearer's panties (or other undergarments) and the forces exerted on the absorbent article at this stage simulate the forces exerted on the absorbent article by the panties through the panty fastener when the panties straighten out.

6. Other Alternative Embodiments

While several preferred sanitary napkin embodiments have been described, numerous other sanitary napkin embodiments are disclosed iri the literature. These could be provided with the panty covering component of the present invention. Some of such sanitary napkins are described in U.S. Patents 4,950,264 and 5,009,653 issued to Osborn on August 21, 1990, and April 23, 1991, respectively, in U.S. Patent 5286644, entitled "Curved, Shaped Absorbent Article" filed in the name of Johnson, et al. (PCT Publication No. WO 93/0178 1), in Patent Application WO 93/21879 entitled "Generally, Thin, Flexible Sanitary Napkin With Stiffened Center" and WO 94/16658 entitled "Generally Thin, Flexible Sanitary Napkin With Central Absorbent Hump" in Patent Application WO 95/17150 entitled "Sanitary Napkin Having A Pleated Lifting Member", and in Patent Application WO 95/17149 entitled "Sanitary Napkin Having an Internal Shaping Component" filed in the name of Bergman both filed on December 20, 1993.

The terms "panty liner" and "pantiliner" refer to absorbent articles that are less bulky than sanitary napkins which are generally worn by women between their menstrual periods. Suitable absorbent articles in the form of pantiliners are diseased in U.S. Patent 4,73 8,676 entitled "Pantiliner" issued to Osborn on April 19, 1988.

The term "incontinence article" refers to pads, undergarments (pads held in place by a suspension system of some type, such as a belt, or the like), inserts for absorbent articles, capacity boosters for absorbent articles, briefs, bed pads, and the like, regardless of whether they are worn by adults or other incontinent persons. Suitable incontinent articles that can be provided with the panty covering components described herein are disclosed in US Patent 4,253,461 issued to Strickland, et al. on March 3, 1981. U.S. Patents 4,597,760 and 4,597,761 issued to Buell; the above-mentioned U.S. Patent 4,704,115; U.S. Patent 4.900,802 issued to Ahr, et al., U.S. Patent 4,964,860 issued to Gipson, et al. on October 23, 1990; and in U.S. Patent Application Serial Numbers 07/637,090 and 07/637,571 filed respectively by Noel, et al. and Feist, et al. on January 3, 1991 (PCT Publication Nos. WO 92/11830 and WO 92/11831, respectively, both published on July 23, 1992).

While particular embodiments of the present invention have been illustrated and described, it would be obvious to those skilled in the art that various other changes and modifications can be made without departing from the scope of the invention. It is to be understood that all of the limits and ranges specified in the foregoing description of the fastening device include all narrower ranges and limits that are within the specified limits and ranges. Thus, for example if a range is specified as being between about 6 and about 42 $g/m^2$, all narrower ranges, such as between about 10 and about 40 $g/m^2$, and between about 20 and about 30 $g/m^2$, etc may be claimed even though these ranges are not separately listed. It is therefore intended to cover in the appended Claims all such changes and modifications that are within the scope of this invention.

**Claims**

1. An absorbent article (20) for wearing in an undergarment said absorbent article (20) having a longitudinal dimension oriented in a longitudinal direction a transverse dimension oriented in a transverse direction, a first end region (28), a second end region (30), and a central region (32) disposed between said first end region (28) and said second end region (30), said absorbent article (20) comprising:

   a main body portion (21) comprising a liquid pervious topsheet (38), a liquid impervious backsheet (40), and an absorbent core (42) positioned between said topsheet (38) and said backsheet (40), said absorbent article (20) being extensible in at least one of said longitudinal direction and said transverse direction, wherein said main body portion (21) has an initial relaxed dimension when laid flat as measured as the distance between two marks which are made on said backsheet (40) on opposite sides of one of said centerlines, and said main body portion (21) has at least one extended dimension during wear that is greater than said initial dimension as measured by measuring the distance between said marks during wear of said absorbent article (20) in a direction parallel to at least one of said longitudinal direction and said transverse direction, said absorbent article (20)

   having said liquid impervious backsheet (40) joined to said topsheet (38) along at least a portion of the periphery (26) of said absorbent article (20) by a liquid impervious peripheral seal comprising an adhesive (92), said absorbent article (20) **characterized in that** said liquid impervious peripheral seal further comprises a plurality of spaced apart mechanical bonds (94) arranged in intermittent zones of bonded and unbonded areas, wherein said bonded areas are spaced apart in the direction of extensibility; wherein said joined portion of said periphery (26) and said peripheral seal are extensible.

**Patentansprüche**

1. Absorptionsartikel (20) zum Tragen in Unterwäsche, wobei der Absorptionsartikel (20) eine in Längsrichtung ausgerichtete Längsabmessung, eine in Querrichtung ausgerichtete Querabmessung, einen ersten Endbereich (28), einen zweiten Endbereich (30) und einen Mittelbereich (32), der sich zwischen dem ersten Endbereich (28) und dem zweiten Endbereich (30) befindet, umfasst, wobei der Absorptionsartikel (20) Folgendes umfasst:

   einen Hauptteil (21), umfassend eine flüssigkeitsdurchlässige Oberschicht (38), eine flüssigkeitsundurchlässige Unterschicht (40) und einen Absorptionskern (42), der zwischen der Oberschicht (38) und der Unterschicht (40) positioniert ist, wobei sich der Absorptionsartikel (20) in mindestens einer der Längsrichtung und der Querrichtung befindet, worin der Hauptteil (21) eine anfängliche entspannte Abmessung hat, wenn er flach ausgelegt ist, wie sie als Abstand zwischen zwei Markierungen, die auf der Unterschicht (40) auf gegenüber liegenden Seiten einer der Mittelachsen gemacht werden, gemessen wird, und wobei der Hauptteil (21) beim Tragen mindestens eine verlängerte Abmessung hat, die größer ist als die anfängliche Abmessung, wie durch Abstandsmessung zwischen den Markierungen beim Tragen des Absorptionsartikels (20) in einer Richtung parallel zu mindestens einer der Längsrichtung und der Querrichtung gemessen, wobei beim Absorptionsartikel (20) die flüssigkeitsundurchlässige Unterschicht (40) entlang mindestens eines Teils des Umfangs (26) des Absorptionsartikels (20) durch eine flüssigkeitsundurchlässige Randversiegelung, die ein Haftmittel umfasst (92), mit der Oberschicht (38) verbunden ist,
   der Absorptionsartikel (20) **dadurch gekennzeichnet ist, dass** die flüssigkeitsundurchlässige Randversiegelung ferner eine Vielzahl von auf Abstand befindliche mechanische Bindungen (94) umfasst, die in intermittierenden Zonen von gebundenen und ungebundenen Bereichen angeordnet sind, wobei die gebundenen Bereiche in Dehnbarkeitsrichtung auf Abstand angeordnet sind; wobei der verbundene Teil des Umfangs (26) und die Randversiegelung dehnbar sind.

**Revendications**

1. Article absorbant (20) destiné à être porté dans un sous-vêtement, ledit article absorbant (20) ayant une dimension longitudinale orientée dans une direction longitudinale, une dimension transversale orientée dans une direction transversale, une première région de fin (28), une deuxième région de fin (30), et une région centrale (32) disposée entre ladite première région de fin (28) et ladite deuxième région de fin (30), ledit article absorbant (20) comprenant :

   une partie de corps principal (21) comprenant une feuille de dessus perméable aux liquides (38), une feuille de fond imperméable aux liquides (40), et une âme absorbante (42) positionnée entre ladite feuille de dessus (38) et ladite feuille de fond (40), ledit article absorbant (20) étant extensible dans au moins une desdites direction longitudinale et direction transversale, dans laquelle ladite partie de corps principal (21) a une dimension initiale relâchée lorsqu'elle est couchée à plat mesurée comme la distance entre deux marques qui sont faites sur ladite feuille de fond (40) sur les côtés opposés de l'une desdites lignes médianes, et ladite partie de corps principal (21) a au moins une dimension en extension pendant le port qui est supérieure à ladite dimension initiale telle que mesurée en mesurant la distance entre lesdites marques pendant le port dudit article absorbant (20) dans une direction parallèle à au moins une desdites direction longitudinale et direction transversale, ledit article absorbant (20)
   ayant ladite feuille de fond imperméable aux liquides (40) jointe à ladite feuille de dessus (38) le long d'au moins une partie de la périphérie (26) dudit article absorbant (20) par un joint périphérique imperméable aux liquides comprenant un adhésif (92),
   ledit article absorbant (20) **caractérisé en ce que** ledit joint périphérique imperméable aux liquides comprend en outre une pluralité de liaisons mécaniques espacées (94) arrangées dans des zones intermittentes de zones liées et non liées, dans lequel lesdites zones liées sont espacées dans la direction de l'extensibilité ; dans lequel ladite partie jointe de ladite périphérie (26) et ledit joint périphérique sont extensibles.

Fig. 1

Fig. 2

Fig. 3

**Fig. 4**

**Fig. 5**

Fig. 6

| | LONGITUDINAL | | | WIDTH | | | ELASTIC WALL | |
|---|---|---|---|---|---|---|---|---|
| | % LONGITUDINAL STRETCH | g. OF FORCE TO EXTEND PAD | % PAD SET | % WIDTH STRETCH | g. OF FORCE TO EXTEND 1.0" STRIP | % PAD SET | % STRETCH | g. FORCE |
| CONDITIONS FOR STRETCH | 40% | ≤1000 g. | ≤10 | 40% | ≤500 g. | ≤10 | 50% | 1500 g. |
| | | ≤800 g. | ≤10 | | ≤400 g. | ≤25 | | 2000 g. |
| | | | ≤25 | 25% | ≤500 g. | ≤10 | 40% | 1500 g. |
| | 25% | ≤800 g. | ≤10 | | ≤400 g. | ≤25 | | 2000 g. |
| | | ≤400 g. | ≤25 | | | | 25% | 1500 g. |
| | | ≤300 g. | | | | | | 2000 g. |
| MINIMUM FORCE TO STRETCH | 25% | ≥50 g. | | | | | | |

Table 1...Typical Values for Stretch Parameters

Fig. 7

EP 0 843 539 B1

Fig. 8

Fig. 9

Fig. 10

Fig. 11

46

Fig. 12

Fig. 13

42C

96

90

22

98

Fig. 14

58A

58C

56

52

58B

54

Fig. 15

56

54

Fig. 16

Fig. 17

Fig. 18

EP 0 843 539 B1

Fig. 19

Fig. 20

Fig. 21

Fig. 21A

Fig. 22

EP 0 843 539 B1

Fig. 22A

LIFT MEASURED AT POSITION #2

Fig. 23

LIFT MEASURED AT POSITION #1

Fig. 24

LIFT MEASURED AT POSITION #3

Fig. 25

TABLE 3

| APPLIED | ALWAYS ULTRA PLUS LIFT IN MILLIMETERS AT POSITION | | | STRETCH PROTOTYPE WITH INSERT LIFT IN MILLIMETERS AT POSITION | | |
|---|---|---|---|---|---|---|
| FORCE (g) | #1 | #2 | #3 | #1 | #2 | #3 |
| 27 | 0.5 | -6.5 | 1.5 | 0.0 | -5.0 | 1.5 |
| 79 | 0.5 | -0.5 | 2.0 | 3.0 | -2.0 | 3.0 |
| 123 | 1.5 | 0.0 | 2.0 | 4.0 | 0.5 | 6.0 |
| 204 | 2.0 | 0.0 | 2.5 | 8.0 | 2.5 | 9.0 |
| 254 | 2.0 | 0.0 | 2.5 | 9.0 | 5.0 | 9.5 |
| 314 | 3.0 | 1.0 | 3.0 | 10.5 | 7.0 | 11.5 |
| 354 | 3.0 | 0.0 | 2.5 | 10.0 | 8.0 | 12.0 |

Fig. 26

EP 0 843 539 B1

% STRAIN

Fig. 27

Fig. 28

Fig. 29

Fig. 30

Fig. 31

Fig. 32

Fig. 33

Fig. 34

Fig. 35

EP 0 843 539 B1